# EUROPEAN PATENT SPECIFICATION

(11) **EP 3 252 052 B1**
(45) Date of publication and mention of the grant of the patent: **19.05.2021**
(21) Application number: 16743201.2
(22) Date of filing: 20.01.2016
(51) Int. Cl.: C07D 471/04, H01L 31/10, H01L 51/50, H05B 33/10

(54) **PHENANTHROLINE DERIVATIVE, ELECTRONIC DEVICE CONTAINING SAME, LIGHT EMITTING ELEMENT, AND PHOTOELECTRIC CONVERSION ELEMENT**
PHENANTHROLINDERIVAT, ELEKTRONISCHE VORRICHTUNG DAMIT, LICHTEMITTIERENDES ELEMENT UND FOTOELEKTRISCHES UMWANDLUNGSELEMENT
DÉRIVÉ DE PHÉNANTHROLINE, DISPOSITIF ÉLECTRONIQUE CONTENANT CELUI-CI, ÉLÉMENT ÉLECTROLUMINESCENT ET ÉLÉMENT DE CONVERSION PHOTOÉLECTRIQUE

(30) Priority: 29.01.2015 JP 2015015452
(43) Date of publication of application: 06.12.2017
(73) Proprietor: Toray Industries, Inc., Tokyo 103-8666 (JP)
(72) Inventor: SAKAINO, Hirotoshi, Otsu-shi Shiga 520-8558 (JP); TANAKA, Daisaku, Otsu-shi Shiga 520-8558 (JP)
(74) Representative: Hoefer & Partner Patentanwälte mbB
(86) International application number: PCT/JP2016/051578
(87) International publication number: WO 2016/121597

(56) References cited:
- WO-A1-2004/026870
- WO-A1-2013/145667
- WO-A1-2014/042163
- CN-A- 102 127 073
- CN-A- 102 127 073
- CN-A- 102 372 709
- JP-A- 2005 108 720
- JP-A- 2008 189 660
- JP-A- 2014 123 687
- KR-A- 20120 072 785
- KR-A- 20120 083 243
- COLLIN,J.-P. ET AL.: "C S I R O Published by CSIRO PUBLISHING for CSIRO Australia and the Australian Academy of Science Synthesis and Structural Characterization of Copper(I) and Copper(II) Complexes with an Ambivalent Phenanthroline-Type Ligand", , 1997, pages 951-957, XP055475070, Retrieved from the Internet: URL:http://www.publish.csiro.au/ch/pdf/C97 106
- SAMANTA,S.K. ET AL.: 'Reversible cargo shipping between orthogonal stations of a nanoscaffold upon redox input' DALTON TRANSACTIONS vol. 43, no. 25, 07 July 2014, pages 9438 - 9447, XP055469316 DOI: 10.1039/C4DT00849A
- BELFREKH,N. ET AL.: 'Synthesis of multifunctional ligands: a 2,9-diaryl-1,10-phenanthroline/ 2,2':6',2''-terpyridine conjugate' TETRAHEDRON LETTERS vol. 42, no. 15, 09 April 2001, pages 2779 - 2781, XP004232315 DOI: 10.1016/S0040-4039(01)00279-9
- ZHANG,M. ET AL.: 'Chromium(III) complexes bearing 2-benzazole-1,10-phenanthrolines: synthesis, molecular structures and ethylene oligomerization and polymerization' DALTON TRANSACTIONS vol. 32, 24 June 2009, pages 6354 - 6363, XP055469317 DOI: 10.1039/B902361E
- CHAMPIN,B. ET AL.: 'A highly rigid ditopic conjugate with orthogonal coordination axes and its zinc(II) and copper(II) complexes' NEW JOURNAL OF CHEMISTRY vol. 32, no. 6, 05 February 2008, pages 1048 - 1054, XP055469319 DOI: 10.1039/B713367G
- YANG,Y. ET AL.: 'Synthesis, structure, and catalytic ethylene oligomerization of nickel complexes bearing 2-pyrazolyl substituted 1,10- phenanthroline ligands' JOURNAL OF MOLECULAR CATALYSIS A: CHEMICAL vol. 296, no. 1-2, 10 December 2008, pages 9 - 17, XP025648386 DOI: 10.1016/J.MOLCATA.2008.08.015
- NOBLAT,S. ET AL.: 'Synthesis of an oblique bis- porphyrin system containing a 1,10- phenanthroline spacer' TETRAHEDRON LETTERS vol. 28, no. 47, 01 January 1987, pages 5829 - 5832, XP055469322

## Description

### TECHNICAL FIELD

The present invention relates to a phenanthroline derivative, an electronic device containing the same, a light emitting element, and a photoelectric conversion element.

### BACKGROUND ART

Researches on an organic thin-film light emitting element in which electrons injected from a cathode and holes injected from an anode emit light when they are recombined in an organic fluorescent body held by both electrodes have been actively conducted in recent years. This light emitting element is characteristic for high luminance light emission in the form of a thin type and under a low driving voltage, and multicolor light emission due to selection of a fluorescent material, and has been paid attention.

Such researches have undergone many studies for practical use since C. W. Tang et al. of Kodak Co., Ltd. showed that an organic thin-film element emits light at high luminance, and organic thin-film light emitting elements have steadily come into practical use as they have been employed in main displays of mobile phones, and the like. However, there are still many technical problems, and it is necessary to satisfy an improvement in luminous efficiency, a reduction in driving voltage and an improvement in durability. Particularly, attainment of both increased efficiency and prolonged life of an element is one of the major challenges.

Under these situations, phenanthroline derivatives having excellent electron transporting property have been developed as electron transporting materials and emissive materials. A phenanthroline skeleton is known to have excellent electron transporting property. For example, bathophenanthroline (BPhen) and bathocuproine (BCP) have been used as electron transporting materials (see, for example, Patent Document 1). Techniques have been disclosed in which heat resistance is improved and crystallinity is reduced by introducing a substituent (see, for example, Patent Documents 2 to 5), and a plurality of phenanthroline skeletons are coupled for improving electron transporting property (see, for example,

Patent Document 6).

Patent Document 7 discloses an organic electroluminescent element which is driven at a low voltage and exhibits high efficiency. The organic electroluminescent element includes an anode, a cathode provided facing the anode, and an organic layer provided between the anode and the cathode. The organic layer has a light emitting layer, and a first electron transport layer and a second electron transport layer, which are provided on the cathode side of the light emitting layer. The first electron transport layer contains a compound represented by the following formula: 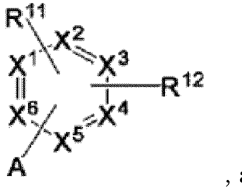 and the second electron transport layer contains a compound represented by the following formula:

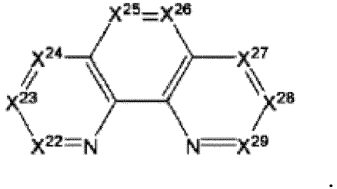

### PRIOR ART DOCUMENTS

### PATENT DOCUMENTS

Patent Document 1: Japanese Patent Laid-open Publication No. 5-331459
Patent Document 2: Japanese Patent Laid-open Publication No. 2004-107263
Patent Document 3: Japanese Patent Laid-open Publication No. 2003-338377
Patent Document 4: Japanese Patent Laid-open Publication No. 2007-45780
Patent Document 5: Japanese Patent Laid-open Publication No. 2005-108720
Patent Document 6: Japanese Patent Laid-open Publication No. 2004-281390
Patent Document 7: JP 2014-123687 A

### SUMMARY OF THE INVENTION

### PROBLEMS TO BE SOLVED BY THE INVENTION

However, it was difficult for conventional technologies to reduce the driving voltage of an element sufficiently, and even if they had been able to reduce the driving voltage, the luminous efficiency and the durable life of an element were insufficient. Thus, technologies capable of realizing all of high luminous efficiency, low driving voltage and durable life have not been found yet.

An object of the present invention is to solve such problems with the conventional technologies and provide an organic thin-film light emitting element that has improved all of luminous efficiency, driving voltage and durable life.

### SOLUTIONS TO THE PROBLEMS

The present invention, which is defined in the appended claims, provides a phenanthroline derivative represented by the following general formula (1): 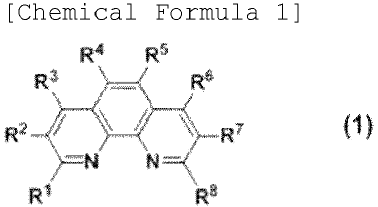
wherein R¹ to R⁸ may be the same or different, and are each selected from the group consisting of hydrogen, an alkyl group, a cycloalkyl group, a heterocyclic group, an alkenyl group, a cycloalkenyl group, an alkynyl group, a halogen, a cyano group, a carbonyl group, a carboxyl group, an oxycarbonyl group, a carbamoyl group, an amino group, a boryl group, a silyl group and -P(=O)R⁹R¹⁰; R⁹ and R¹⁰ each represent an aryl group or a heteroaryl group, with the proviso that any one of R¹ and R² is a group represented by L¹-B, and any one of R⁷ and R⁸ is a group represented by L²-C; R¹ to R¹⁰ may be each substituted or unsubstituted; R¹ to R⁸ has no phenanthroline skeleton;
L¹ and L² may be the same or different, and are each selected from a single bond and a phenylene group;
B is a pyridyl group, a quinolinyl group, an isoquinolinyl group, a pyrazinyl group, a pyrimidinyl group, a pyridazinyl group, a triazinyl group, a quinoxalinyl group, a quinazolinyl group, a benzoquinolinyl group, an acridinyl group, a benzoimidazolyl group or an imidazopyridyl group, C is a naphthyl group, a fluorenyl group, phenanthryl group, a pyrenyl group, a triphenylenyl group or a fluoranthenyl group;
substituents that B and C optionally have are each selected from the group consisting of heavy hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, a halogen, a cyano group, a carbonyl group, a carboxyl group, an oxycarbonyl group, a carbamoyl group, an amino group, a boryl group, a silyl group, a phenyl group, a naphthyl group, a pyridyl group, a quinolinyl group, and -P (=O)R⁹R¹⁰; and these groups may be each substituted with heavy hydrogen, an alkyl group, a halogen, a phenyl group, a naphthyl group, a pyridyl group or a quinolinyl group

### EFFECTS OF THE INVENTION

According to the present invention, there can be provided an organic thin-film light emitting element that realizes all of luminous efficiency, driving voltage and durable life.

### EMBODIMENTS OF THE INVENTION

### <Phenanthroline Derivative>

A phenanthroline derivative represented by the general formula (1) will be described in detail. 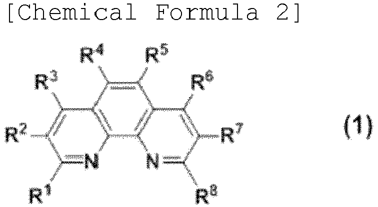 wherein R¹ to R⁸ may be the same or different, and are each selected from the group consisting of hydrogen, an alkyl group, a cycloalkyl group, a heterocyclic group, an alkenyl group, a cycloalkenyl group, an alkynyl group, a halogen, a cyano group, a carbonyl group, a carboxyl group, an oxycarbonyl group, a carbamoyl group, an amino group, a boryl group, a silyl group and -P (=O)R⁹R¹⁰; R⁹ and R¹⁰ each represent an aryl group or a heteroaryl group, with the proviso that any one of R¹ and R² is a group represented by L¹-B, and any one of R⁷ and R⁸ is a group represented by L²-C; R¹ to R¹⁰ may be each substituted or unsubstituted; R¹ to R⁸ has no phenanthroline skeleton;
L¹ and L² may be the same or different, and are each selected from a single bond and a phenylene group;
B is a pyridyl group, a quinolinyl group, an isoquinolinyl group, a pyrazinyl group, a pyrimidinyl group, a pyridazinyl group, a triazinyl group, a quinoxalinyl group, a quinazolinyl group, a benzoquinolinyl group, an acridinyl group, a benzoimidazolyl group or an imidazopyridyl group, C is a naphthyl group, a fluorenyl group, phenanthryl group, a pyrenyl group, a triphenylenyl group or a fluoranthenyl group;
substituents that B and C optionally have are each selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, a halogen, a cyano group, a carbonyl group, a carboxyl group, an oxycarbonyl group, a carbamoyl group, an amino group, a boryl group, a silyl group, a phenyl group, a naphthyl group, a pyridyl group, a quinolinyl group, and -P(=O)R⁹R¹⁰; and these groups may be each substituted with an alkyl group, a halogen, a phenyl group, a naphthyl group, a pyridyl group or a quinolinyl group.

The number of ring-forming carbon atoms denotes the number of carbon atoms that form a ring serving as a main skeleton, and does not include the number of carbon atoms contained in a substituent. For example, the number of ring-forming carbon atoms in a naphthyl group is 10 regardless of presence/absence of a substituent, and the number of ring-forming carbon atoms in a fluorenyl group is 13 regardless of presence/absence of a substituent.

In all the groups described above, hydrogen may be heavy hydrogen.

Hereinafter, for example, the substituted or unsubstituted aryl group having 6 to 40 carbon atoms has 6 to 40 carbon atoms including carbon atoms contained in the substituent with which the aryl group is substituted, and the same applies to other substituents that define the number of carbon atoms.

As substituents that all the above-mentioned groups optionally have, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, a carbonyl group, a carboxyl group, an oxycarbonyl group, a carbamoyl group, an amino group, a silyl group and -P(=O)R⁹R¹⁰ are preferable, and further specific substituents mentioned as preferable substituents in the descriptions of the substituents are preferable. These substituents may be further substituted with the substituents described above.

The term "unsubstituted" associated with the term "substituted or unsubstituted" means that a group is substituted with a hydrogen atom or a heavy hydrogen atom.

The same applies to the term "substituted or unsubstituted" for the compounds described below or substructures thereof.

The alkyl group denotes a saturated aliphatic hydrocarbon group, such as a methyl group, an ethyl group, a n-propyl group, an isopropyl group, a n-butyl group, a sec-butyl group, and a tert-butyl group, and it may or may not have a substituent. When the alkyl group is substituted, the additional substituent is not particularly limited, examples may include a halogen, an alkyl group, an aryl group and a heteroaryl group, and the same holds true in the descriptions below. The number of carbon atoms in the alkyl group is not particularly limited, but from the viewpoints of easy availability and cost, it is preferably within the range of 1 or more and 20 or less, more preferably 1 or more and 8 or less.

The cycloalkyl group denotes a saturated alicyclic hydrocarbon group, such as a cyclopropyl group, a cyclohexyl group, a norbornyl group, and an adamantyl group, and this may or may not have a substituent. The number of carbon atoms in the alkyl group moiety is not particularly limited, but is preferably within the range of 3 or more and 20 or less.

The heterocyclic group denotes an aliphatic ring having an atom other than carbon in the ring, such as a pyran ring, a piperidine ring, and a cyclic amide, and this may or may not have a substituent. The number of carbon atoms in the heterocyclic group is not particularly limited, but is preferably within the range of 2 or more and 20 or less.

The alkenyl group denotes an unsaturated aliphatic hydrocarbon group containing a double bond, such as a vinyl group, an allyl group, and a butadienyl group, and this may or may not have a substituent. The number of carbon atoms in the alkenyl group is not particularly limited, but is preferably within the range of 2 or more and 20 or less.

The cycloalkenyl group denotes an unsaturated alicyclic hydrocarbon group containing a double bond, such as a cyclopentenyl group, a cyclopentadienyl group, and a cyclohexenyl group, and this may or may not have a substituent.

The alkynyl group denotes an unsaturated aliphatic hydrocarbon group containing a triple bond, such as an ethynyl group, and this may or may not have a substituent. The number of carbon atoms in the alkynyl group is not particularly limited, but is preferably within the range of 2 or more and 20 or less.

The alkoxy group denotes a functional group with an aliphatic hydrocarbon group bonded via an ether bond, such as a methoxy group, an ethoxy group, and a propoxy group, and this aliphatic hydrocarbon group may or may not have a substituent. The number of carbon atoms in the alkoxy group is not particularly limited, but is preferably within the range of 1 or more and 20 or less.

The alkylthio group denotes a group in which an oxygen atom of an ether bond in an alkoxy group is substituted with a sulfur atom. The hydrocarbon group of the alkylthio group may or may not have a substituent. The number of carbon atoms in the alkylthio group is not particularly limited, but is preferably within the range of 1 or more and 20 or less.

The aryl ether group denotes a functional group with an aromatic hydrocarbon group bonded via an ether bond, such as a phenoxy group, and the aromatic hydrocarbon group may or may not have a substituent. The number of carbon atoms in the aryl ether group is not particularly limited, but is preferably within the range of 6 or more and 40 or less.

The aryl thioether group denotes a group in which an oxygen atom of an ether bond in an aryl ether group is substituted with a sulfur atom. The aromatic hydrocarbon group in the aryl ether group may or may not have a substituent. The number of carbon atoms in the aryl ether group is not particularly limited, but is preferably within the range of 6 or more and 40 or less.

For example, the aryl group denotes an aromatic hydrocarbon group such as a phenyl group, a biphenyl group, a terphenyl group, a naphthyl group, a fluorenyl group, a benzofluorenyl group, a dibenzofluorenyl group, a phenanthryl group, an anthracenyl group, a benzophenanthryl group, a benzoanthracenyl group, a chrysenyl group, a pyrenyl group, a fluoranthenyl group, a triphenylenyl group, a benzofluoranthenyl group, a dibenzoanthracenyl group, a perylenyl group or a helicenyl group.

Particularly, a phenyl group, a biphenyl group, a terphenyl group, a naphthyl group, a fluorenyl group, a phenanthryl group, an anthracenyl group, a pyrenyl group, a fluoranthenyl group and a triphenylenyl group are preferable. The aryl group may or may not have a substituent. The number of carbon atoms in the aryl group is not particularly limited, but is preferably within the range of 6 or more and 40 or less, more preferably within the range of 6 or more and 30 or less.

As aryl groups with which B, C, L¹ and L² are optionally substituted, or with which the substituents thereof are optionally further substituted, a phenyl group, a biphenyl group, a terphenyl group and a naphthyl group are preferable, and a phenyl group and a naphthyl group are more preferable.

The heteroaryl group denotes a cyclic aromatic group having one or a plurality of atoms other than carbon in the ring, such as a pyridyl group, a furanyl group, a thiophenyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a benzofuranyl group, a benzothiophenyl group, an indolyl group, a dibenzofuranyl group, a dibenzothiophenyl group, a carbazolyl group, a benzocarbazolyl group, a carbonyl group, an indolocarbazolyl group, a benzofurocarbazolyl group, a benzothienocarbazolyl group, a dihydroindenocarbazolyl group, a benzoquinolinyl group, an acridinyl group, a dibenzoacridinyl group, a benzoimidazolyl group, an imidazopyridyl group, a benzoxazolyl group, a benzothiazolyl group or a phenanthrolinyl group. The naphthyridinyl group denotes any one of a 1,5-naphthyridinyl group, a 1,6-naphthyridinyl group, a 1,7-naphthyridinyl group, a 1,8-naphthyridinyl group, a 2,6-naphthyridinyl group and a 2,7-naphthyridinyl group. The heteroaryl group may or may not have a substituent. The number of carbon atoms in the heteroaryl group is not particularly limited, but is preferably within the range of 2 or more and 40 or less, more preferably within the range of 2 or more and 30 or less.

As heteroaryl groups with which B, C, L¹ and L² are optionally substituted, or with which the substituents thereof are optionally further substituted, a pyridyl group, a quinolinyl group, a pyrimidyl group, a triazinyl group, a quinoxalinyl group, a carbazolyl group and a dibenzofuranyl group are preferable, a pyridyl group, a quinolinyl group, a pyrimidyl group, a triazinyl group and a quinoxalinyl group are more preferable, and a pyridyl group and a quinolinyl group are especially preferable.

The electron-accepting nitrogen in the phrase "containing electron-accepting nitrogen" denotes a nitrogen atom which forms a multiple bond with an adjoining atom. Examples of the aromatic heterocyclic ring containing electron-accepting nitrogen include a pyridine ring, a pyridazine ring, a pyrimidine ring, pyrazine ring, a triazine ring, an oxadiazole ring, a thiazole ring, a quinoline ring, an isoquinoline ring, a naphthyridine ring, a cinnoline ring, a phthalazine ring, a quinazoline ring, a quinoxaline ring, a benzoquinoline ring, a phenanthroline ring, an acridine ring, a benzothiazole ring, and a benzoxazole ring. The naphthyridine denotes any one of a 1,5-naphthyridine, 1,6-naphthyridine, 1,7-naphthyridine, 1,8-naphthyridine, 2,6-naphthyridine and 2,7-naphthyridine.

The amino group is a substituted or unsubstituted amino group. Examples of the substituent with which the amino group is optionally substituted include an aryl group, a heteroaryl group, a linear alkyl group and a branched alkyl group, and among them, an aryl group and a heteroaryl group are preferable. More specifically, a phenyl group, a naphthyl group, a fluorenyl group, a phenanthryl group, an anthracenyl group, a pyrenyl group, a fluoranthenyl group, a triphenylenyl group, a benzofluoranthenyl group, a pyridyl group, a quinolinyl group, a pyrimidyl group, a triazinyl group, a dibenzofuranyl group, a dibenzothiophenyl group and a carbazolyl group are preferable, and a phenyl group, a naphthyl group, a fluorenyl group, an anthracenyl group, a pyrenyl group, a fluoranthenyl group, a pyridyl group, a quinolinyl group, a dibenzofuranyl group, a dibenzothiophenyl group, a carbazolyl group and a phenanthrolinyl group are more preferable. A phenyl group, a naphthyl group, a pyridyl group and a quinolinyl group are especially preferable. These substituents may be further substituted. The number of carbon atoms is not particularly limited, but is preferably within the range of 2 or more and 50 or less, more preferably within the range of 6 or more and 40 or less, especially preferably within the range of 6 or more and 30 or less.

The halogen denotes an atom selected from fluorine, chlorine, bromine, and iodine.

For example, the silyl group denotes an alkylsilyl group such as a trimethylsilyl group, a triethylsilyl group, a tert-butyldimethylsilyl group, a propyldimethylsilyl group or a vinyldimethylsilyl group, or an arylsilyl group such as a phenyldimethylsilyl group, a tert-butyldiphenylsilyl group, a triphenylsilyl group or a trinaphthylsilyl group. The substituent on silicon may be further substituted. The number of carbon atoms in the silyl group is not particularly limited, but is preferably within the range of 1 or more and 30 or less.

The boryl group is a substituted or unsubstituted boryl group. Examples of the substituent with which the boryl group is optionally substituted include an aryl group, a heteroaryl group, a linear alkyl group, a branched alkyl group, an aryl ether group, an alkoxy group and a hydroxy group, and among them, an aryl group and an aryl ether group are preferable.

The carbonyl group, the carboxyl group, the oxycarbonyl group and the carbamoyl group may or may not have a substituent. Here, examples of the substituent include an alkyl group, a cycloalkyl group, an aryl group and a heteroaryl group, and these substituents may be further substituted.

The phosphine oxide group -P(=O)R⁹R¹⁰ is not particularly limited, and specific examples include those of the following formulae.

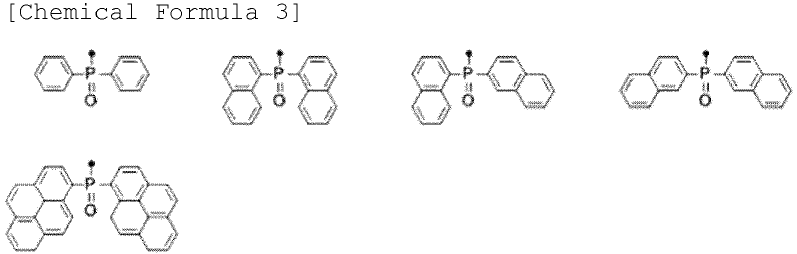

The phenanthroline skeleton has high stability to charges, so that reduction by electrons and oxidation by holes can be smoothly repeatedly performed. Thus, when the phenanthroline derivative of the present invention exhibits high charge stability, and hardly causes electrochemical degeneration when used in a light emitting element. Degradation of a material and a change in charge transporting property due to electrochemical degeneration hardly occur, and therefore the life of a light emitting element can be improved.

In some light emitting elements, some of holes injected into an emissive layer may reach an electron transporting layer without being recombined, resulting in deterioration of durability of the light emitting element. The phenanthroline derivative of the present invention has a large band gap derived from a phenanthroline skeleton, and therefore when the phenanthroline derivative is used for an electron transporting layer that is in contact with an emissive layer, a difference in ionization potential energy increases at an interface with the emissive layer, leading to exhibition of high hole blocking property. The phenanthroline derivative also has high charge durability, and therefore high durability to a hole attack is exhibited, so that the life of the element can be improved.

The phenanthroline derivative of the present invention contains only one phenanthroline skeleton, and therefore has good heat resistance during sublimation purification. A compound containing a plurality of phenanthroline skeletons has an increased sublimation temperature, so that a problem often arises in heat resistance during vacuum evaporation.

A substituent having high heat resistance, such as an aryl group or a heteroaryl group, can be introduced at a specific position in a phenanthroline skeleton to improve the heat resistance, reduce the crystallinity and increase the glass transition temperature of the compound. When the heat resistance is improved, decomposition of the material during preparation of the element can be suppressed, and therefore durability is improved. In addition, by reducing the crystallinity, and increasing the glass transition temperature, thin-film stability can be improved. When thin-film stability is improved, degeneration of the film is suppressed even when the light emitting element is driven for a long period of time, and therefore durability is improved.

By introduction of an aryl group or a heteroaryl group at a specific position in a phenanthroline skeleton, conjugation can be efficiently expanded, and therefore the charge transporting property of the compound is improved.

An aryl group and a heteroaryl group are substituents having high electrochemical stability, and therefore by introduction of these substituents, excellent electrochemical stability and charge durability can be imparted to the compound. When the compound has high electrochemical stability and charge durability, defects due to degeneration of a material, etc. do not occur, and thus the durability of the light emitting element is improved.

The phenanthroline derivative of the present invention has a substituted or unsubstituted heteroaryl group containing electron-accepting nitrogen in a group represented by L¹-B. Since a nitrogen atom has high electronegativity, a multiple bond between the nitrogen atom and an adjacent atom has an electron-accepting nature. For this reason, a group represented by L¹-B has high electron affinity, and contributes to the electron transporting property of a molecule as a whole.

Electron-accepting nitrogen has an unshared electron pair on a nitrogen atom, and therefore exhibits high coordination property to a metal atom. For this reason, a group represented by L¹-B has high metal coordination property. The phenanthroline skeleton is known to have high metal coordination property, and since a group represented by L¹-B is adjacent to the phenanthroline skeleton, higher metal coordination property can be exhibited. It is preferred that L¹ is a single bond because further high metal coordination property can be exhibited.

Further, a group represented by L¹-B has a moderate degree of freedom of rotation, rigidity is suppressed, so that high coordination property can be exhibited to various kinds of metals.

Thus, when the phenanthroline derivative represented by the general formula (1) is used for an electron transporting layer of the light emitting element, coordination to a metal serving as a cathode is facilitated, and therefore the interaction with the cathode is enhanced. Enhancement of the interaction with the cathode promotes electron injection property from the cathode, so that the driving voltage of the light emitting element can be reduced. In addition, since supply of electrons to an emissive layer is increased and a recombining probability is increased, luminous efficiency is improved.

Further, the phenanthroline derivative represented by the general formula (1) can strongly interact with a substance containing a metal element. The phenanthroline derivative can strongly interact particularly with alkali metal elements and alkali earth metal elements, and therefore favorably interacts with substances containing an alkali metal element and substances containing an alkali earth metal element, such as lithium, cesium, calcium, barium, LiF, CaF₂, CaO, BaO and lithium quinolinol. Thus, for example, when the phenanthroline derivative represented by the general formula (1) is used for the electron transporting layer of the light emitting element, and a substance containing an alkali metal element or a substance containing an alkali earth metal element is mixed in this layer, an electron transporting ability is improved, so that the driving voltage of the light emitting element can be reduced.

An electron transporting layer in which a substance containing a metal element, particularly a substance containing an alkali metal element or a substance containing an alkali earth metal element is mixed with the phenanthroline derivative represented by the general formula (1) as described above can also be suitably used as an N-type charge generation layer in a tandem structure type element in which a plurality of light emitting elements are coupled. The N-type charge generation layer including the phenanthroline derivative represented by the general formula (1) exhibits an excellent electron transporting ability, and therefore exhibits an efficient charge separating ability when coming into contact with a P-type charge generation layer, so that the driving voltage of the light emitting element can be reduced. As a result, luminous efficiency of the light emitting element can be improved, and also durability of the light emitting element can be improved.

Further, when the phenanthroline derivative represented by the general formula (1) is used for an electron extraction layer of a photoelectric conversion element, extraction of electrons in a cathode is promoted, and therefore the conversion efficiency and the on-off ratio of the photoelectric conversion element can be improved.

Examples of the heteroaryl group containing electron-accepting nitrogen that is suitably used in the present invention include a pyridyl group, a quinolinyl group, an isoquinolinyl group, a pyrazinyl group, a pyrimidyl group, a pyridazinyl group, a triazinyl group, a naphthyridinyl group, a cinnolinyl group, a quinoxalinyl group, a quinazolinyl group, a benzoquinolinyl group, an acridinyl group, a dibenzoacridinyl group, a benzoimidazolyl group, an imidazopyridyl group, a benzoxazolyl group, and a benzothiazolyl group. Particularly, a pyridyl group, a quinolinyl group, an isoquinolinyl group, a pyrazinyl group, a pyrimidyl group, a pyridazinyl group, a triazinyl group, a quinoxalinyl group, a quinazolinyl group, a benzoquinolinyl group, an acridinyl group, a benzoimidazolyl group, an imidazopyridyl group, a benzoxazolyl group and a benzothiazolyl group are preferable, and a pyridyl group, a quinolinyl group, an isoquinolinyl group, a pyrazinyl group, a pyrimidyl group, a pyridazinyl group, a triazinyl group, a quinoxalinyl group, a quinazolinyl group, a benzoquinolinyl group, an acridinyl group, a benzoimidazolyl group, an imidazopyridyl group, a benzoxazolyl group and a benzothiazolyl group, each of which contains only nitrogen as a hetero atom, are more preferable. Among them, a pyridyl group, a quinolinyl group, an isoquinolinyl group, a pyrimidyl group, a triazinyl group, a quinazolinyl group, a benzoquinolinyl group and an imidazopyridyl group are preferable, a pyridyl group, a quinolinyl group and an isoquinolinyl group are more preferable, and a pyridyl group and a quinolinyl group are especially preferable.

The phenanthroline derivative of the present invention has an aryl group having an aryl group having less than 20 ring-forming carbon atoms in a group represented by L²-C. Due to existence of an aryl group having high flatness and relatively wide π conjugation, molecules are well superimposed on one another, so that high charge transporting property can be exhibited. Thus, an aryl group having moderately wide π conjugation is preferable. An aryl group having an excessively large number of ring-forming carbon atoms is not preferable because it causes excessive superimposition of π conjugation planes between molecules, so that crystallinity is increased, leading to deterioration of thin-film stability.

The phenanthroline derivative of the present invention exhibits proper carrier mobility and electron-accepting property due to existence of an aryl group having moderate bulkiness and moderately wide π conjugation. As a result, carriers for electrons and holes can be well balanced in the light emitting element, so that durability of the light emitting element can be improved. It is preferred that L² is a phenylene group because a moderate space is generated between a phenanthroline skeleton and an aryl group represented by C, and therefore molecules are well oriented, so that desirable carrier mobility and electron-accepting property can be exhibited.

Examples of preferable aryl groups having less than 20 ring-forming carbon atoms are not particularly limited, and specific examples thereof include the following.

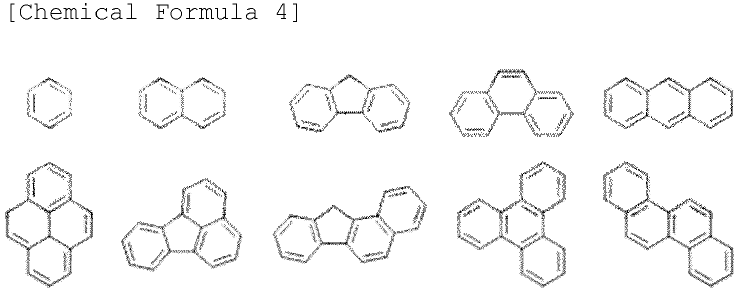

Particularly, a phenyl group, a naphthyl group, a fluorenyl group, a phenanthryl group, an anthracenyl group, a pyrenyl group, a fluoranthenyl group and a triphenylenyl group are preferable, and a naphthyl group, a fluorenyl group, a phenanthryl group, a pyrenyl group and a fluoranthenyl group are more preferable. Among them, a fluorenyl group, a phenanthryl group, a pyrenyl group, a triphenylenyl group and a fluoranthenyl group are preferable because they have moderately wide π conjugation, and the triplet energy level is not excessively low. When the triplet energy level is excessively low, the triplet exciton blocking function is reduced, so that luminous efficiency is deteriorated when the compound is combined with a phosphorescence emitting material. A phenanthryl group, a pyrenyl group and a fluoranthenyl group are preferable, and a pyrenyl group and a fluoranthenyl group are especially preferable.

An aryl group represented by C and a heteroaryl group represented by B are substituents having different polarities, and when these groups are asymmetrically introduced, the intramolecular dipole moment increases, so that charge transporting property can be further improved. The increase in intramolecular dipole moment also contributes to orientation of molecules, so that molecules can be moderately oriented to exhibit high charge transporting property. Since the molecule is asymmetric, the glass transition temperature increases, and thin-film stability is improved. It is preferred that a group represented by L¹-B is different from a group represented by L²-C because the intramolecular dipole moment further increases.

Preferable combinations of B, C, L¹ and L² include, but are not particularly limited to, the following combinations. Here, R¹¹ represents an alkyl group, an aryl group or a heteroaryl group. B, C, L¹, L² and R¹¹ each optionally have a substituent. "-" denotes a single bond.

| [Chemical Formula 5] | | | |
|---|---|---|---|
| B | L¹ | L² | C |
| 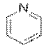 | - | - | 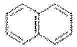 |
| 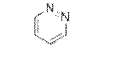 | - | - | 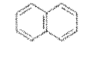 |
| 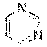 | - | - | 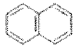 |
| 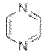 | - | - | 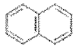 |
| 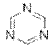 | - | - | 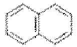 |
| 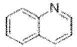 | - | - | 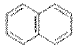 |
| 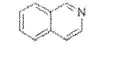 | - | - | 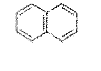 |
| 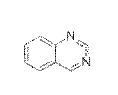 | - | - | 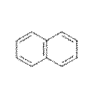 |
| 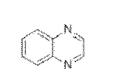 | - | - | 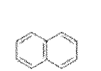 |
| 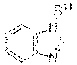 | - | - | 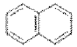 |
| 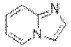 | - | - | 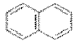 |
| 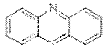 | - | - | 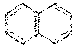 |
| 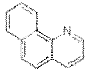 | - | - | 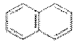 |

| [Chemical Formula 6] | | | |
|---|---|---|---|
| B | L¹ | L² | C |
| 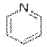 | - | 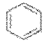 | 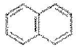 |
| 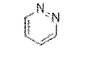 | - | 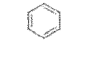 | 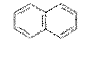 |
| 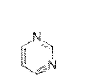 | - | 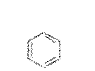 | 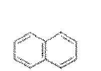 |
| 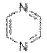 | - | 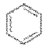 | 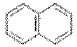 |
| 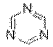 | - | 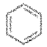 | 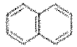 |
| 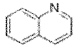 | - | 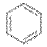 | 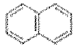 |
| 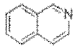 | - | 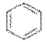 | 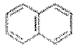 |
| 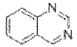 | - | 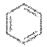 | 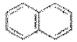 |
| 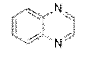 | - | 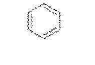 | 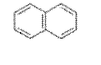 |
| 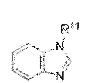 | - | 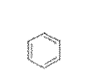 | 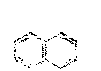 |
| 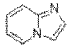 | - | 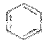 | 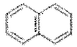 |
| 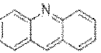 | - | 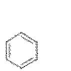 | 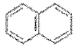 |
| 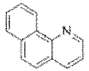 | - | 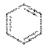 | 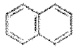 |

| [Chemical Formula 7] | | | |
|---|---|---|---|
| B | L¹ | L² | C |
| 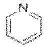 | 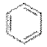 | - | 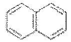 |
| 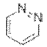 | 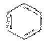 | - | 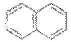 |
| 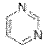 | 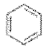 | - | 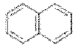 |
| 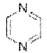 | 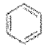 | - | 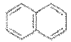 |
| 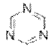 | 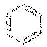 | - | 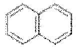 |
| 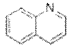 | 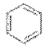 | - | 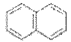 |
| 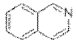 | 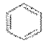 | - | 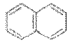 |
| 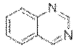 | 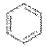 | - | 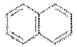 |
| 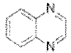 | 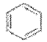 | - | 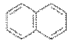 |
| 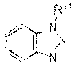 | 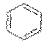 | - | 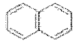 |
| 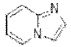 | 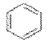 | - | 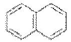 |
| 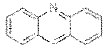 | 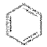 | - | 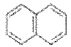 |
| 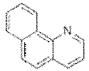 | 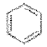 | - | 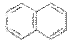 |

| [Chemical Formula 8] | | | |
|---|---|---|---|
| B | L¹ | L² | C |
| 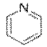 | 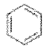 | 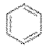 | 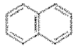 |
| 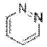 | 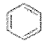 | 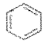 | 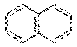 |
| 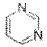 | 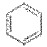 | 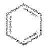 | 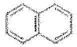 |
| 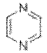 | 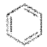 | 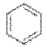 | 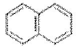 |
| 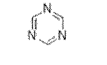 | 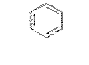 | 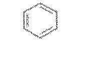 | 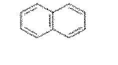 |
| 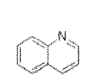 | 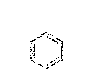 | 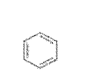 | 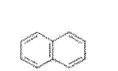 |
| 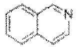 | 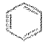 | 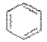 | 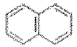 |
| 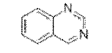 | 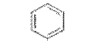 | 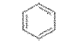 | 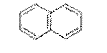 |
| 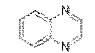 | 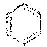 | 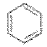 | 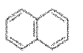 |
| 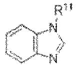 | 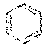 | 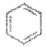 | 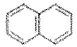 |
| 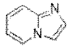 | 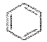 | 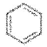 | 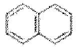 |
| 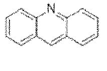 | 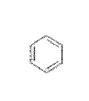 | 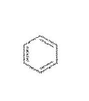 | 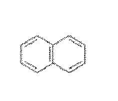 |
| 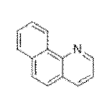 | 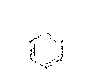 | 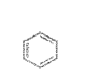 | 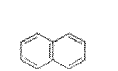 |

| [Chemical Formula 9] | | | |
|---|---|---|---|
| B | L¹ | L² | C |
| 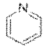 | - | - | 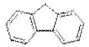 |
| 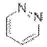 | - | - | 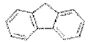 |
| 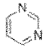 | - | - | 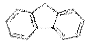 |
| 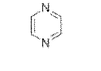 | - | - | 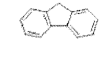 |
| 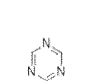 | - | - | 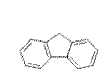 |
| 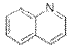 | - | - | 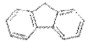 |
| 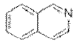 | - | - | 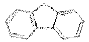 |
| 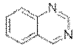 | - | - | 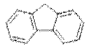 |
| 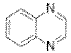 | - | - | 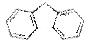 |
| 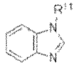 | - | - | 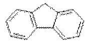 |
| 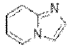 | - | - | 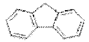 |
| 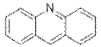 | - | - | 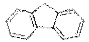 |
| 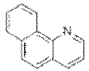 | - | - | 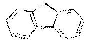 |

| [Chemical Formula 10] | | | |
|---|---|---|---|
| B | L¹ | L² | C |
| 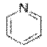 | - | 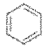 | 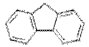 |
| 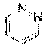 | - | 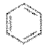 | 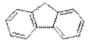 |
| 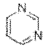 | - | 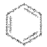 | 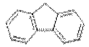 |
| 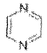 | - | 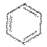 | 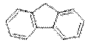 |
| 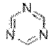 | - | 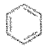 | 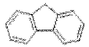 |
| 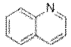 | - | 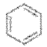 | 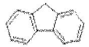 |
| 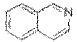 | - | 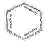 | 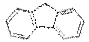 |
| 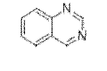 | - | 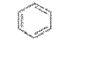 | 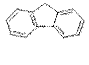 |
| 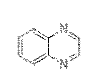 | - | 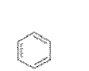 | 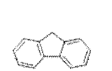 |
| 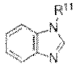 | - | 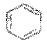 | 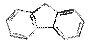 |
| 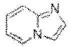 | - | 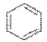 | 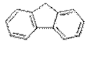 |
| 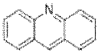 | - | 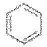 | 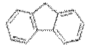 |
| 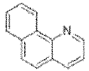 | - | 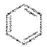 | 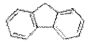 |

| [Chemical Formula 11] | | | |
|---|---|---|---|
| B | L¹ | L² | C |
| 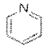 | 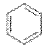 | - | 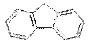 |
| 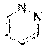 | 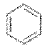 | - | 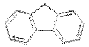 |
| 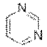 | 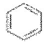 | - | 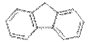 |
| 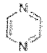 | 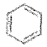 | - | 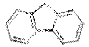 |
| 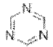 | 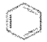 | - | 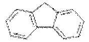 |
| 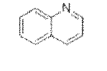 | 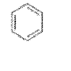 | - | 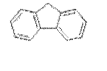 |
| 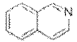 | 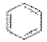 | - | 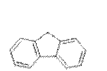 |
| 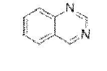 | 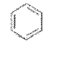 | - | 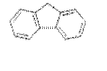 |
| 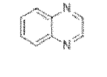 | 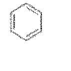 | - | 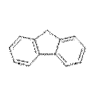 |
| 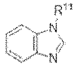 | 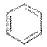 | - | 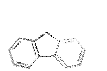 |
| 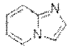 | 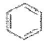 | - | 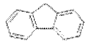 |
| 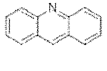 | 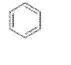 | - | 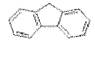 |
| 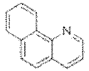 | 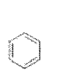 | - | 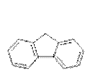 |

| [Chemical Formula 12] | | | |
|---|---|---|---|
| B | L¹ | L² | C |
| 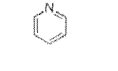 | 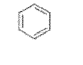 | 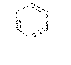 | 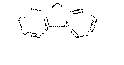 |
| 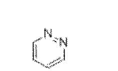 | 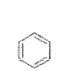 | 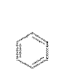 | 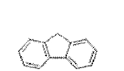 |
| 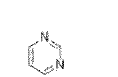 | 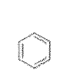 | 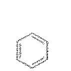 | 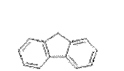 |
| 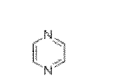 | 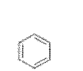 | 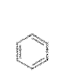 | 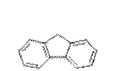 |
| 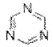 | 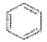 | 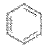 | 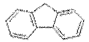 |
| 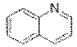 | 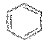 | 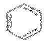 | 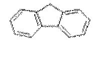 |
| 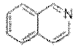 | 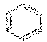 | 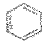 | 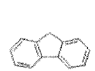 |
| 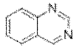 | 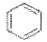 | 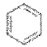 | 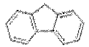 |
| 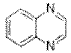 | 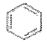 | 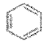 | 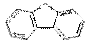 |
| 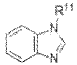 | 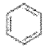 | 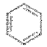 | 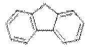 |
| 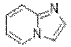 | 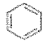 | 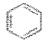 | 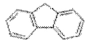 |
| 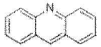 | 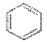 | 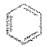 | 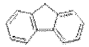 |
| 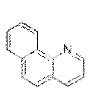 | 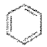 | 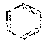 | 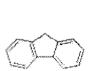 |

| [Chemical Formula 13] | | | |
|---|---|---|---|
| B | L¹ | L² | C |
| 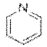 | - | - | 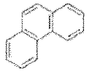 |
| 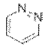 | - | - | 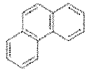 |
| 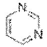 | - | - | 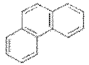 |
| 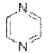 | - | - | 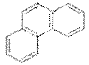 |
| 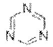 | - | - | 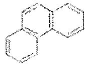 |
| 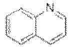 | - | - | 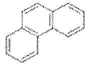 |
| 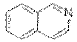 | - | - | 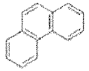 |
| 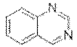 | - | - | 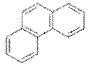 |
| 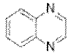 | - | - | 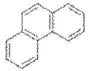 |
| 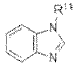 | - | - | 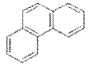 |
| 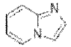 | - | - | 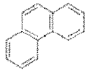 |
| 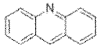 | - | - | 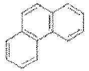 |
| 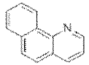 | - | - | 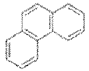 |

| [Chemical Formula 14] | | | |
|---|---|---|---|
| B | L¹ | L² | C |
| 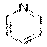 | - | 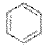 | 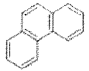 |
| 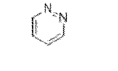 | - | 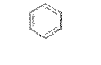 | 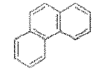 |
| 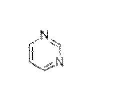 | - | 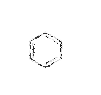 | 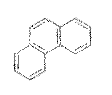 |
| 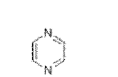 | - | 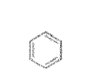 | 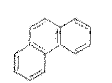 |
| 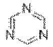 | - | 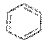 | 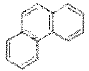 |
| 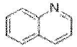 | - | 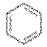 | 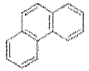 |
| 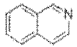 | - | 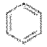 | 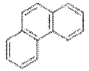 |
| 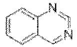 | - | 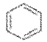 | 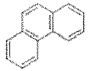 |
| 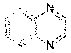 | - | 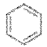 | 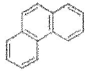 |
| 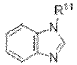 | - | 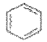 | 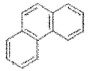 |
| 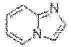 | - | 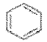 | 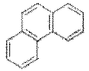 |
| 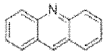 | - | 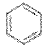 | 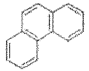 |
| 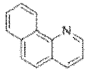 | - | 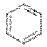 | 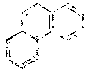 |

| [Chemical Formula 15] | | | |
|---|---|---|---|
| B | L¹ | L² | C |
| 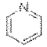 | 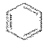 | - | 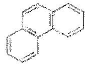 |
| 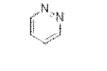 | 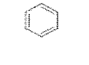 | - | 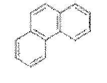 |
| 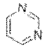 | 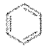 | - | 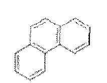 |
| 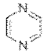 | 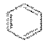 | - | 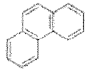 |
| 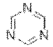 | 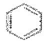 | - | 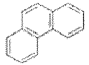 |
| 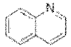 | 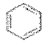 | - | 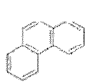 |
| 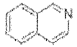 | 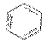 | - | 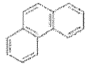 |
| 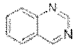 | 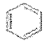 | - | 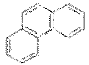 |
| 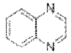 | 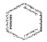 | - | 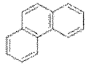 |
| 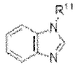 | 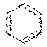 | - | 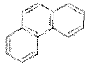 |
| 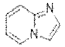 | 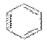 | - | 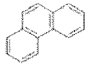 |
| 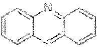 | 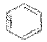 | - | 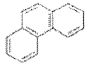 |
| 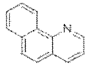 | 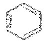 | - | 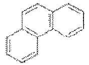 |

| [Chemical Formula 16] | | | |
|---|---|---|---|
| B | L¹ | L² | C |
| 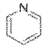 | 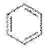 | 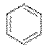 | 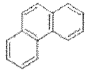 |
| 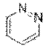 | 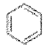 | 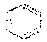 | 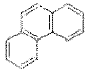 |
| 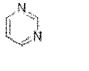 | 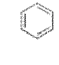 | 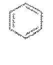 | 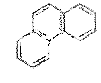 |
| 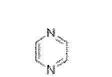 | 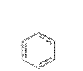 | 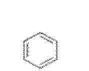 | 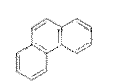 |
| 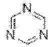 | 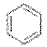 | 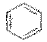 | 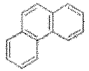 |
| 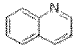 | 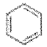 | 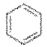 | 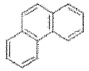 |
| 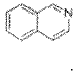 | 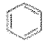 | 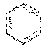 | 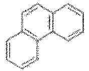 |
| 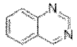 | 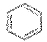 | 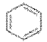 | 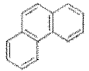 |
| 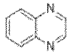 | 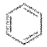 | 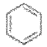 | 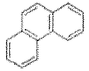 |
| 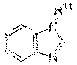 | 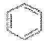 | 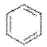 | 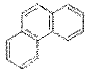 |
| 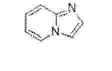 | 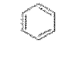 | 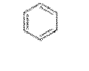 | 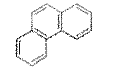 |
| 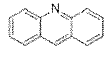 | 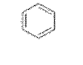 | 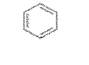 | 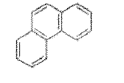 |
| 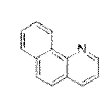 | 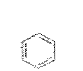 | 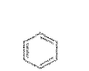 | 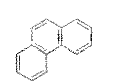 |

| [Chemical Formula 17] | | | |
|---|---|---|---|
| B | L¹ | L² | C |
| 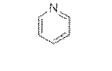 | - | - | 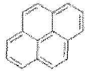 |
| 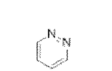 | - | - | 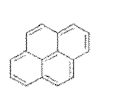 |
| 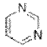 | - | - | 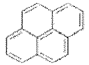 |
| 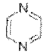 | - | - | 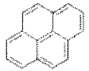 |
| 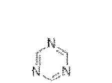 | - | - | 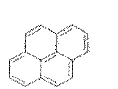 |
| 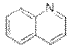 | - | - | 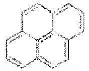 |
| 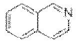 | - | - | 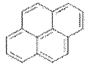 |
| 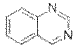 | - | - | 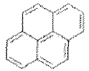 |
| 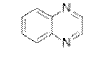 | - | - | 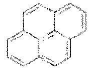 |
| 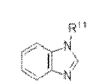 | - | - | 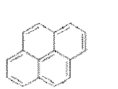 |
| 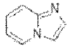 | - | - | 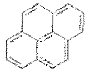 |
| 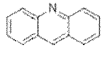 | - | - | 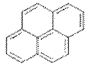 |
| 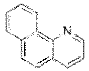 | - | - | 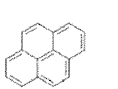 |

| [Chemical Formula 18] | | | |
|---|---|---|---|
| B | L¹ | L² | C |
| 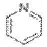 | - | 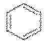 | 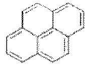 |
| 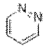 | - | 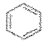 | 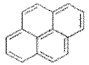 |
| 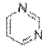 | - | 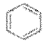 | 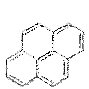 |
| 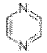 | - | 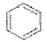 | 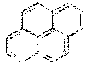 |
| 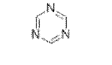 | - | 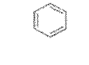 | 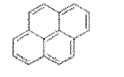 |
| 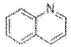 | - | 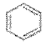 | 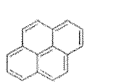 |
| 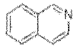 | - | 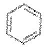 | 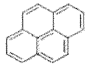 |
| 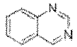 | - | 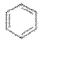 | 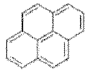 |
| 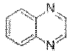 | - | 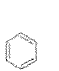 | 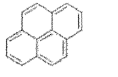 |
| 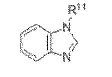 | - | 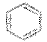 | 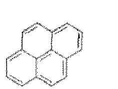 |
| 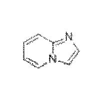 | - | 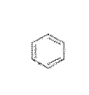 | 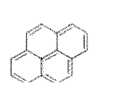 |
| 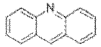 | - | 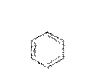 | 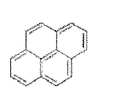 |
| 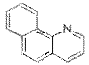 | - | 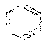 | 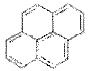 |

| [Chemical Formula 19] | | | |
|---|---|---|---|
| B | L¹ | L² | C |
| 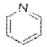 | 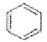 | - | 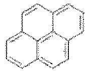 |
| 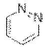 | 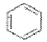 | - | 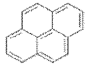 |
| 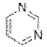 | 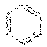 | - | 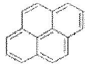 |
| 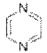 | 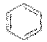 | - | 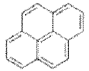 |
| 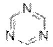 | 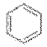 | - | 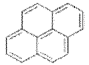 |
| 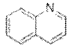 | 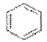 | - | 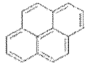 |
| 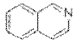 | 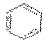 | - | 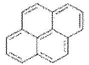 |
| 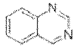 | 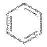 | - | 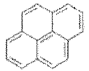 |
| 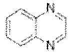 | 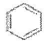 | - | 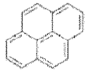 |
| 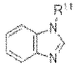 | 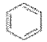 | - | 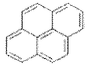 |
| 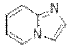 | 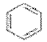 | - | 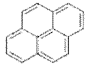 |
| 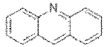 | 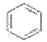 | - | 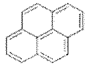 |
| 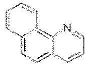 | 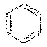 | - | 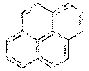 |

| [Chemical Formula 20] | | | |
|---|---|---|---|
| B | L¹ | L² | C |
| 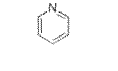 | 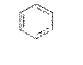 | 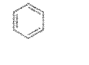 | 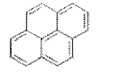 |
| 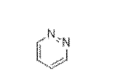 | 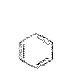 | 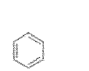 | 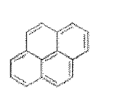 |
| 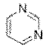 | 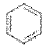 | 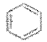 | 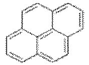 |
| 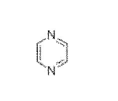 | 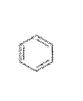 | 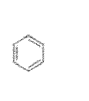 | 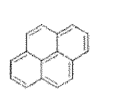 |
| 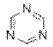 | 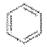 | 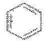 | 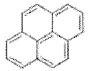 |
| 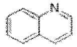 | 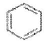 | 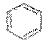 | 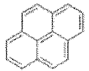 |
| 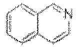 | 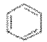 | 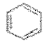 | 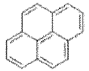 |
| 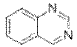 | 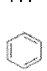 | 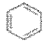 | 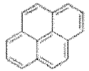 |
| 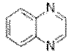 | 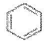 | 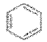 | 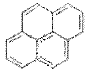 |
| 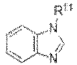 | 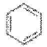 | 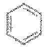 | 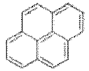 |
| 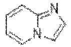 | 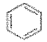 | 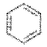 | 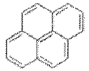 |
| 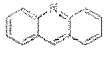 | 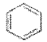 | 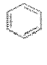 | 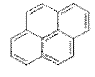 |
| 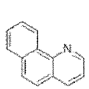 | 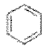 | 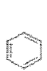 | 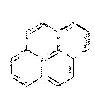 |

| [Chemical Formula 21] | | | |
|---|---|---|---|
| B | L¹ | L² | C |
| 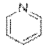 | - | - | 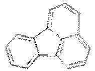 |
| 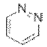 | - | - | 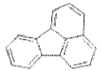 |
| 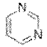 | - | - | 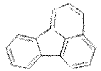 |
| 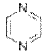 | - | - | 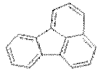 |
| 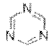 | - | - | 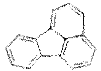 |
| 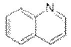 | - | - | 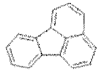 |
| 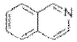 | - | - | 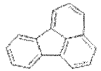 |
| 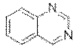 | - | - | 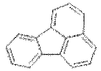 |
| 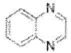 | - | - | 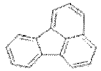 |
| 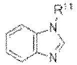 | - | - | 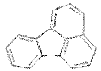 |
| 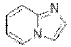 | - | - | 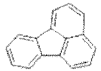 |
| 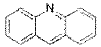 | - | - | 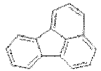 |
| 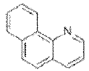 | - | - | 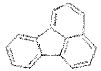 |

| [Chemical Formula 22] | | | |
|---|---|---|---|
| B | L¹ | L² | C |
| 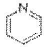 | - | 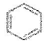 | 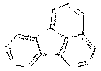 |
| 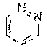 | - | 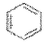 | 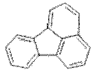 |
| 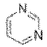 | - | 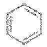 | 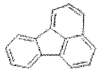 |
| 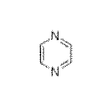 | - | 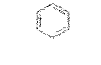 | 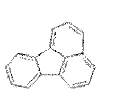 |
| 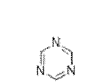 | - | 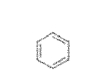 | 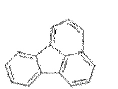 |
| 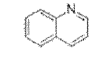 | - | 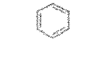 | 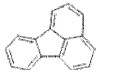 |
| 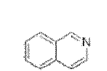 | - | 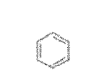 | 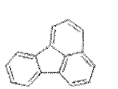 |
| 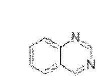 | - | 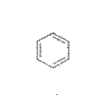 | 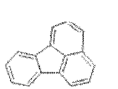 |
| 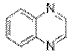 | - | 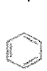 | 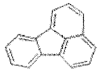 |
| 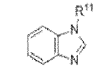 | - | 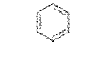 | 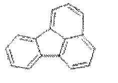 |
| 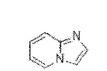 | - | 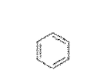 | 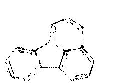 |
| 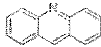 | - | 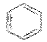 | 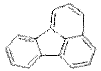 |
| 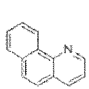 | - | 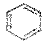 | 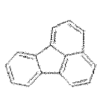 |

| [Chemical Formula 23] | | | |
|---|---|---|---|
| B | L¹ | L² | C |
| 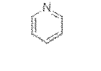 | 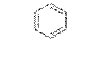 | - | 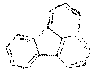 |
| 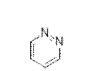 | 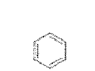 | - | 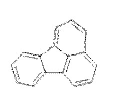 |
| 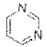 | 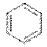 | - | 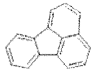 |
| 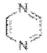 | 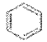 | - | 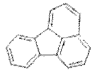 |
| 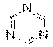 | 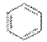 | - | 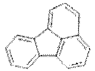 |
| 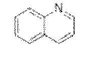 | 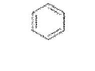 | - | 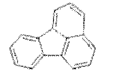 |
| 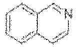 | 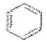 | - | 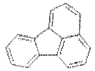 |
| 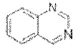 | 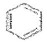 | - | 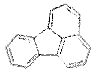 |
| 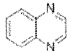 | 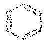 | - | 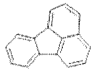 |
| 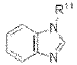 | 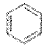 | - | 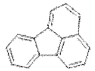 |
| 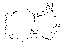 | 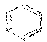 | - | 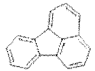 |
| 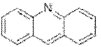 | 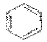 | - | 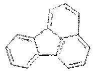 |
| 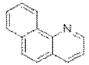 | 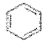 | - | 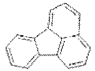 |

| [Chemical Formula 24] | | | |
|---|---|---|---|
| B | L¹ | L² | C |
| 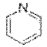 | 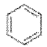 | 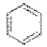 | 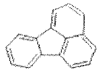 |
| 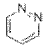 | 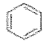 | 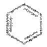 | 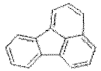 |
| 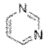 | 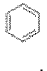 | 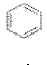 | 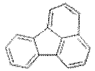 |
| 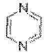 | 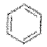 | 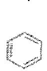 | 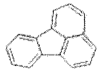 |
| 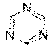 | 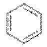 | 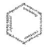 | 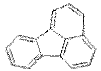 |
| 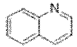 | 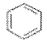 | 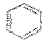 | 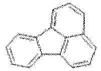 |
| 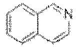 | 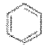 | 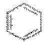 | 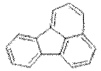 |
| 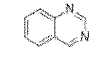 | 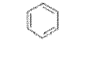 | 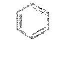 | 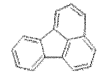 |
| 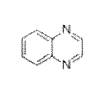 | 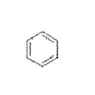 | 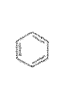 | 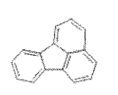 |
| 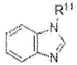 | 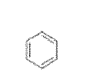 | 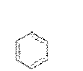 | 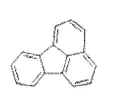 |
| 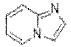 | 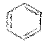 | 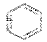 | 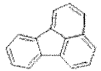 |
| 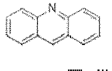 | 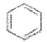 | 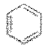 | 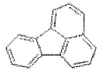 |
| 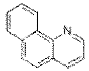 | 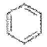 | 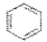 | 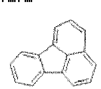 |

| [Chemical Formula 25] | | | |
|---|---|---|---|
| B | L¹ | L² | C |
| 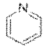 | - | - | 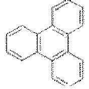 |
| 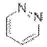 | - | - | 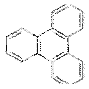 |
| 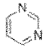 | - | - | 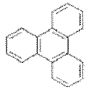 |
| 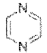 | - | - | 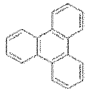 |
| 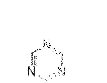 | - | - | 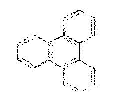 |
| 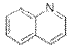 | - | - | 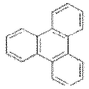 |
| 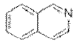 | - | - | 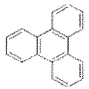 |
| 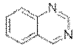 | - | - | 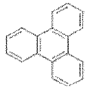 |
| 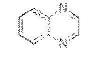 | - | - | 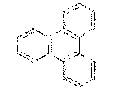 |
| 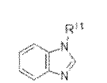 | - | - | 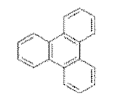 |
| 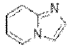 | - | - | 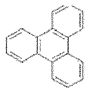 |
| 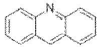 | - | - | 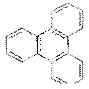 |
| 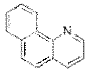 | - | - | 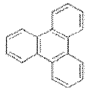 |

| [Chemical Formula 26] | | | |
|---|---|---|---|
| B | L¹ | L² | C |
| 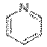 | - | 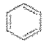 | 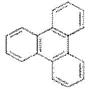 |
| 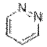 | - | 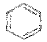 | 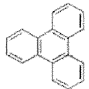 |
| 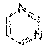 | - | 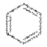 | 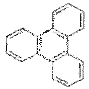 |
| 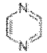 | - | 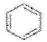 | 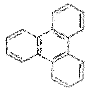 |
| 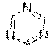 | - | 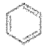 | 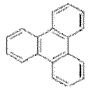 |
| 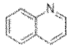 | - | 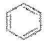 | 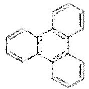 |
| 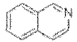 | - | 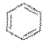 | 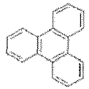 |
| 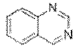 | - | 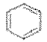 | 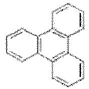 |
| 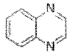 | - | 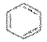 | 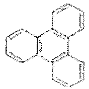 |
| 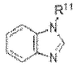 | - | 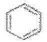 | 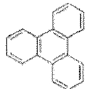 |
| 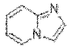 | - | 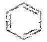 | 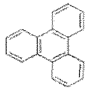 |
| 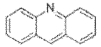 | - | 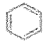 | 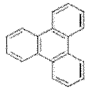 |
| 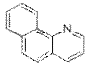 | - | 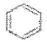 | 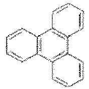 |

| [Chemical Formula 27] | | | |
|---|---|---|---|
| B | L¹ | L² | C |
| 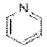 | 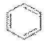 | - | 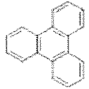 |
| 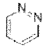 | 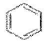 | - | 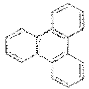 |
| 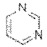 | 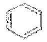 | - | 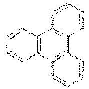 |
| 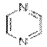 | 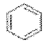 | - | 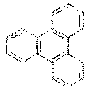 |
| 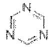 | 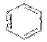 | - | 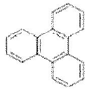 |
| 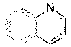 | 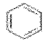 | - | 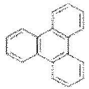 |
| 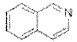 | 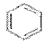 | - | 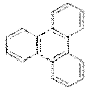 |
| 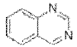 | 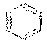 | - | 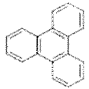 |
| 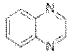 | 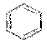 | - | 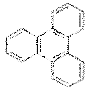 |
| 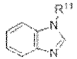 | 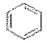 | - | 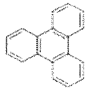 |
| 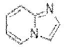 | 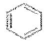 | - | 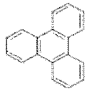 |
| 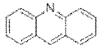 | 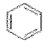 | - | 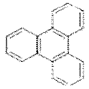 |
| 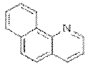 | 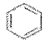 | - | 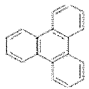 |

| [Chemical Formula 28] | | | |
|---|---|---|---|
| B | L¹ | L² | C |
| 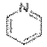 | 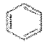 | 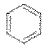 | 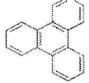 |
| 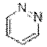 | 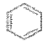 | 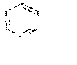 | 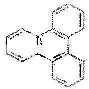 |
| 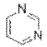 | 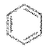 | 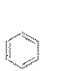 | 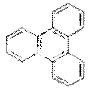 |
| 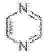 | 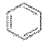 | 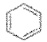 | 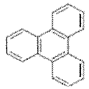 |
| 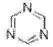 | 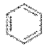 | 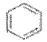 | 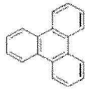 |
| 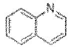 | 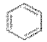 | 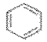 | 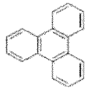 |
| 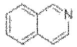 | 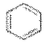 | 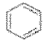 | 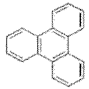 |
| 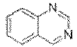 | 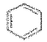 | 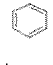 | 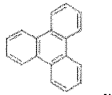 |
| 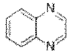 | 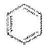 | 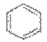 | 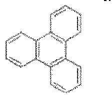 |
| 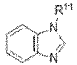 | 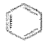 | 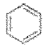 | 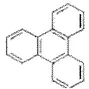 |
| 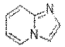 | 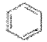 | 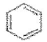 | 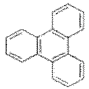 |
| 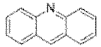 | 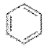 | 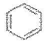 | 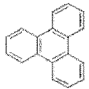 |
| 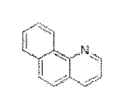 | 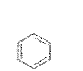 | 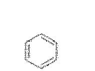 | 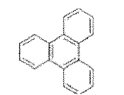 |

In the phenanthroline derivative represented by the general formula (1), any of R¹ and R² is a group represented by L¹-B. When two nitrogen atoms of 1,10-phenanthroline and the group L¹-B having electron-accepting nitrogen are in close contact with each other, higher electron affinity and metal coordination property are exhibited, and excellent electron transporting property and electron injection property are exhibited, so that the driving voltage of the light emitting element can be reduced. Particularly, R¹ is preferably a group represented by L¹-B.

Any of R⁷ and R⁸ is a group represented by L²-C. Specifically, the phenanthroline derivative represented by the general formula (1) has any of the following structures.

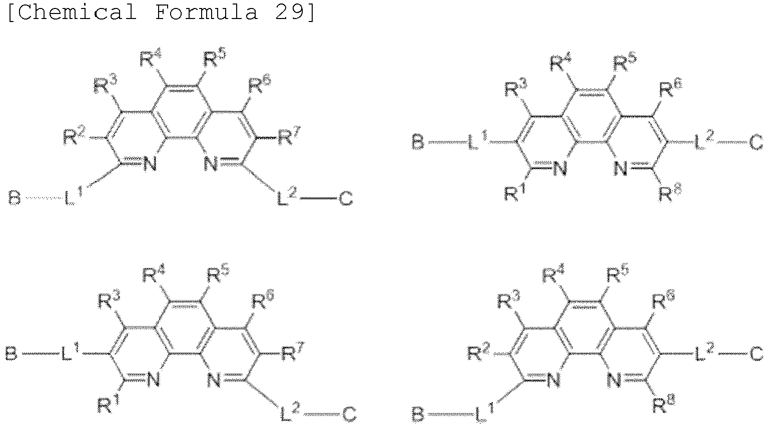

Particularly, it is preferred that R¹ is a group represented by L¹-B and R⁸ is a group represented by L²-C because the magnitude and the direction of the intramolecular dipole moment become appropriate, so that charge transporting property is further improved, and also thin-film stability is further improved. Here, a π plane of the phenanthroline skeleton is easily superimposed on other π plane, and therefore charge transporting property can be further improved.

The phenanthroline derivative of the present invention does not have either an aryl group or a heteroaryl group in R³ to R⁶ in the general formula (1). Accordingly, owing to high flatness and relatively wide π conjugation of the phenanthroline skeleton, molecules are well superimposed on one another, so that high charge transporting property can be exhibited.

It is not preferable that an aryl group and a heteroaryl group exist in R³ to R⁶ in the general formula (1) because charge transporting property is deteriorated, leading to an increase in driving voltage and a reduction in durability life of the element.

It is more preferable that all substituents on the phenanthroline skeleton, other than groups represented by L¹-B and L²-C, are hydrogen because a π plane of the phenanthroline skeleton is easily superimposed on other π plane.

Specifically, it is more preferable that the compound represented by the general formula (1) in the present invention has any of the following structures.

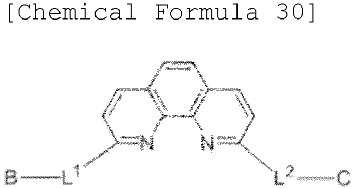

Thus, the phenanthroline derivative of the present invention exhibits good electron transporting property and high durability, and when the phenanthroline derivative is used in the light emitting element, a low driving voltage, high luminous efficiency and excellent durability life can be attained.

The molecular weight of the phenanthroline derivative of the present invention is not particularly limited, but is preferably 800 or less, more preferably 750 or less from the viewpoint of heat resistance and film formability. The molecular weight of the phenanthroline derivative is still more preferably 700 or less, especially preferably 650 or less. Generally, the glass transition temperature tends to increase as the molecular weight becomes larger, and the glass transition temperature increases, thin-film stability is improved. Thus, the molecular weight is preferably 400 or more, more preferably 450 or more. The molecular weight is still more preferably 500 or more.

The compound represented by the general formula (1) is not particularly limited, and specific examples include those of the following formulae.

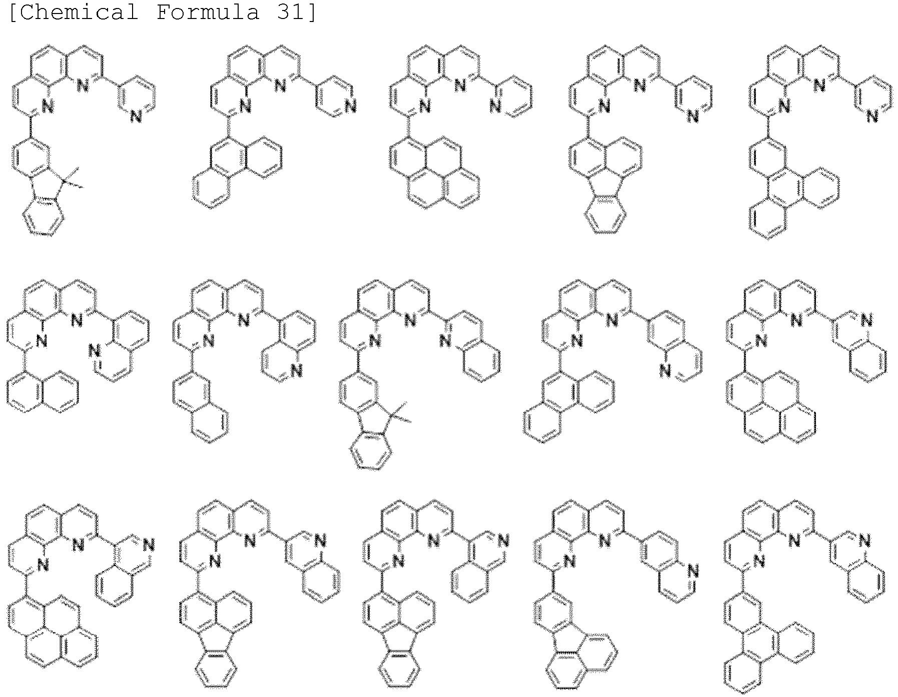

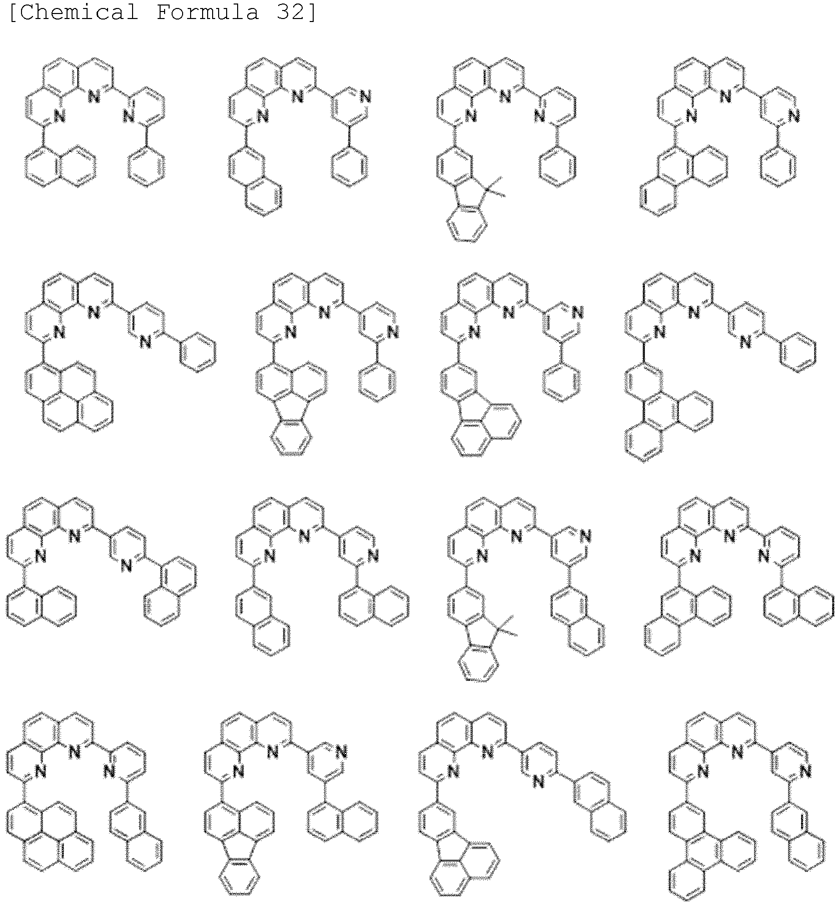

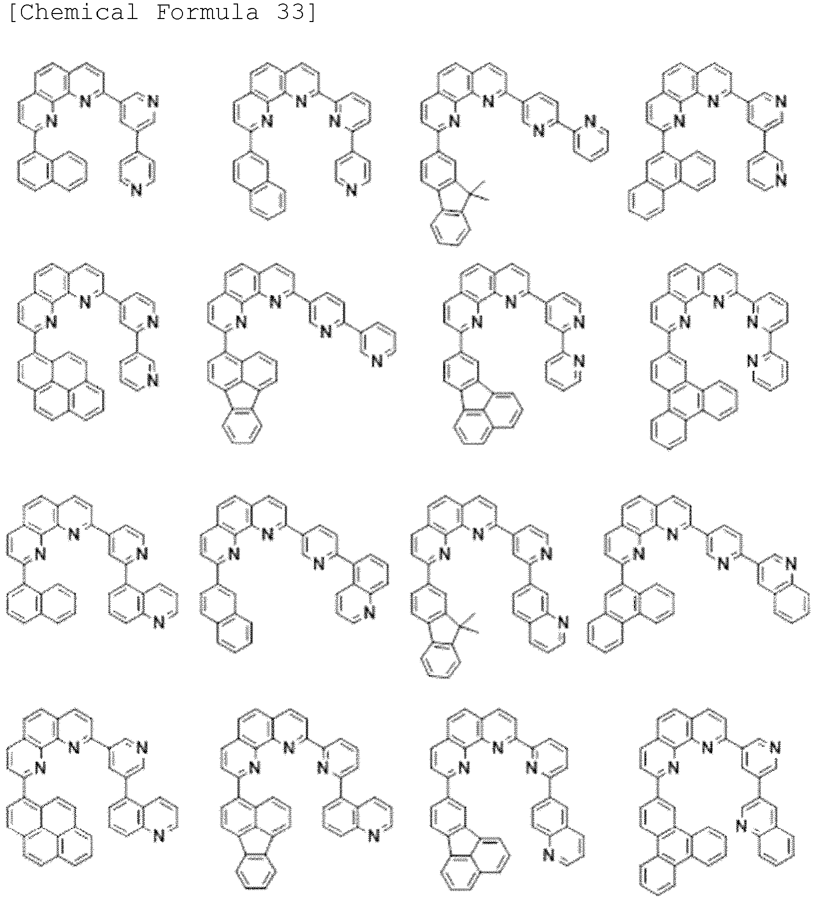

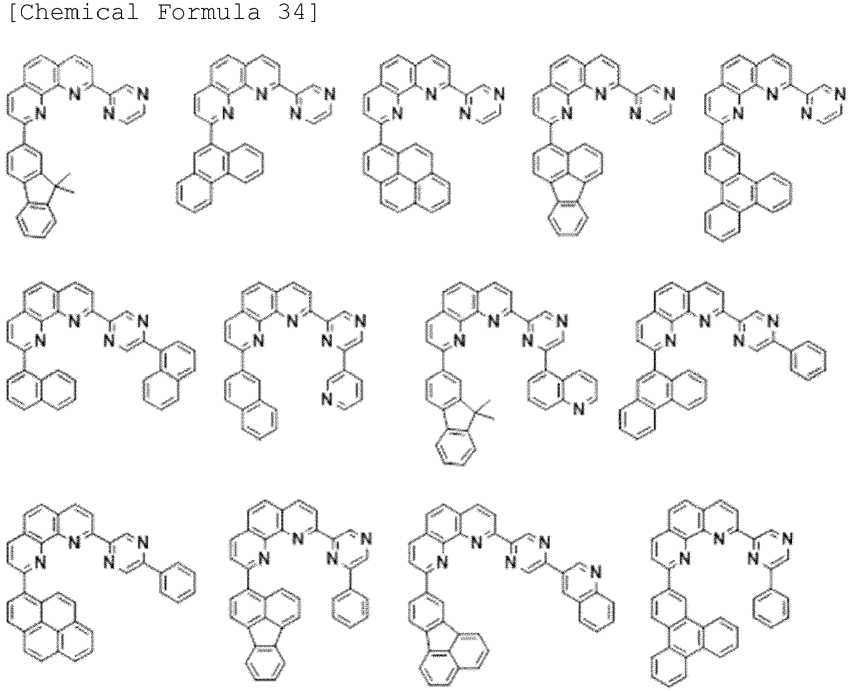

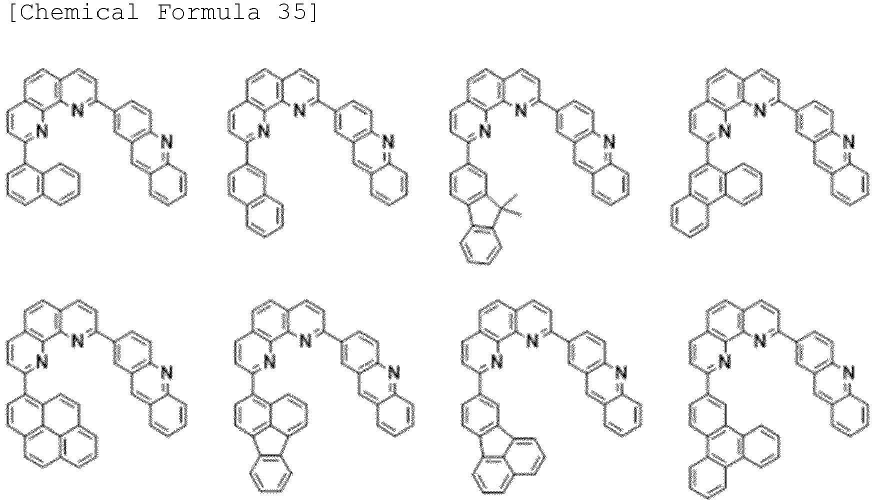

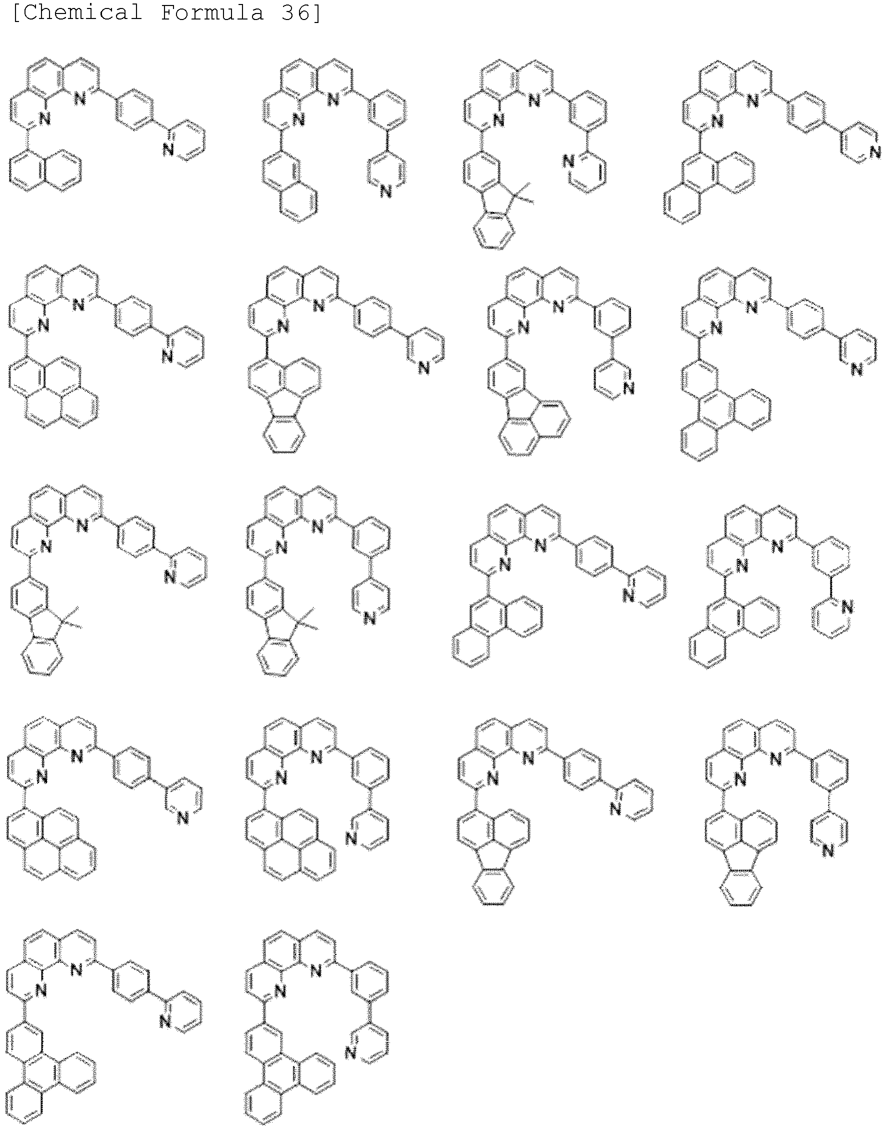

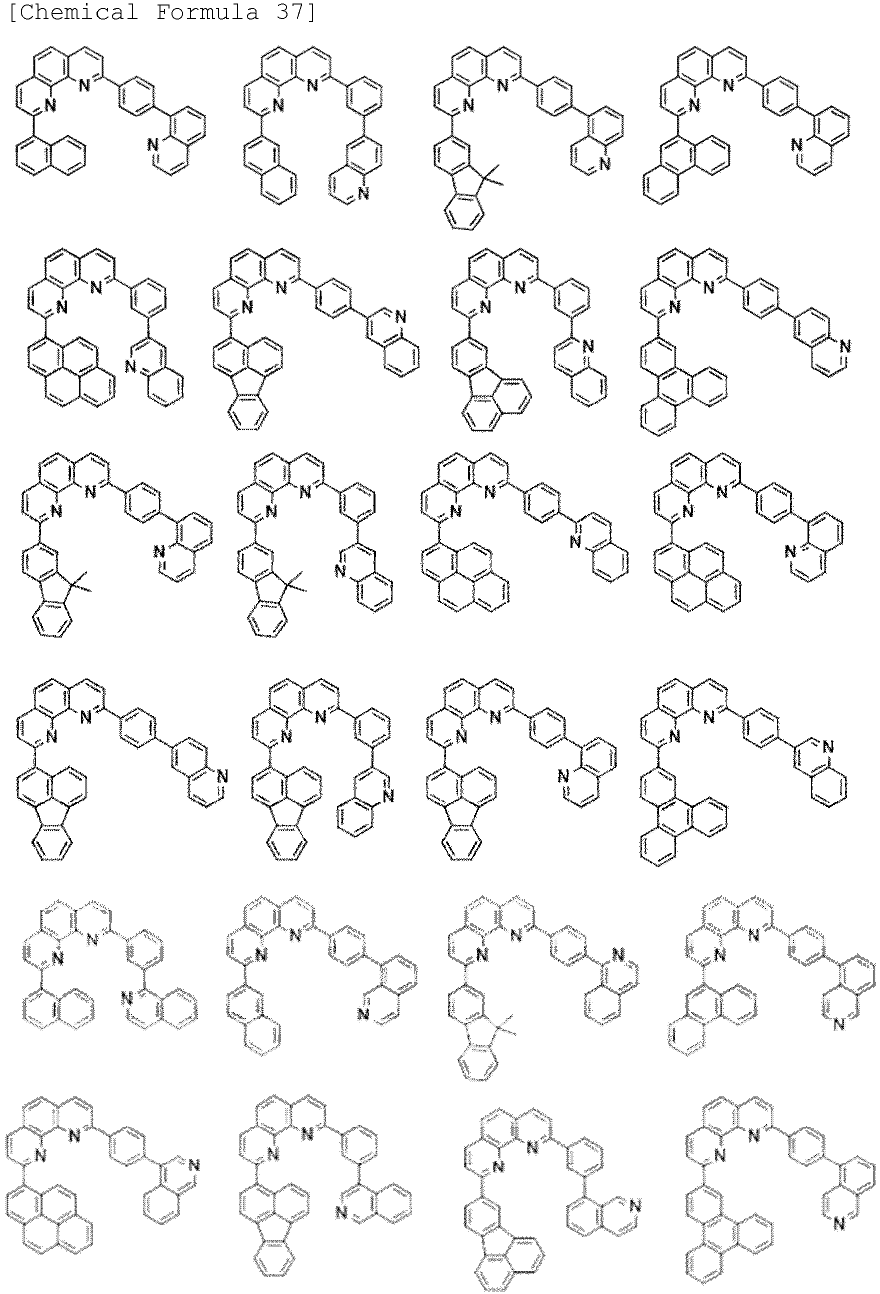

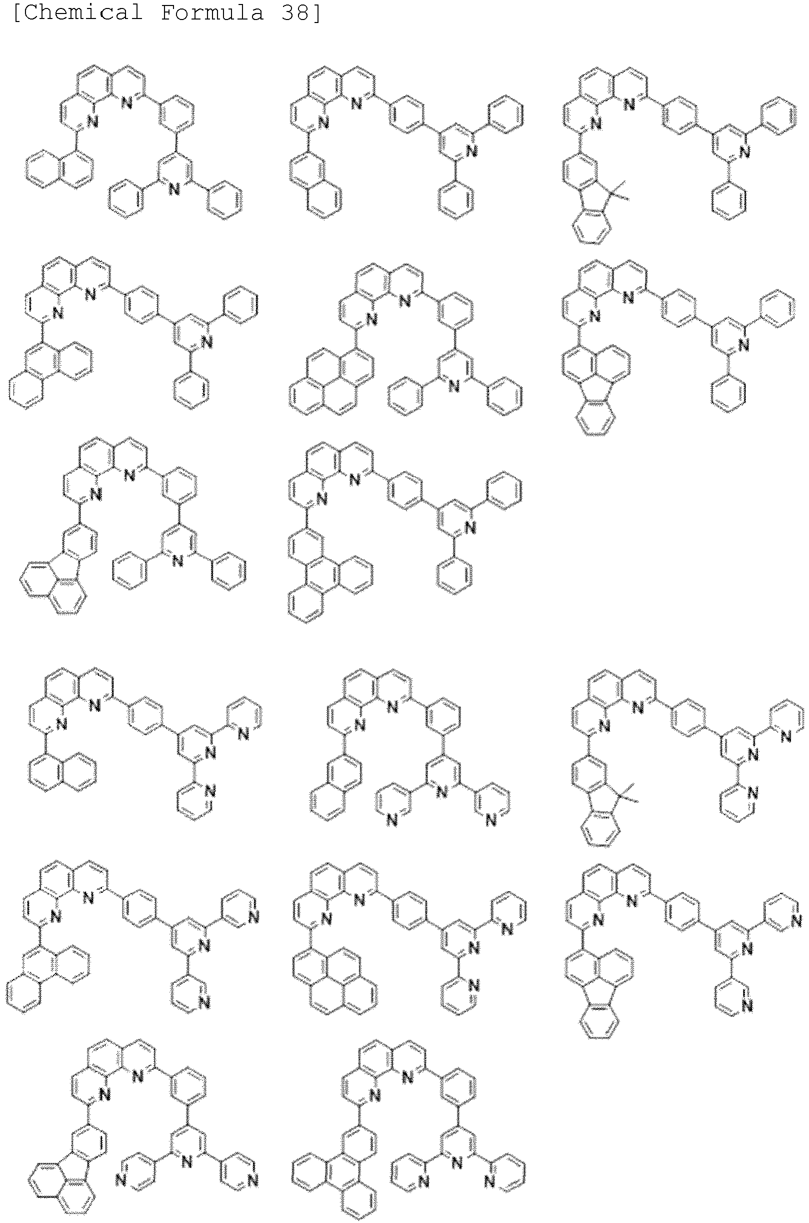

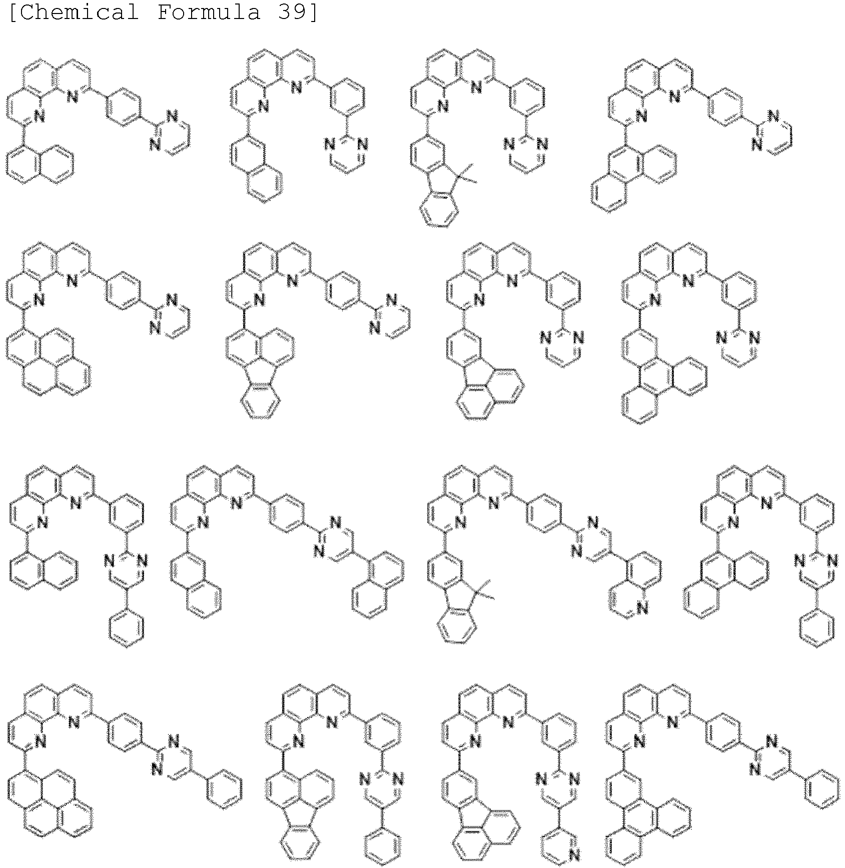

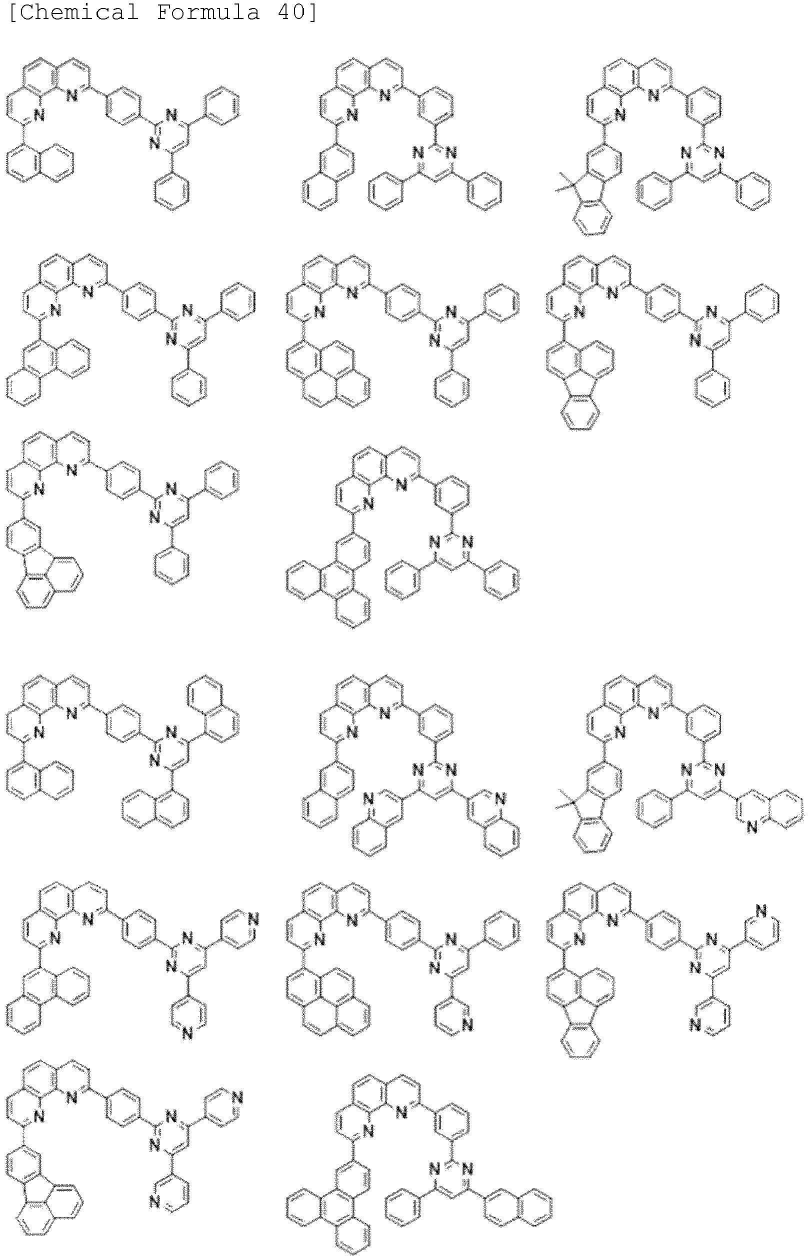

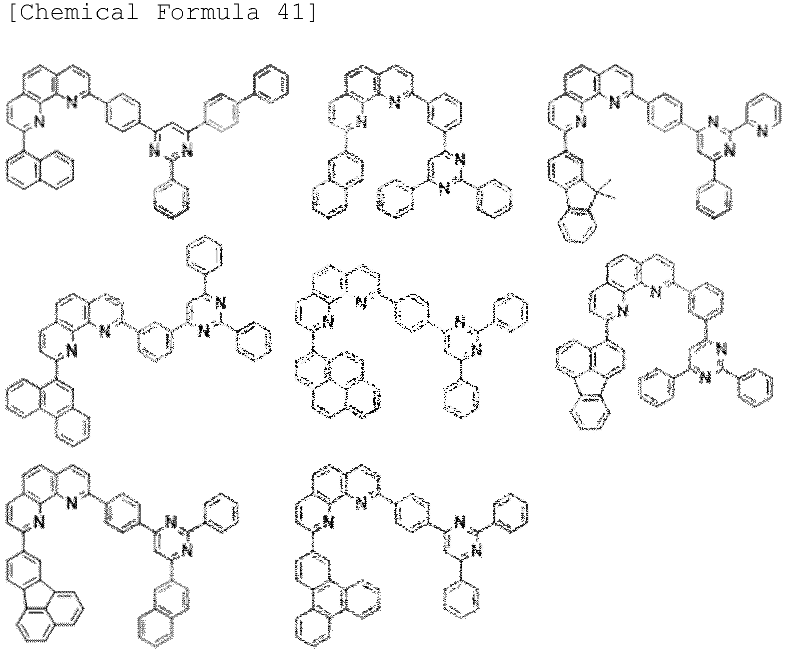

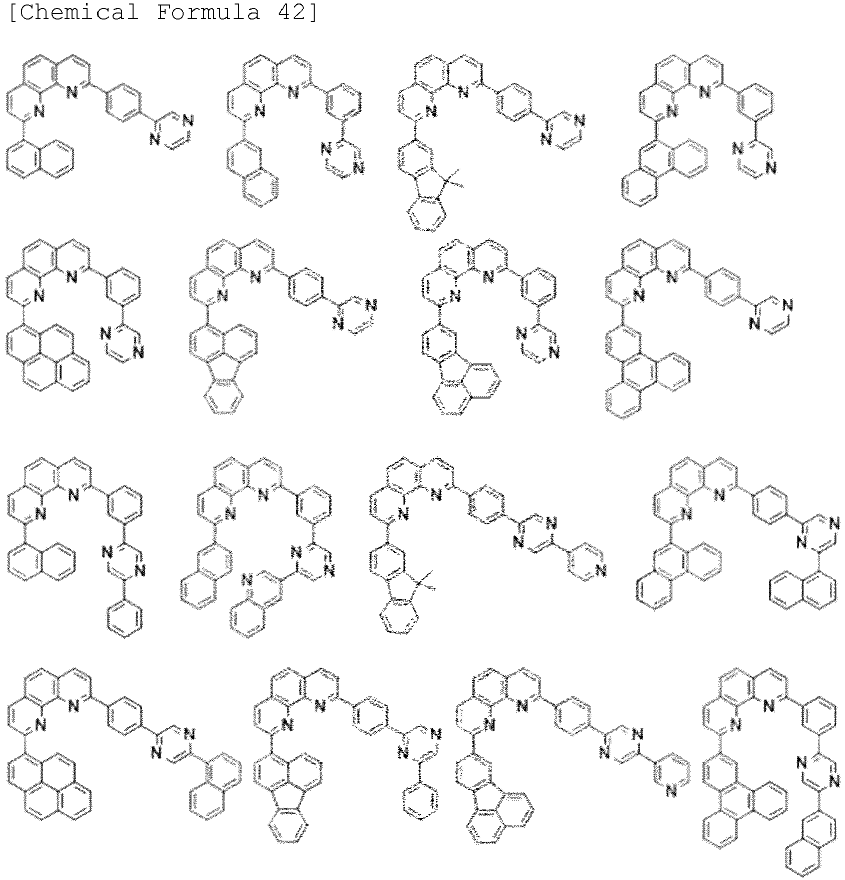

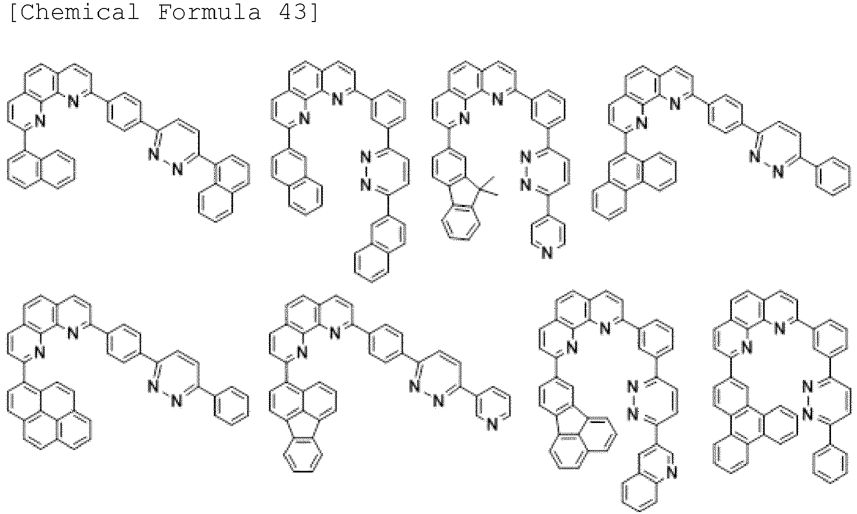

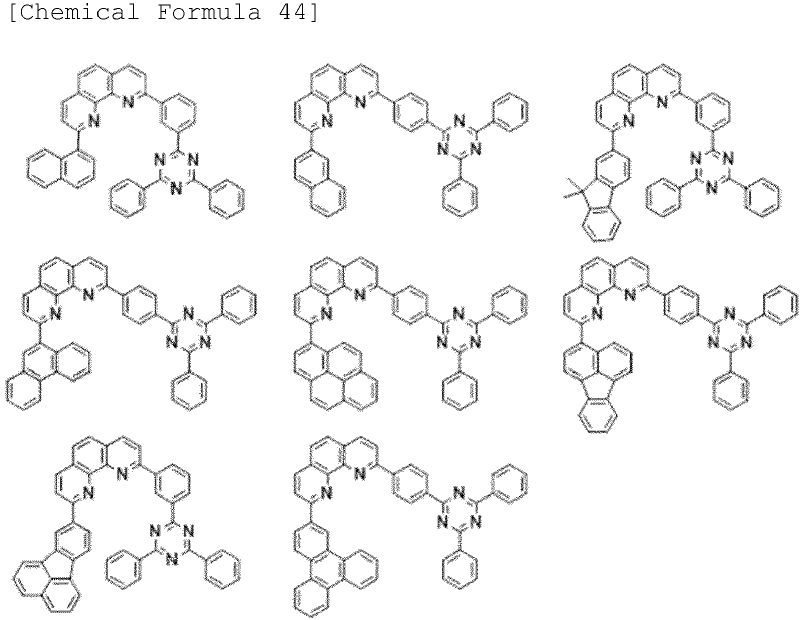

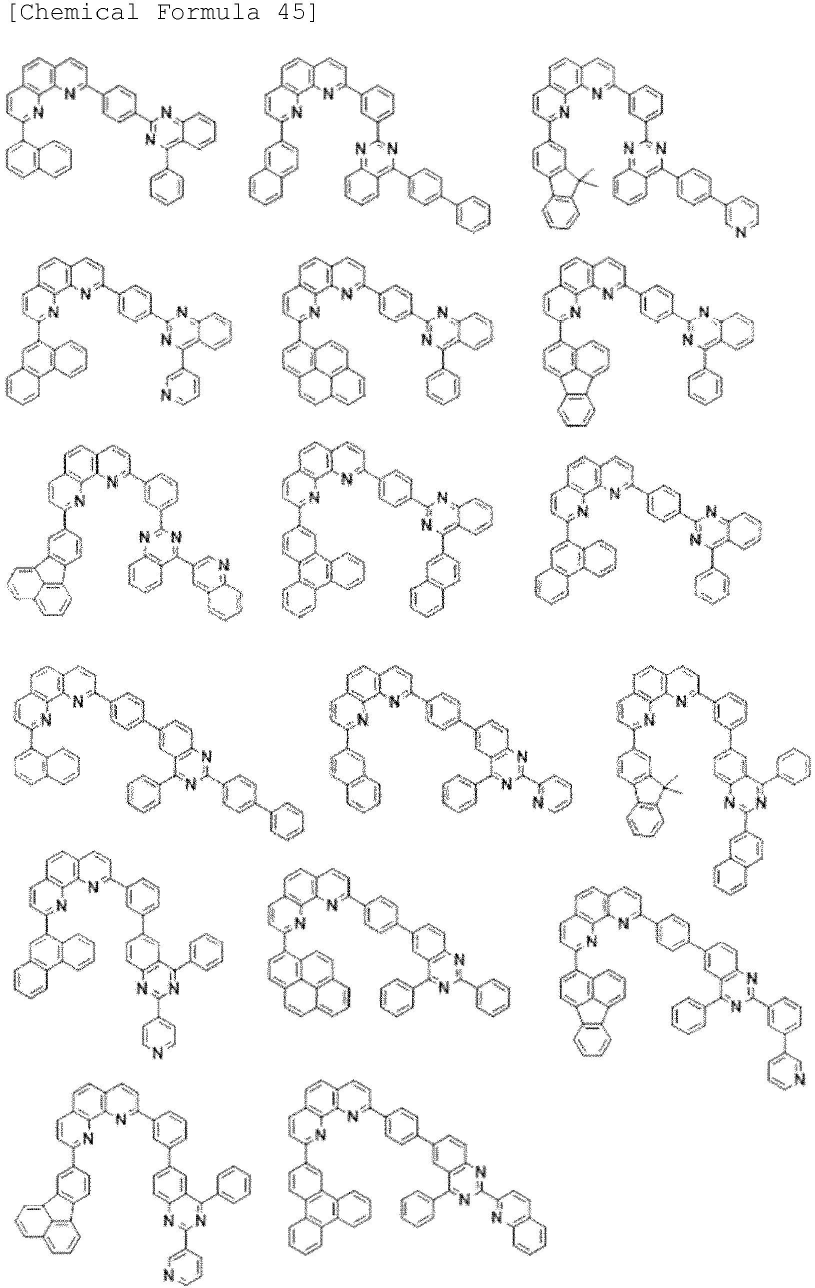

Formulas marked with "☆" do not fall under the scope of the present invention.

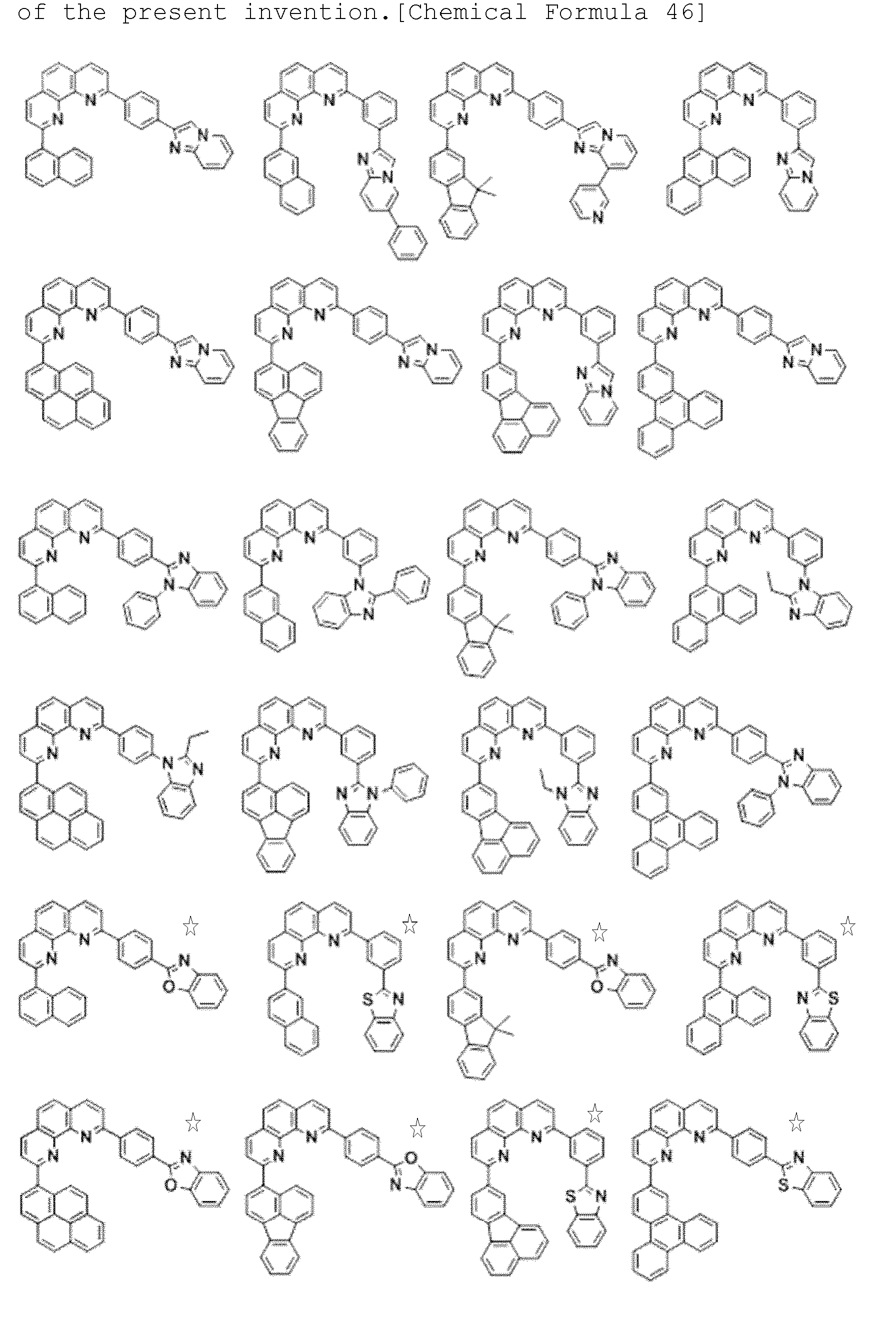

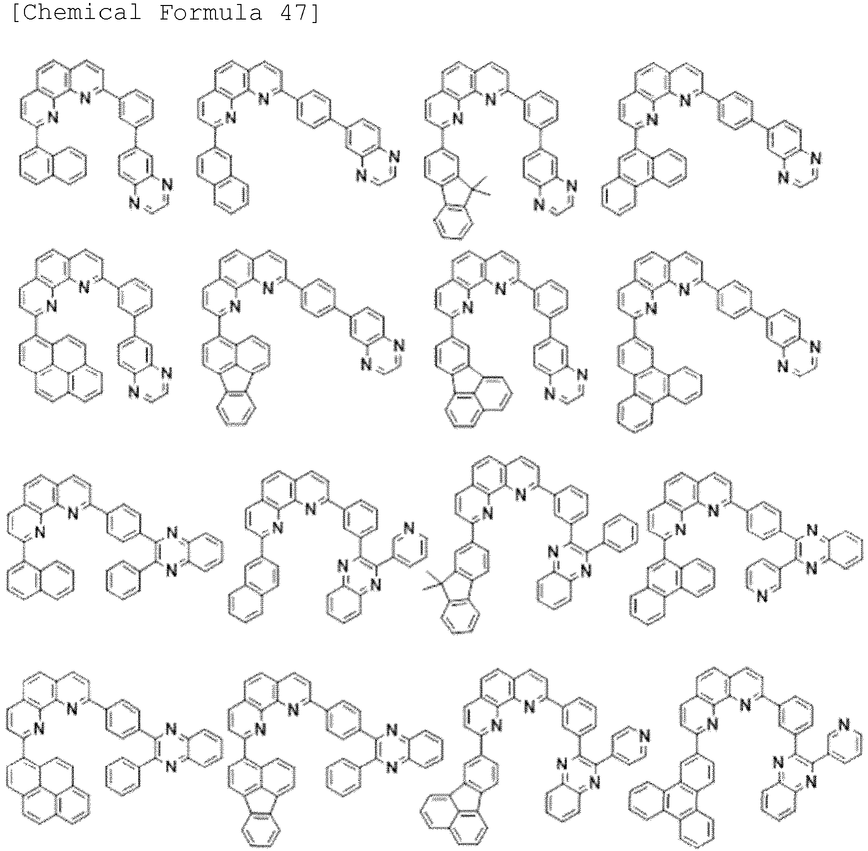

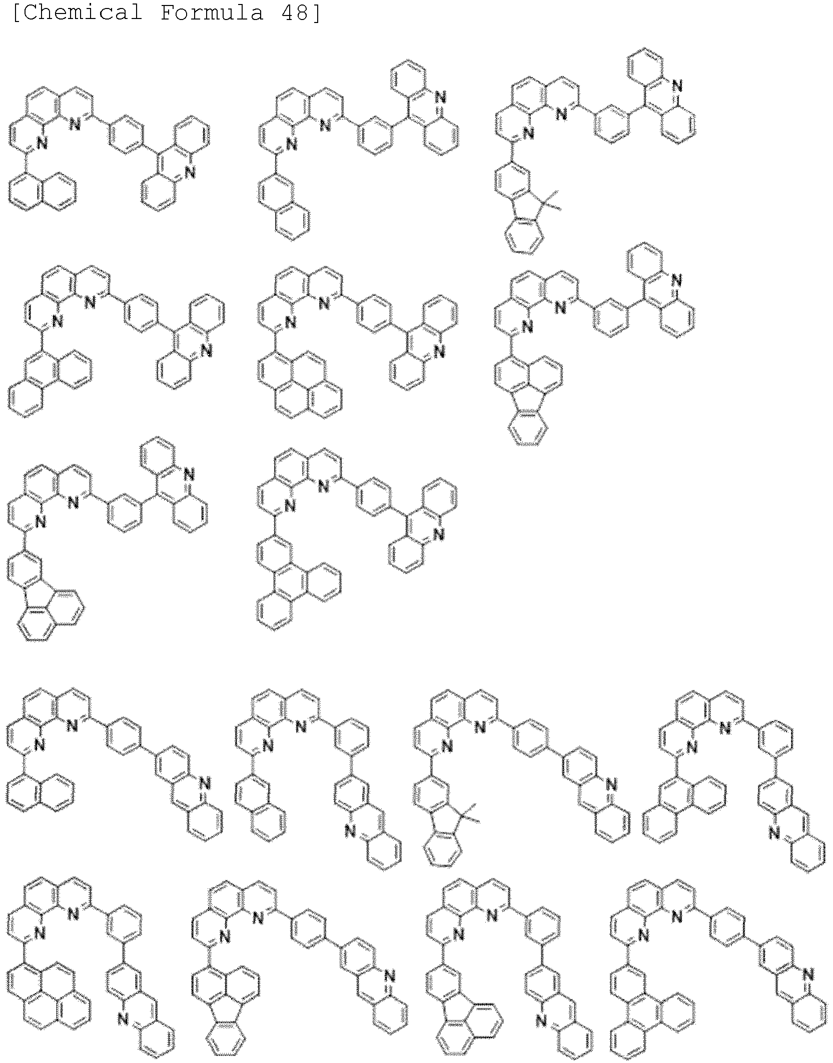

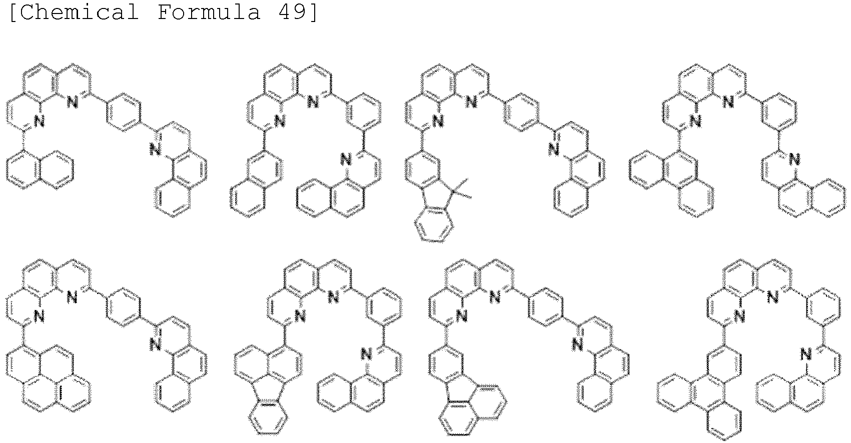

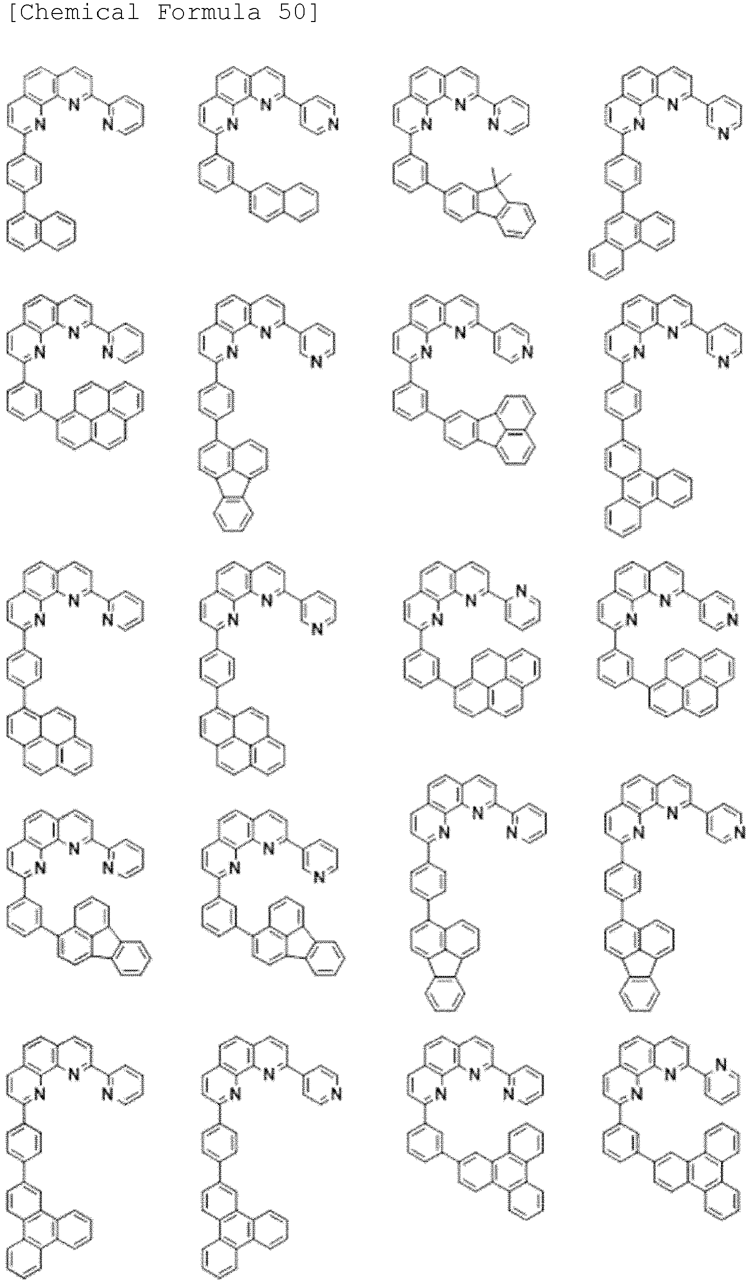

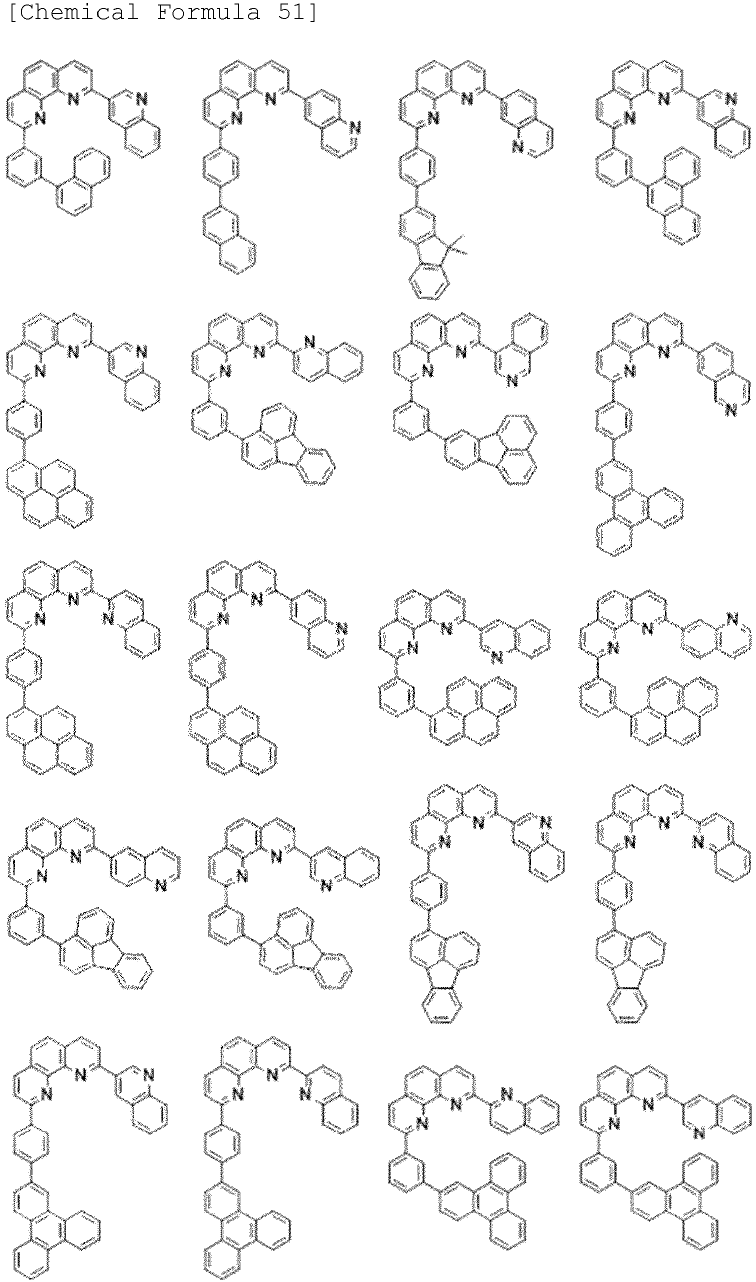

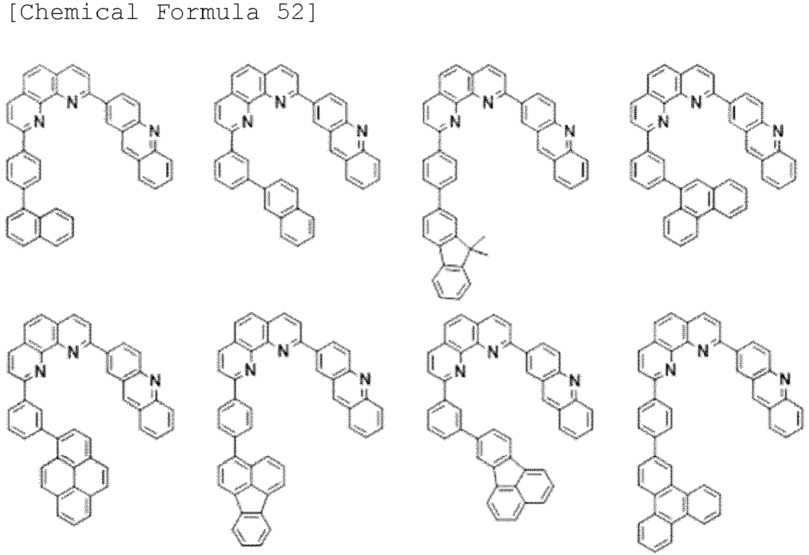

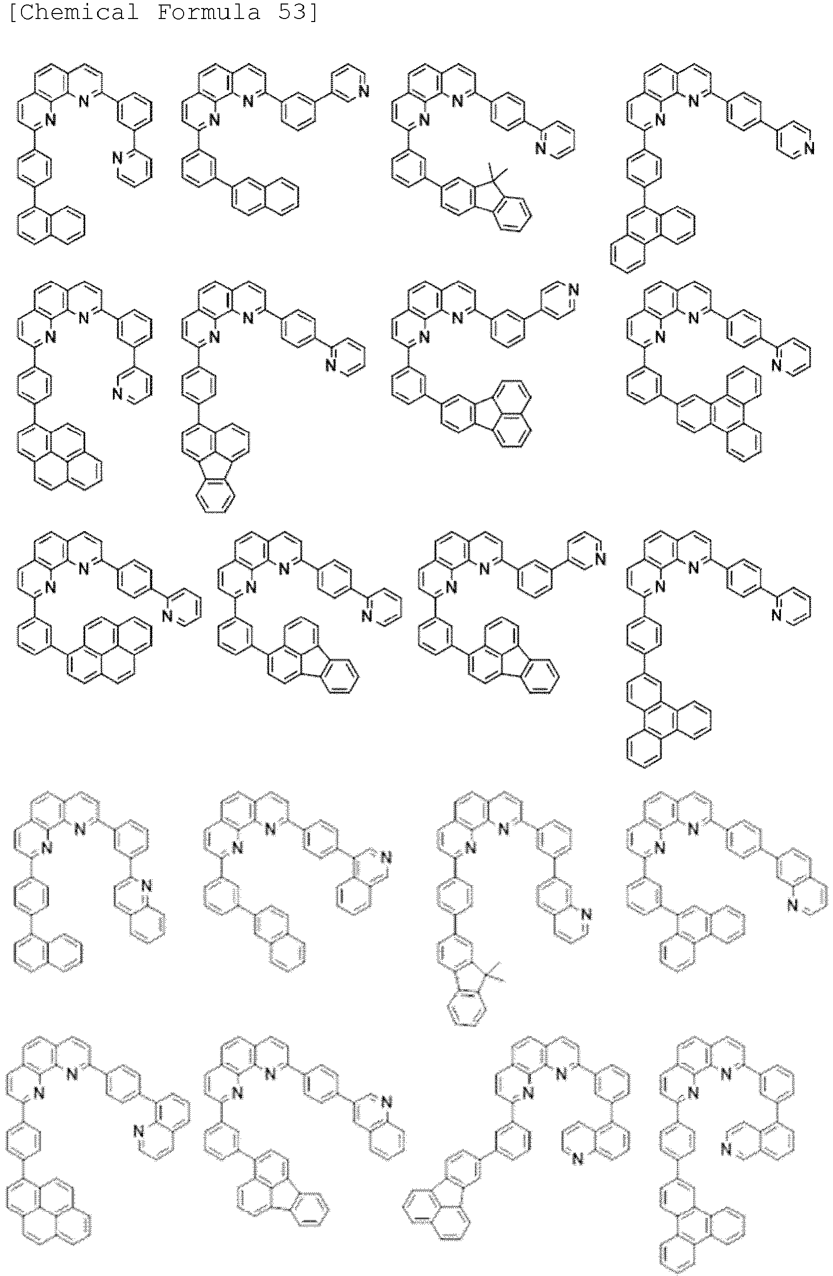

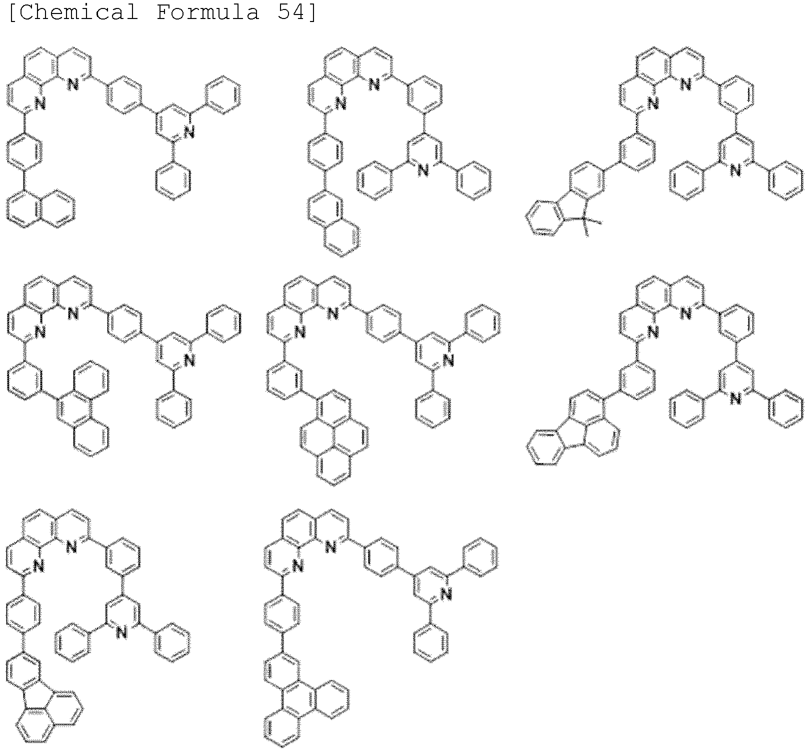

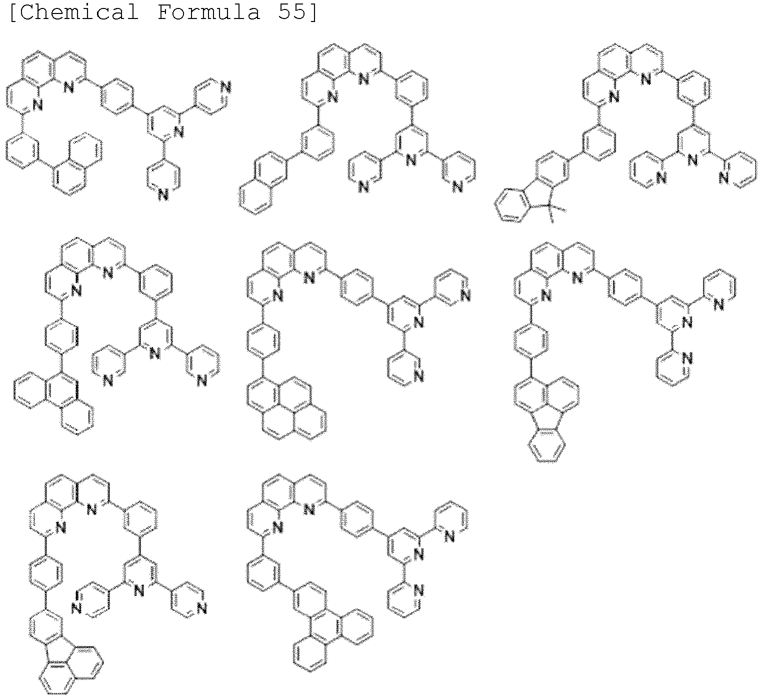

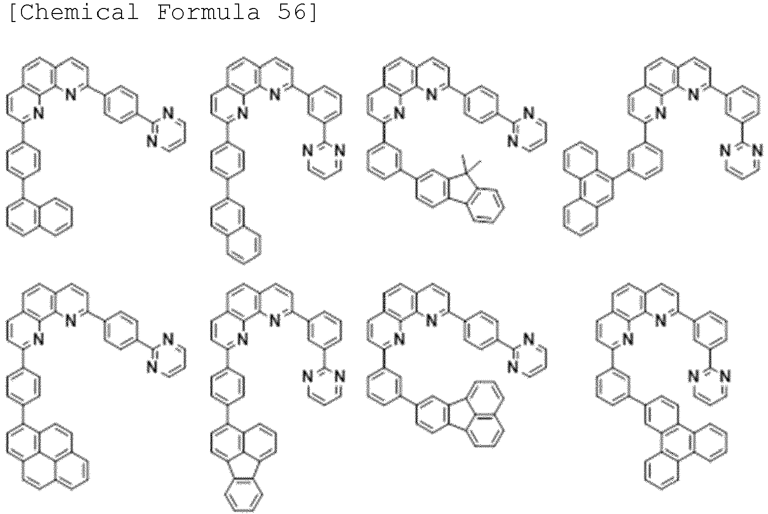

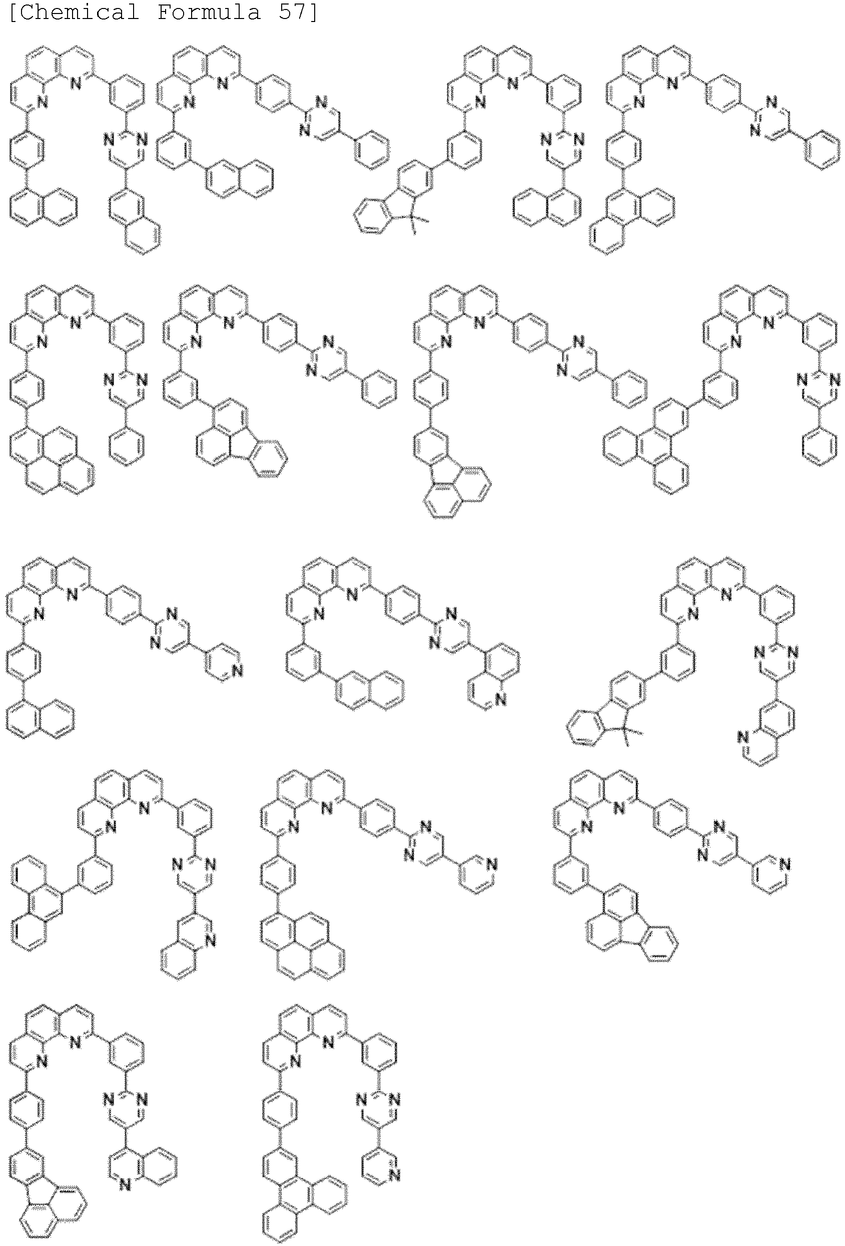

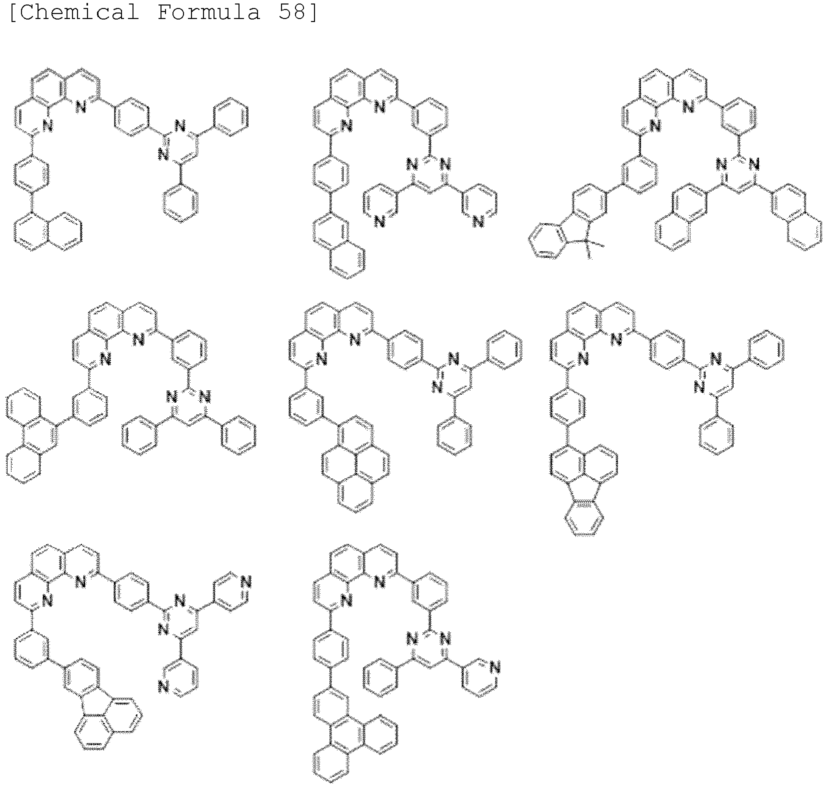

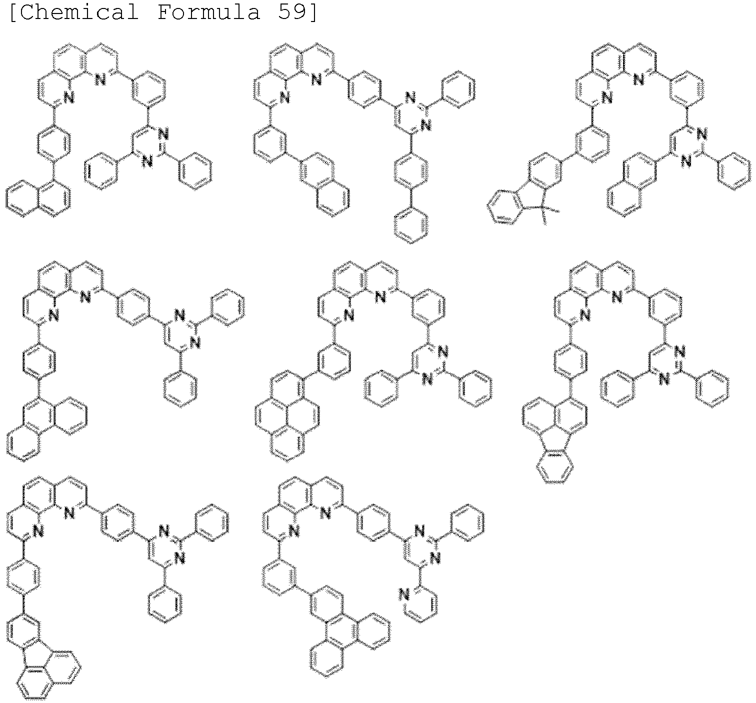

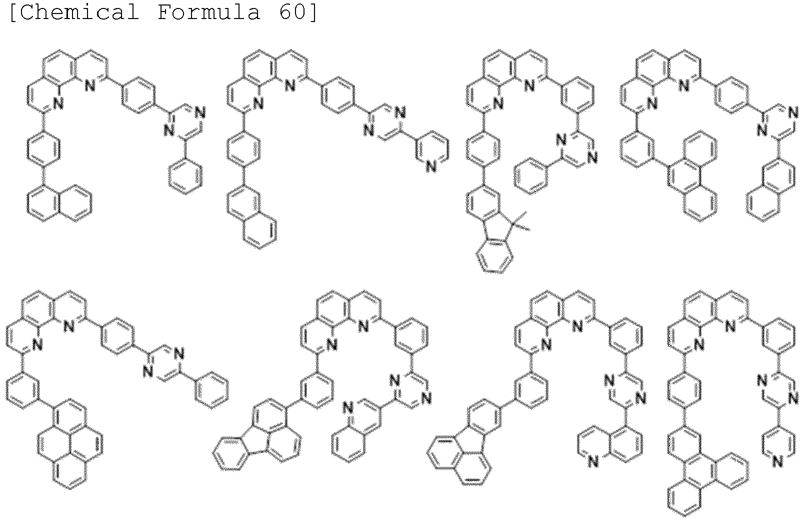

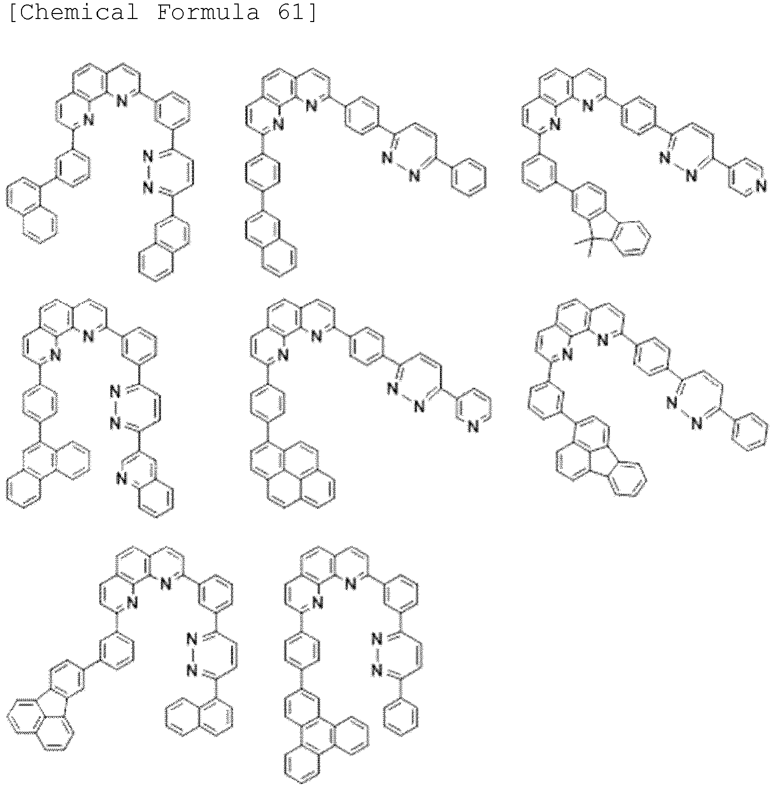

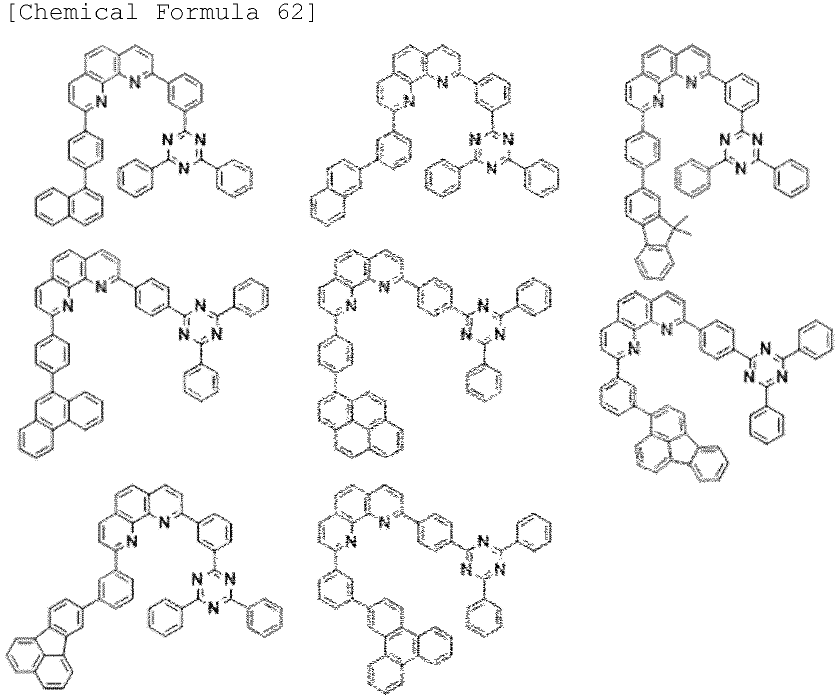

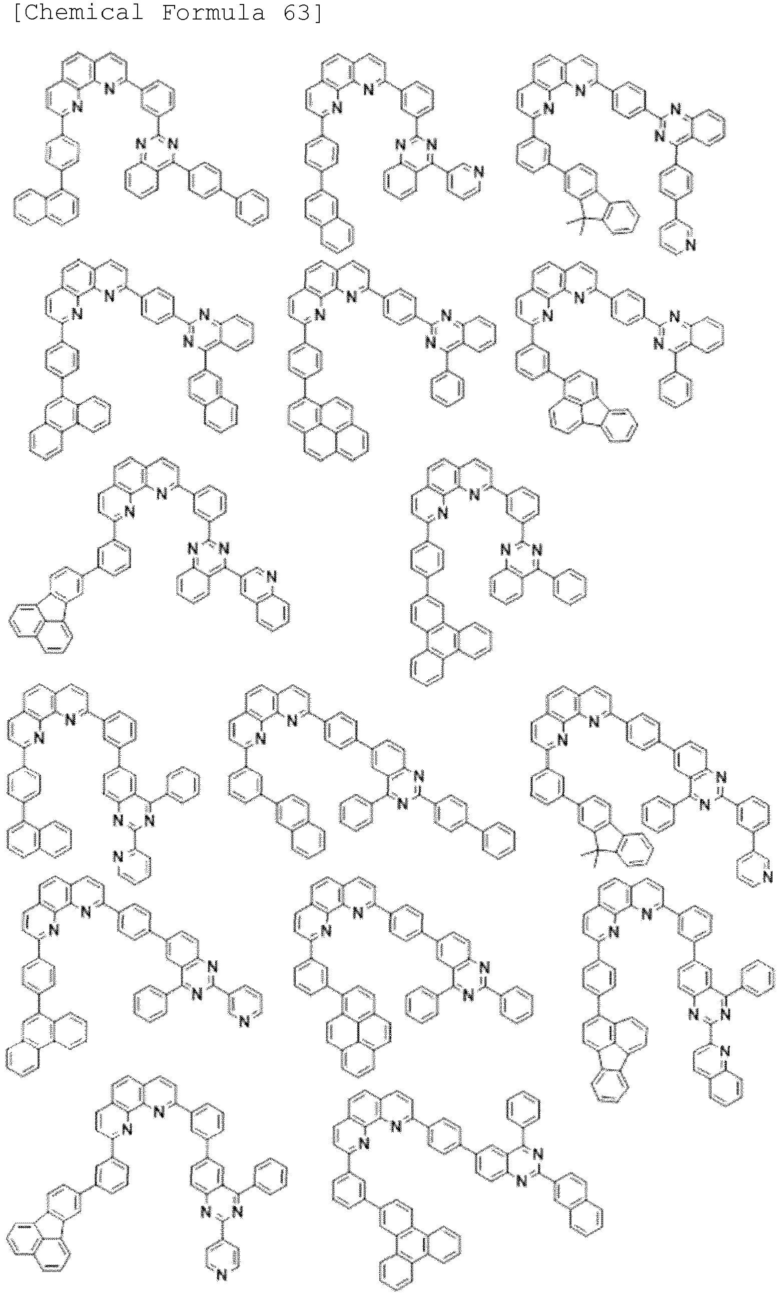

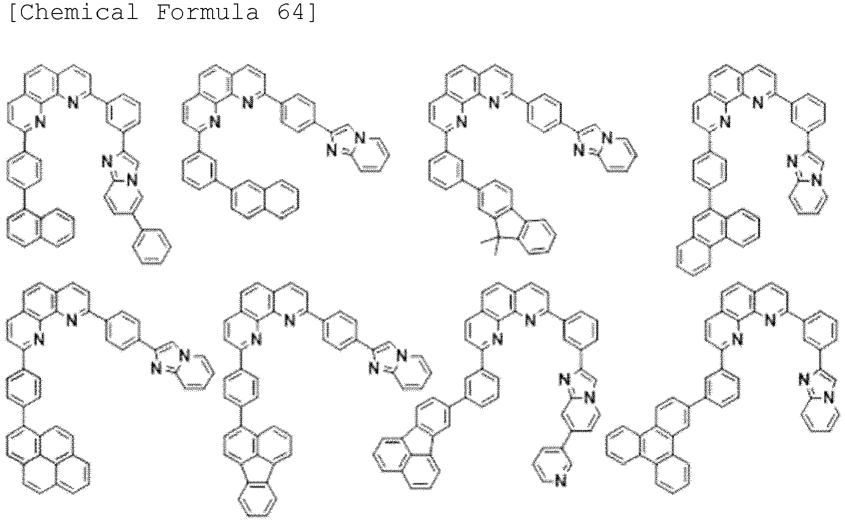

Formulas marked with "☆" do not fall under the scope of the present invention.

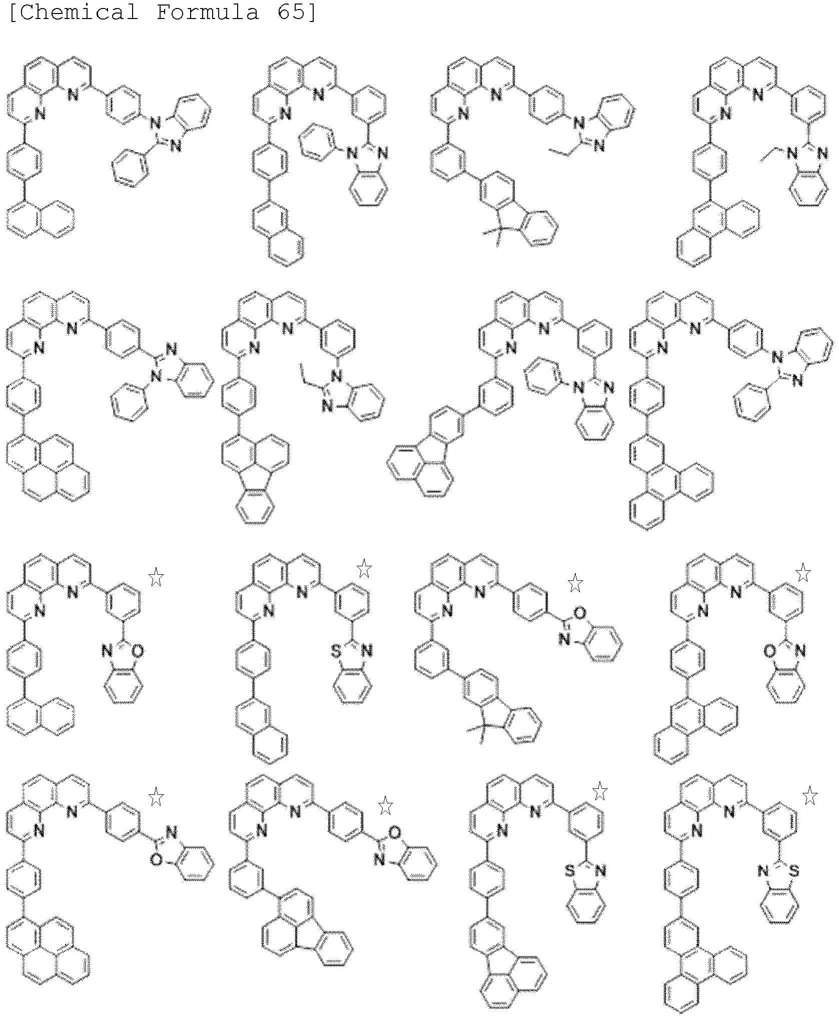

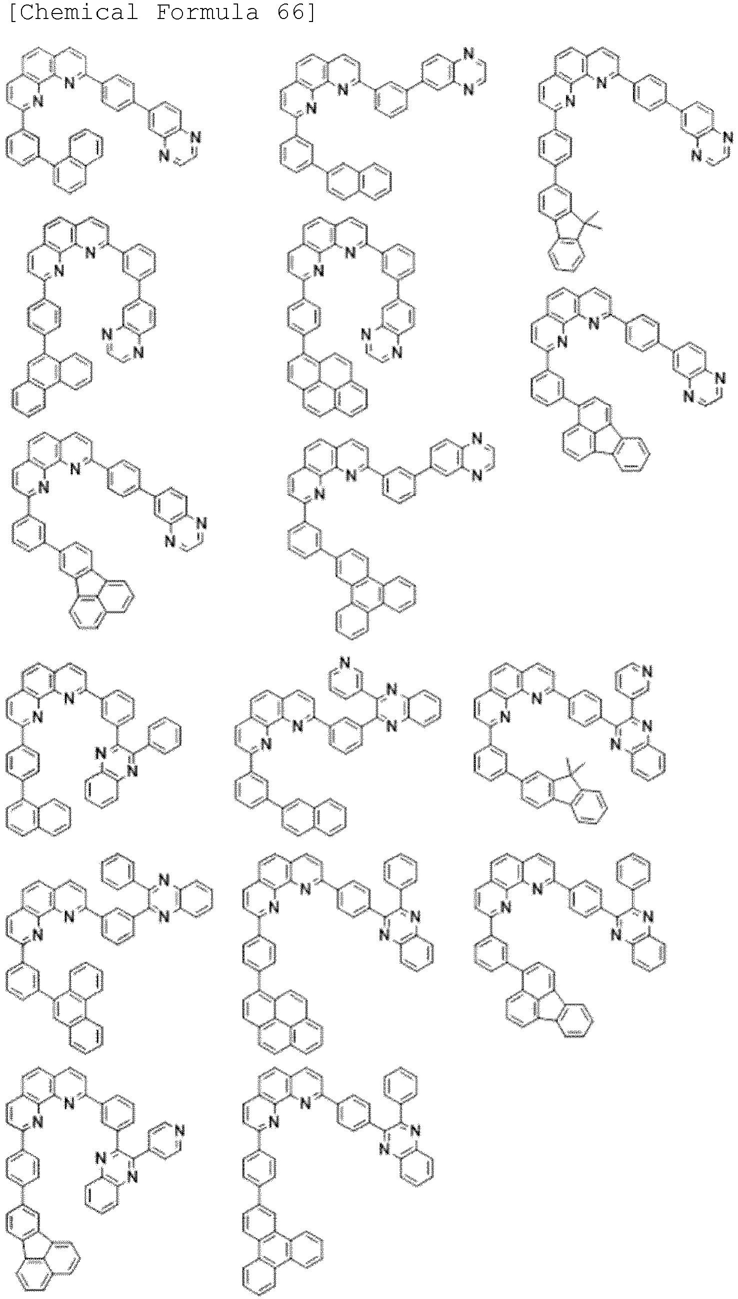

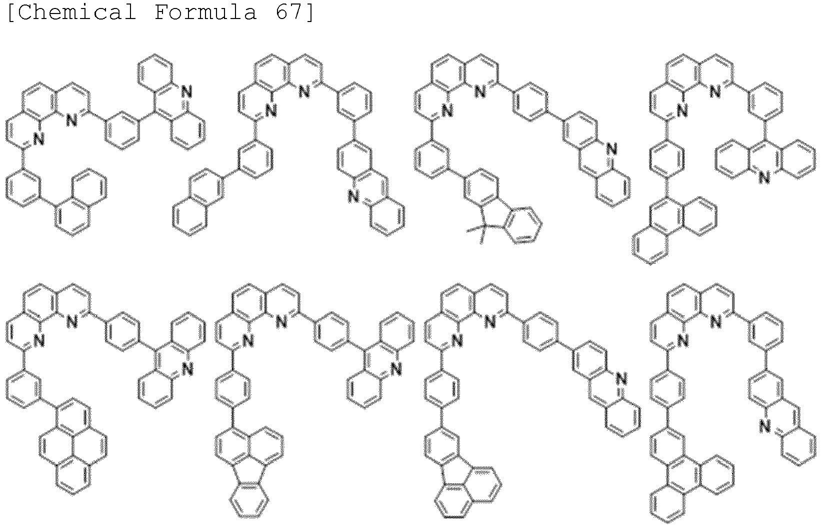

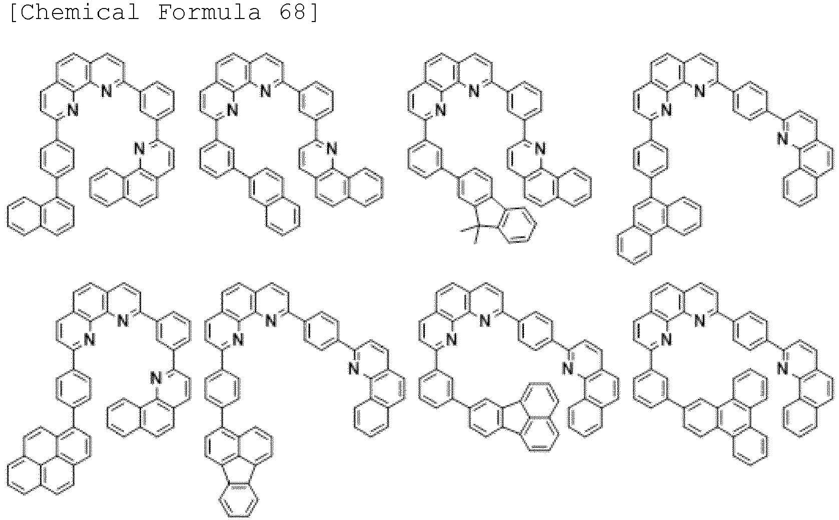

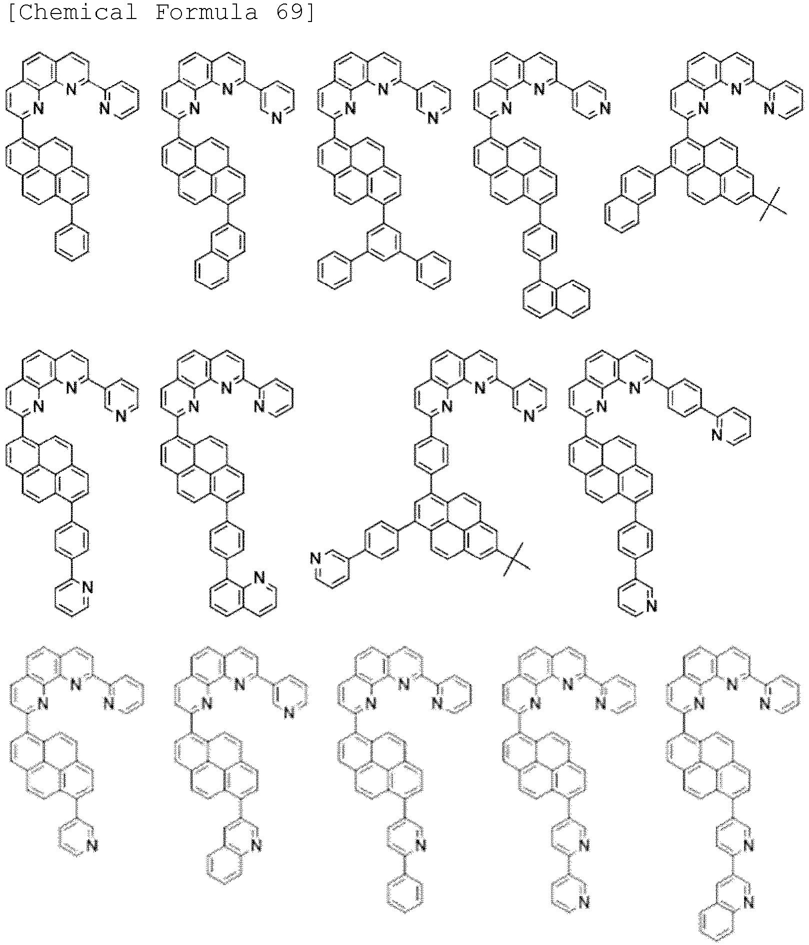

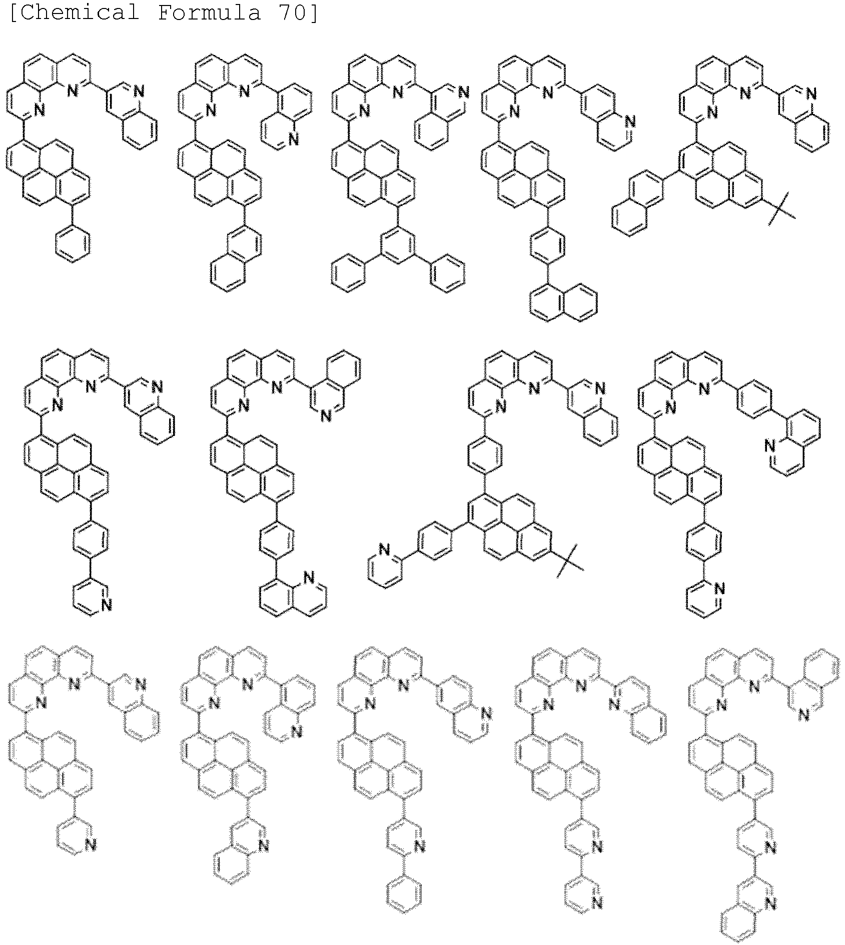

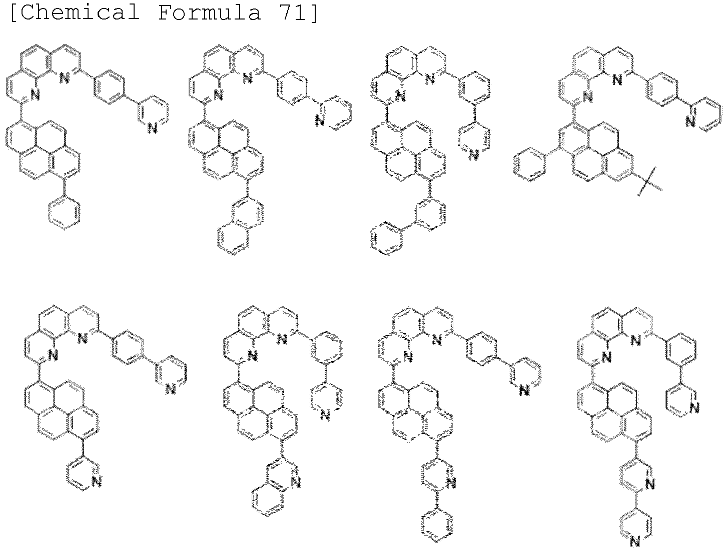

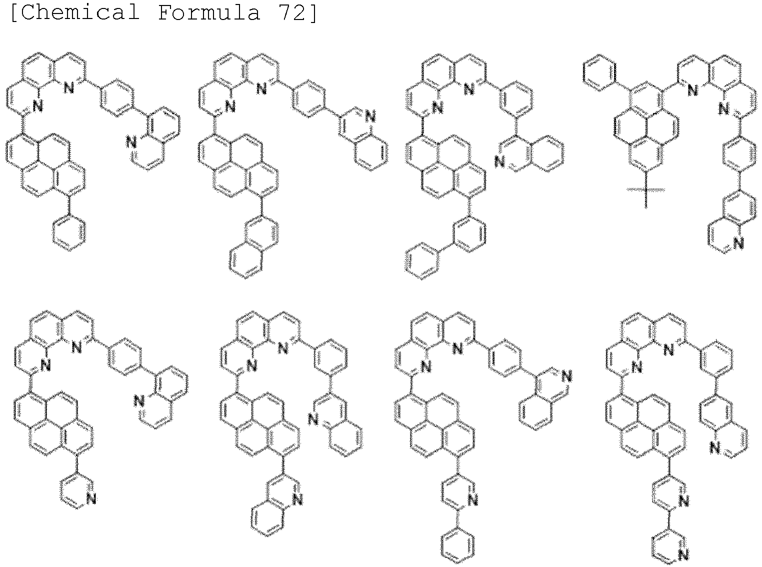

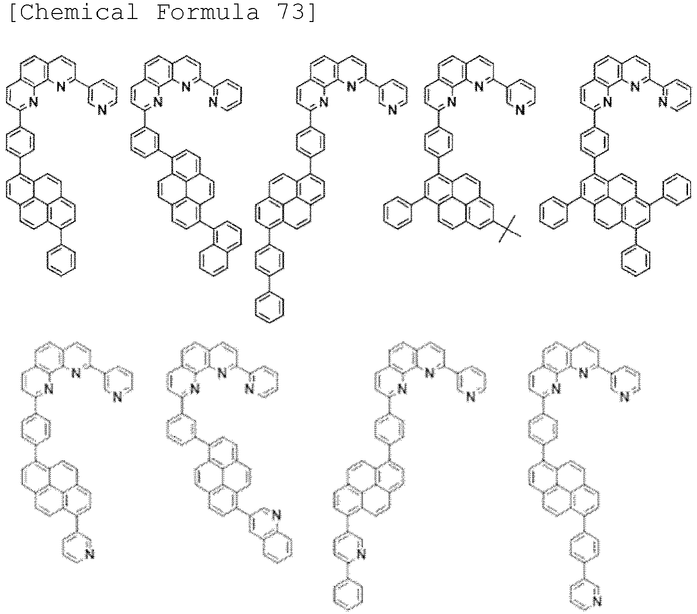

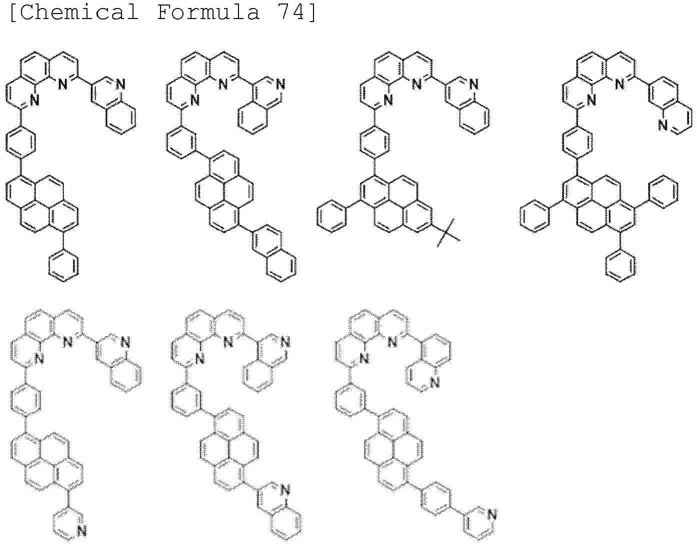

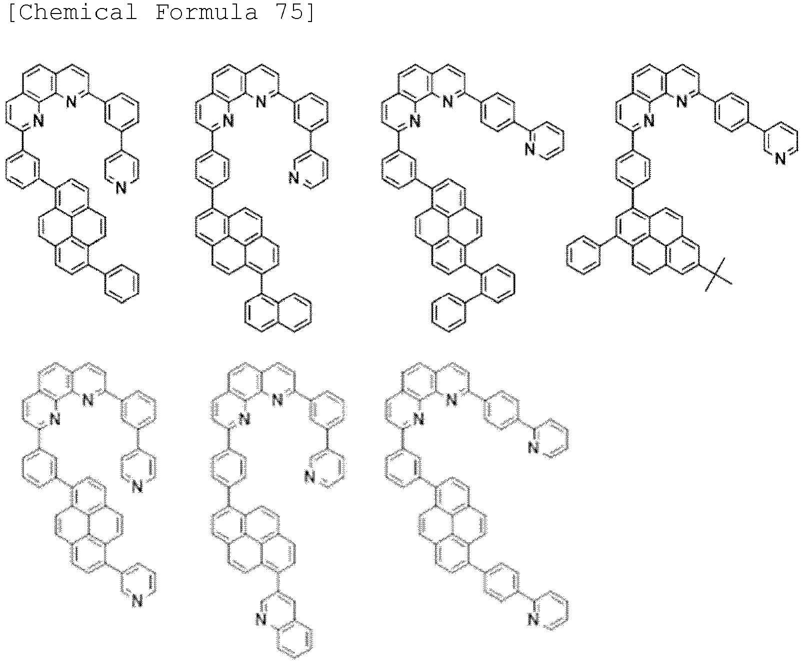

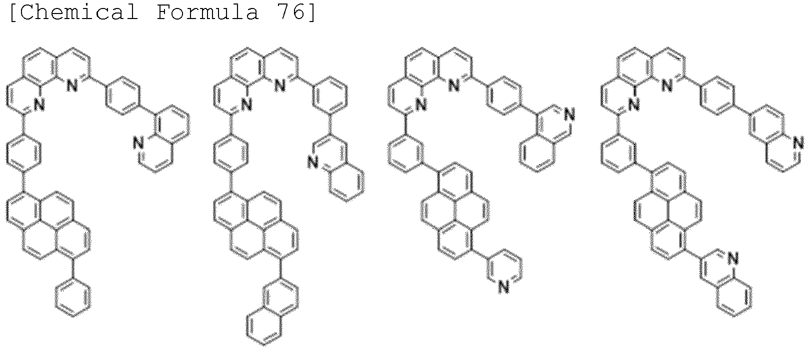

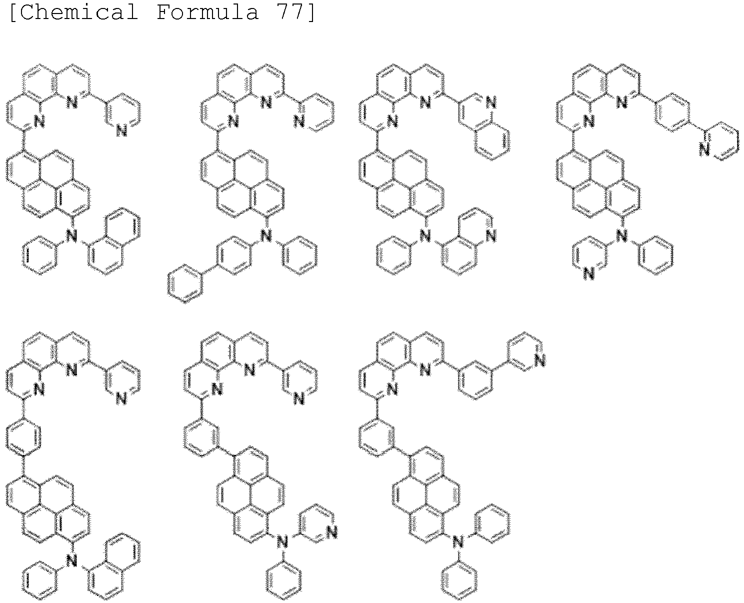

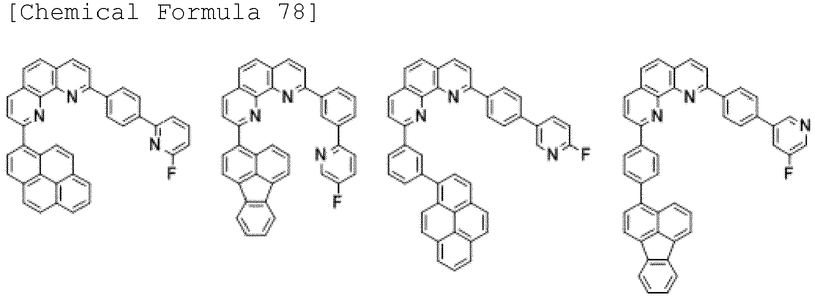

A known method can be used for synthesis of the phenanthroline derivative of the present invention. Examples of the method for introducing an aryl group or a heteroaryl group include, but are not limited to, a method in which a carbon-carbon bond is generated by using a coupling reaction of a halogenated derivative with boronic acid or an esterified derivative of boronic acid. Examples of the method for introducing a substituent into a phenanthroline skeleton include, but are not limited to, a method in which a halogenated derivative is lithiated by halogen-lithium exchange using an organic lithium reagent or the like, and made to nucleophilically act on the phenanthroline skeleton, so that a carbon-carbon bond is generated.

Preferably, the phenanthroline derivative of the present invention is used in electronic devices such as light emitting elements, photoelectric conversion elements, lithium ion batteries, fuel cells and transistors. Preferably, the compound of the present invention is used as an electronic device material in an electronic device, particularly as a light emitting element material or a photoelectric conversion element material in a light emitting element or a photoelectric conversion element.

The light emitting element material denotes a material to be used for any layer of a light emitting element and includes a material to be used for a protective layer (cap layer) of an electrode, in addition to materials to be used for one of a hole transporting layer, an emissive layer and an electron transporting layer as described later. Use of the compound of the present invention in any layer of a light emitting element can afford high luminous efficiency and also can afford a light emitting element having a low driving voltage and high durability.

The photoelectric conversion element material of the present invention denotes a material to be used for any layer of a photoelectric conversion element, and is used for a layer selected from a hole extraction layer, a photoelectric conversion layer and an electron extraction layer. Use of the compound of the present invention in any layer of a photoelectric conversion element can afford high conversion efficiency.

### <Photoelectric conversion element>

The photoelectric conversion element includes an anode, a cathode, and an organic layer interposed between the anode and the cathode, and light energy is converted into an electric signal at the organic layer. Preferably, the organic layer includes at least a photoelectric conversion layer. More preferably, the photoelectric conversion layer includes a p-type material and an n-type material. The p-type material is an electron-donating (donor) material, and has a shallow HOMO energy level, so that holes are easily transported. The n-type material is an electron-attracting (acceptor) material, and has a deep LUMO energy level, so that electrons are easily transported. The p-type material and the n-type material may be laminated or mixed with each other.

Examples of the laminated configuration of the organic layer include, besides a configuration made up of only a photoelectric conversion layer, laminated configurations such as 1) hole extraction layer/photoelectric conversion layer, 2) photoelectric conversion layer/electron extraction layer, and 3) hole extraction layer/photoelectric conversion layer/electron extraction layer. The electron extraction layer is a layer which is provided so that electrons are easily extracted from a photoelectric conversion layer to a cathode. The electron extraction layer is usually provided between the photoelectric conversion layer and the cathode. The hole extraction layer is a layer which is provided so that holes are easily extracted from a photoelectric conversion layer to an anode. The hole extraction layer is usually provided between the anode and the photoelectric conversion layer. Each of the layers may be in the form of a single layer or a plurality of layers.

The phenanthroline derivative of the present invention may be used for any of the layers in the photoelectric conversion element, but is preferably used for the photoelectric conversion layer because it has high electron affinity and thin-film stability, and a strong absorption in a visible light region. Particularly, the phenanthroline derivative has an excellent electron transporting ability, and is therefore preferably used as an n-type material of the photoelectric conversion layer. The compound of the present invention can also be suitably used for the electron extraction layer because it has particularly high electron affinity. Accordingly, electron extraction efficiency from the photoelectric conversion layer to the cathode is improved, and therefore conversion efficiency can be improved.

The photoelectric conversion element can be used for an optical sensor. The photoelectric conversion element in this embodiment can also be used for a solar cell.

### <Light emitting element>

Next, embodiments of the light emitting element of the present invention will be described in detail. The light emitting element of the present invention has an anode and a cathode, and an organic layer interposed between the anode and the cathode, the organic layer emits light by electric energy.

Examples of the laminated configuration of the organic layer include, besides a configuration made up of only an emissive layer, laminated configurations such as 1) hole transporting layer/emissive layer, 2) emissive layer/electron transporting layer, 3) hole transporting layer/emissive layer/electron transporting layer, 4) hole transporting layer/emissive layer/electron transporting layer/electron injection layer, and 5) hole injection layer/hole transporting layer/emissive layer/electron transporting layer/electron injection layer. Each of the layers may be in the form of a single layer or a plurality of layers. The light emitting element may be a laminated-type light emitting element including a plurality of phosphorescence emitting layers and fluorescence emitting layers, or a light emitting element in which a phosphorescence emitting layer and a fluorescence emitting layer are combined with each other. Emissive layers showing mutually different emitted colors can be laminated.

The light emitting element may be a tandem-type light emitting element in which a plurality of element configurations as described above are laminated with intermediate layers interposed therebetween. Among the layers, at least one layer is preferably a phosphorescence emitting layer. Generally, the intermediate layer is also called an intermediate electrode, an intermediate electroconductive layer, a charge generation layer, an electron draw-out layer, a connection layer or an intermediate insulating layer, and for the intermediate layer, a known material configuration can be used. Specific examples of the tandem-type include laminated configurations in which a charge generation layer exists as an intermediate layer between an anode and a cathode, such as 6) hole transporting layer/emissive layer/electrode transporting layer/charge generation layer/hole transporting layer/emissive layer/electron transporting layer, and 7) hole injection layer/hole transporting layer/emissive layer/electron transporting layer/electron injection layer/charge generation layer/hole injection layer/hole transporting layer/emissive layer/electron transporting layer/electron injection layer.

The phenanthroline derivative of the present invention may be used for any layer in the above-mentioned element configuration, but is preferably used for the emissive layer, electron transporting layer or charge generation layer of the light emitting element because it has high electron injection/transporting abilities, a high fluorescence quantum yield and high thin-film stability. Particularly, the phenanthroline derivative has excellent electron injection/transporting abilities, and is therefore more preferably used for the electron transporting layer or charge generation layer. Particularly, the phenanthroline derivative can be suitably used for the electron transporting layer.

### (Anode and Cathode)

In the light emitting element of the present invention, the anode and the cathode have a role for supplying a sufficient current for light emission of the element, and it is preferred that at least one of them is transparent or translucent in order to take out light. Usually, the anode formed on a substrate is made to be a transparent electrode.

While the material to be used for an anode is not particularly limited and may be electroconductive metal oxides, such as tin oxide, indium oxide, indium tin oxide (ITO), and indium zinc oxide (IZO), metals, such as gold, silver, and chromium, inorganic electroconductive substances, such as copper iodide and copper sulfide, or electroconductive polymers, such as polythiophene, polypyrrole, and polyaniline as long as being a material that is capable of injecting holes into an organic layer efficiently and that is transparent or translucent in order to take out light, use of ITO glass or NESA glass is particularly preferable. These electrode materials may be used alone, or a plurality of materials may be used in lamination or in admixture. Since it is favorable that a sufficient current for light emission of the element can be supplied, the resistance of a transparent electrode is not limited, but from the viewpoint of the power consumption of the element, a low resistance is desirable. For example, an ITO substrate having a resistance of 300 Ω/□ or lower functions as an element electrode, but since it is currently possible to supply a substrate having a resistance of about 10 Ω/□, it is particularly preferable to use a substrate having a low resistance of 20 Ω/□ or lower. The thickness of ITO can be arbitrarily selected according to a resistance value, but ITO is usually used at a thickness of between 100 to 300 nm in many cases.

In addition, in order to retain the mechanical strength of the light emitting element, it is preferred to form the light emitting element on a substrate. As the substrate, a glass substrate such as soda glass or alkali-free glass is suitably used. Since it is favorable that the thickness of a glass substrate has a sufficient thickness for retaining the mechanical strength, a thickness of 0.5 mm or more is sufficient. Regarding the material of glass, since it is preferred that the amount of ions eluted from glass is small, alkali-free glass is more preferable. Alternatively, since soda lime glass provided with a barrier coating such as SiO₂ is commercially available, it can also be used. Further, as far as the first electrode stably functions, it is not necessary that the substrate is glass and, for example, the anode may be formed on a plastic substrate. Examples of a method of forming an ITO film include, but are not particularly limited to, an electron beam method, a sputtering method, and a chemical reaction method.

A material to be used in the cathode is not particularly limited, as far as it is a substance which can efficiently inject electrons into the emissive layer. Generally, metals such as platinum, gold, silver, copper, iron, tin, aluminum, and indium, or alloys or multilayer lamination of these metals with metals having a low work function such as lithium, sodium, potassium, calcium and magnesium are preferred. Among them, as a main component, aluminum, silver, and magnesium are preferred from the viewpoints of electric resistance value, easiness of making a film, stability of a film, and luminous efficiency. In particular, it is preferred that the material is constituted by magnesium and silver because electron injection into the electron transporting layer and the electron injection layer in the present invention becomes easy, and low voltage driving becomes possible.

Further, preferable examples include lamination of metals such as platinum, gold, silver, copper, iron, tin, aluminum, and indium, or alloys using these metals, inorganic substances such as silica, titania, and silicon nitride, and organic polymer compounds such as polyvinyl alcohol, polyvinyl chloride, and a hydrocarbon-based polymer compound as a protective film layer on the cathode for protecting the cathode . The phenanthroline derivative of the present invention can also be used as the protective film layer (cap layer) . However, in the case of an element structure for taking out light from the cathode side (top emission structure), the protective film layer is selected from materials having light permeability in a visible light region. Examples of a method for preparation of these electrodes include, but are not particularly limited to, resistance heating, electron beam, sputtering, ion plating and coating.

### (Hole Transporting Layer)

The hole transporting layer is formed by a method in which one or more hole transporting materials are laminated or mixed, or a method using a mixture of a hole transporting material and a polymer binder. The hole transporting material is required to efficiently transport holes from a positive electrode between electrodes given an electric field, and preferably has high hole injection efficiency and efficiently transports injected holes. For this purpose, the hole transporting material is required to be a substance having an appropriate ionization potential and, moreover, great hole mobility and, further, excellent stability, and generating impurities that become a trap with difficulty at the time of production and at the time of use.

The substance satisfying the above-mentioned requirements is not particularly limited, and examples thereof include benzidine derivatives such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB) and bis(N,N'-diphenyl-4-aminophenyl)-N,N-diphenyl-4,4'-diamino-1,1'-biphenyl (TPD232); materials called starburst arylamines, such as 4,4',4"-tris(3-methylphenyl(phenyl)amino)triphenylamine (m-MTDATA) and 4,4',4"-tris(1-naphthyl(phenyl)amino)triphenylamine (1-TNATA); and materials having a carbazole skeleton.

Particularly, carbazole oligomer, specifically derivatives of a carbazole dimer such as bis(N-arylcarbazole) or bis (N-alkylcarbazole), derivatives of a carbazole trimer and derivatives of a carbazole tetramer are preferable, and derivatives of a carbazole dimer and derivatives of a carbazole trimer are more preferable. Asymmetric bis(N-arylcarbazole) derivatives are especially preferable. Materials having one carbazole skeleton and one triarylamine skeleton are also preferable. Materials having an arylene group as a coupling group between the nitrogen atom of amine and a carbazole skeleton are more preferable, and materials having skeletons represented by the following general formulae (3) and (4) are especially preferable.

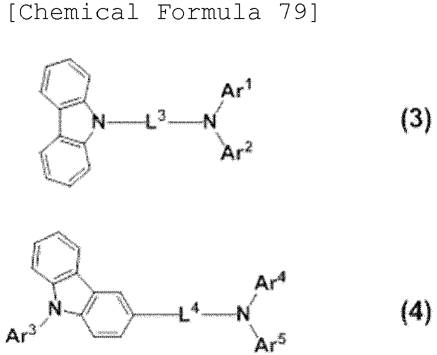

L³ and L⁴ each represent an arylene group, and Ar¹ to Ar⁵ each represent an aryl group.

Besides the above-mentioned compounds, heterocyclic compounds such as triphenylene compounds, pyrazoline derivatives, stilbene-based compounds, hydrazone-based compounds, benzofuran derivatives and thiophene derivatives, oxadiazole derivatives, phthalocyanine derivatives and porphyrin derivatives; fullerene derivatives; and polymer-based compounds such as polycarbonate, styrene derivatives, polythiophene, polyaniline, polyfluorene, polyvinyl carbazole and polysilane each having the above-mentioned monomer on the side chain can be preferably used as hole transporting materials. Further, inorganic compounds such as p-type Si and p-type SiC can also be used. The compound of the present invention can also be used as a hole transporting material because it has excellent electrochemical stability.

In some light emitting elements, some of electrons injected into an emissive layer may reach a hole transporting layer without being recombined, resulting in deterioration of durability of the light emitting element. Therefore, it is preferred that a compound excellent in electron blocking property is used for the hole transporting layer. Particularly, a compound containing a carbazole skeleton is preferred because it is excellent in electron blocking property, and can contribute to an increase in efficiency of the light emitting element. Further, the compound containing a carbazole skeleton is preferably a material having a carbazole oligomer, or skeletons represented by the general formulae (3) and (4) . The compound having a multi-meric carbazole skeleton is preferably a derivative of a carbazole dimer, a derivative of a carbazole trimer, or a derivative of a carbazole tetramer. The compound having a multi-meric carbazole skeleton is more preferably a derivative of a carbazole dimer, or a derivative of a carbazole trimer, especially preferably an asymmetric bis(N-arylcarbazole) derivative. This is because they have both a proper electron blocking property and proper hole injection/transporting properties. Further, when the compound containing a carbazole skeleton is used for the hole transporting layer, it is more preferable that an emissive layer to be combined contains the later-described phosphorescence emitting material. This is because the compound having a carbazole skeleton has a high triplet exciton blocking function, so that luminous efficiency can be increased when the compound is combined with a phosphorescence emitting material.

Use of a triphenylene skeleton-containing compound, excellent in that it has high hole mobility, for the hole transporting layer is preferred because a carrier balance is improved, so that the effects of improving luminous efficiency and improving durable life can be obtained. It is further preferable that the compound containing a triphenylene skeleton has two or more diarylamino groups.

The compound containing a carbazole skeleton and the compound containing a triphenylene skeleton may be each used alone as a hole transporting layer, or may be mixed and used. Other materials may be mixed as long as the effects of the present invention are not impaired. When the hole transporting layer includes a plurality of layers, it is preferred that any one layer contains the compound containing a carbazole skeleton or the compound containing a triphenylene skeleton.

### (Hole Injection Layer)

A hole injection layer may be provided between an anode and a hole transporting layer. When a hole injection layer is provided, the light emitting element has a reduced driving voltage, and durable life is improved.

A material having an ionization potential smaller than that of a material which is usually used for the hole transporting layer is preferably used for the hole injection layer. Specific examples include benzidine derivatives such as TPD232, and starburst arylamine materials, and besides, phthalocyanine derivatives can also be used.

It is preferred that the hole injection layer is formed of an acceptor compound alone, or used with another hole transporting material doped with an acceptor compound. Examples of the acceptor compound include metal chlorides such as iron(III) chloride, aluminum chloride, gallium chloride, indium chloride, and antimony chloride, metal oxides such as molybdenum oxide, vanadium oxide, tungsten oxide, and ruthenium oxide, and charge transfer complexes such as tris(4-bromophenyl)aminium hexachloroantimonate (TBPAH). Moreover, organic compounds having a nitro group, a cyano group, halogen, or a trifluoromethyl group in the molecule, quinone-based compounds, acid anhydride-based compounds, and fullerene can also be used suitably.

Specific examples of such compounds include hexacyanobutadiene, hexacyanobenzene, tetracyanoethylene, tetracyanoquinodimethane (TCNQ), tetrafluorotetracyanoquinodimethane (F₄-TCNQ), 2,3,6,7,10,11-hexacyano-1,4,5,8,9,12-hexaazatriphenylene (HAT-CN₆), p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C₆₀, and C₇₀.

Of these, metal oxides and cyano group-containing compounds are preferred because they can be easily handled and deposited, and therefore the above-described effects can be obtained easily. Examples of the preferred metal oxide include molybdenum oxide, vanadium oxide and ruthenium oxide. Among cyano group-containing compounds, (a) a compound having in the molecule at least one electron-accepting nitrogen atom in addition to the nitrogen atom of the cyano group, (b) a compound having both halogen and a cyano group in the molecule, (c) a compound having both a carbonyl group and a cyano group in the molecule, or (d) a compound having in the molecule both halogen and a cyano group and further, at least one electron-accepting nitrogen atom in addition to the nitrogen atom of the cyano group is more preferable because it serves as a strong electron acceptor. Specific examples of the above-mentioned compound include the following compounds.

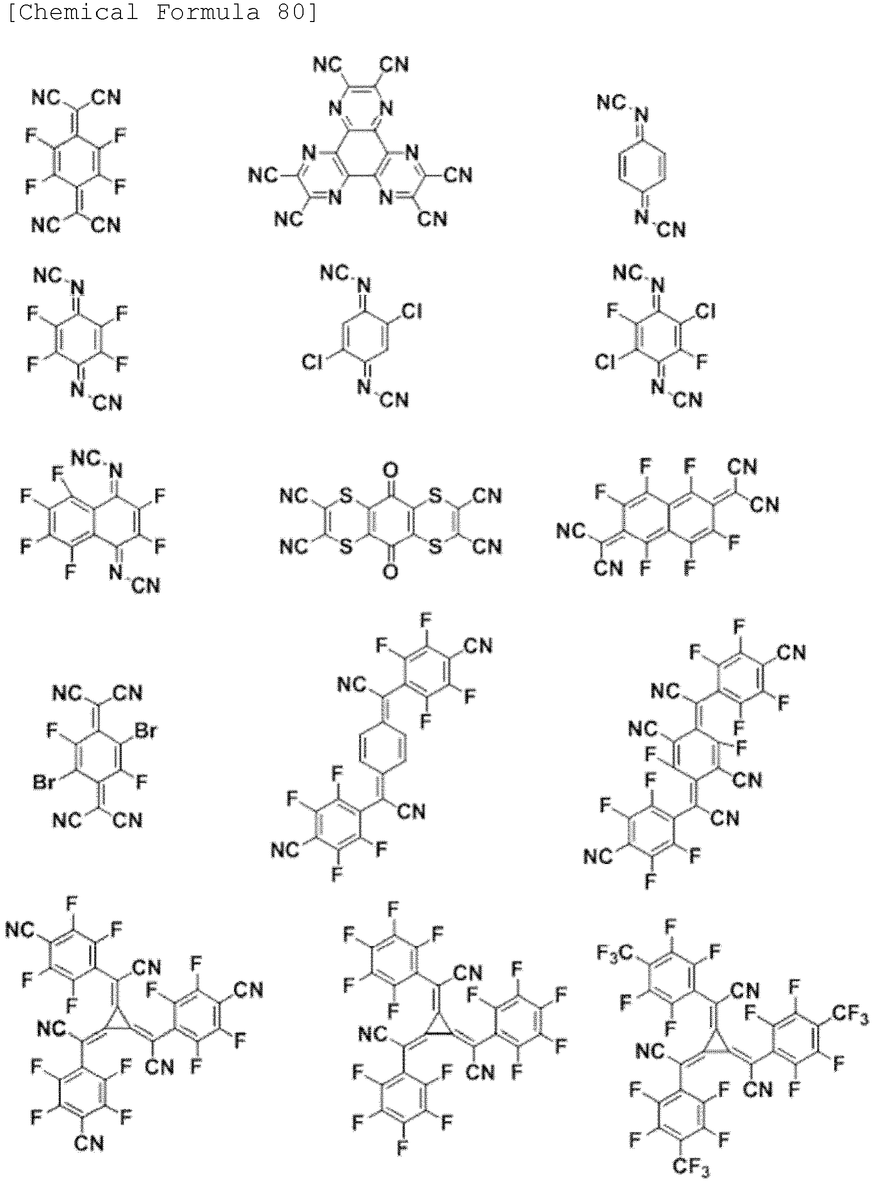

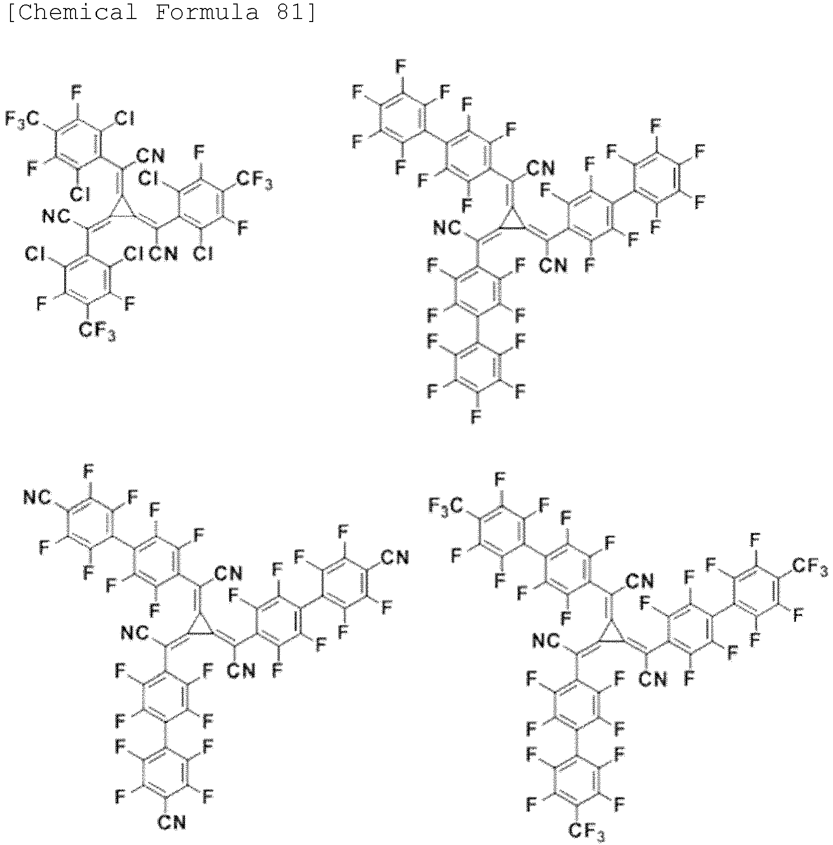

In either of the case where a hole injection layer is formed of an acceptor compound alone or the case where a hole injection layer is doped with an acceptor compound, the hole injection layer may be a single layer or may be a laminate of a plurality of layers. The hole injection material to be used in combination when the hole injection layer is doped with an acceptor compound is preferably the same compound as the compound to be used for the hole transporting layer because a barrier to injection of holes into the hole transporting layer can be mitigated.

### (Emissive Layer)

The emissive layers may be in the form of a single layer or a plurality of layers, each of which is formed of an emissive material (host material, dopant material), and this may be a mixture of the host material and the dopant material, or the host material alone. That is, in the light emitting element of the present invention, only the host material or the dopant material may emit light, or both of the host material and the dopant material may emit light, in each emissive layer. From the viewpoints that electric energy is efficiently utilized and light emission at high color purity is obtained, it is preferred that the emissive layer includes a mixture of the host material and the dopant material.

In addition, the host material and the dopant material may be one kind or a combination of a plurality of kinds, respectively. The dopant material may be contained in a whole host material, or may be partially contained therein. The dopant material may be laminated, or may be dispersed.

The dopant material can control an emitted color. When the amount of the dopant material is too large, concentration quenching occurs, and therefore the dopant material is preferably used in an amount of 20% by weight or less, further preferably 10% by weight or less based on the host material. As a doping method, the dopant material can be co-deposited with the host material, or the dopant material may be mixed with the host material in advance to be deposited simultaneously.

Specific examples of the emissive material that can be used include, but are not particularly limited to, fused ring derivatives such as anthracene and pyrene, metal chelated oxinoid compounds including tris(8-quinolinolato)aluminum, bisstyryl derivatives such as bisstyrylanthracene derivatives and distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives, and indolocarbazole derivatives and, as a polymer series, polyphenylenevinylene derivatives, polyparaphenylene derivatives, and polythiophene derivatives, which have hitherto been known as a light emitting body.

The host material contained in the emissive material is not particularly limited, and examples of the host material which can be used include, but are not particularly limited to, compounds having a fused aryl ring such as naphthalene, anthracene, phenanthrene, pyrene, chrysene, naphthacene, triphenylene, perylene, fluoranthene, fluorene and indene, and derivatives thereof, aromatic amine derivatives such as N,N'-dinaphthyl-N,N'-diphenyl-4,4'-diphenyl-1,1'-diamine, metal chelated oxinoid compounds including tris(8-quinolinato)aluminum (III), bisstyryl derivatives such as distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives and triazine derivatives and, as a polymer series, polyphenylenevinylene derivatives, polyparaphenylene derivatives, polyfluorene derivatives, polyvinylcarbazole derivatives, and polythiophene derivatives.

The dopant material is not particularly limited, and examples of the dopant material that can be used include compounds having a fused aryl ring such as naphthalene, anthracene, phenanthrene, pyrene, chrysene, triphenylene, perylene, fluoranthene, fluorene and indene, and derivatives thereof (e.g., 2-(benzothiazol-2-yl)-9,10-diphenylanthracene and 5,6,11,12-tetraphenylnaphthacene); compounds having a heteroaryl ring such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyridine, pyrazine, naphthyridine, quinoxaline, pyrrolopyridine and thioxanthene, and derivatives thereof; borane derivatives; distyrylbenzene derivatives; aminostyryl derivatives such as 4,4'-bis(2-(4-diphenylaminophenyl)ethenyl)biphenyl and 4,4'-bis(N-(stilben-4-yl)-N-phenylamino)stilbene; aromatic acetylene derivatives; tetraphenylbutadiene derivatives; stilbene derivatives; aldazine derivatives; pyrromethene derivatives; diketopyrrolo[3,4-c] pyrrole derivatives; coumarin derivatives such as 2,3,5,6-1H,4H-tetrahydro-9-(2'-benzothiazolyl)quinolizino[9 ,9a,1-gh] coumarin; azole derivatives such as imidazole, thiazole, thiadiazole, carbazole, oxazole, oxadiazole and triazole, and metal complexes thereof; and aromatic amine derivatives typified by N,N'-diphenyl-N,N'-di(3-methylphenyl)-4,4'-diphenyl-1,1'-di amine.

The emissive layer may contain a phosphorescence emitting material. The phosphorescence emitting material is a material that emits phosphorescence at room temperature. When a phosphorescence emitting material is used as a dopant, basically it is required to obtain phosphorescence emission at room temperature, and the phosphorescence emitting material is not particularly limited, and is preferably an organic metal complex compound containing at least one metal selected from the group consisting of iridium (Ir), ruthenium (Ru), rhodium (Rh), palladium (Pd), platinum (Pt), osmium (Os), and rhenium (Re). Among them, an organic metal complex having iridium or platinum is more preferred because it has a high phosphorescence emission yield at room temperature.

As the host to be used in combination with a phosphorescence emitting dopant, aromatic hydrocarbon compound derivatives such as indole derivatives, carbazole derivatives, indolocarbazole derivatives, nitrogen-containing aromatic compound derivatives having a pyridine, pyrimidine or triazine skeleton, polyarylbenzene derivatives, spirofluorene derivatives, truxene derivatives and triphenylene derivatives; compounds containing a chalcogen element, such as dibenzofuran derivatives and dibenzothiophene derivatives; organic metal complexes such as beryllium quinolinol complexes; and the like are suitably used, but the host is not limited thereto as long as basically it has higher triplet energy than a dopant used, and electrons and holes are smoothly injected and transported from the respective transporting layers. Two or more triplet emissive dopants may be contained, and two or more host materials may be contained. Further, one or more triplet emissive dopants and one or more fluorescence emitting dopants may be contained.

The preferable phosphorescence emitting host or dopant is not particularly limited, and specific examples thereof include the following.

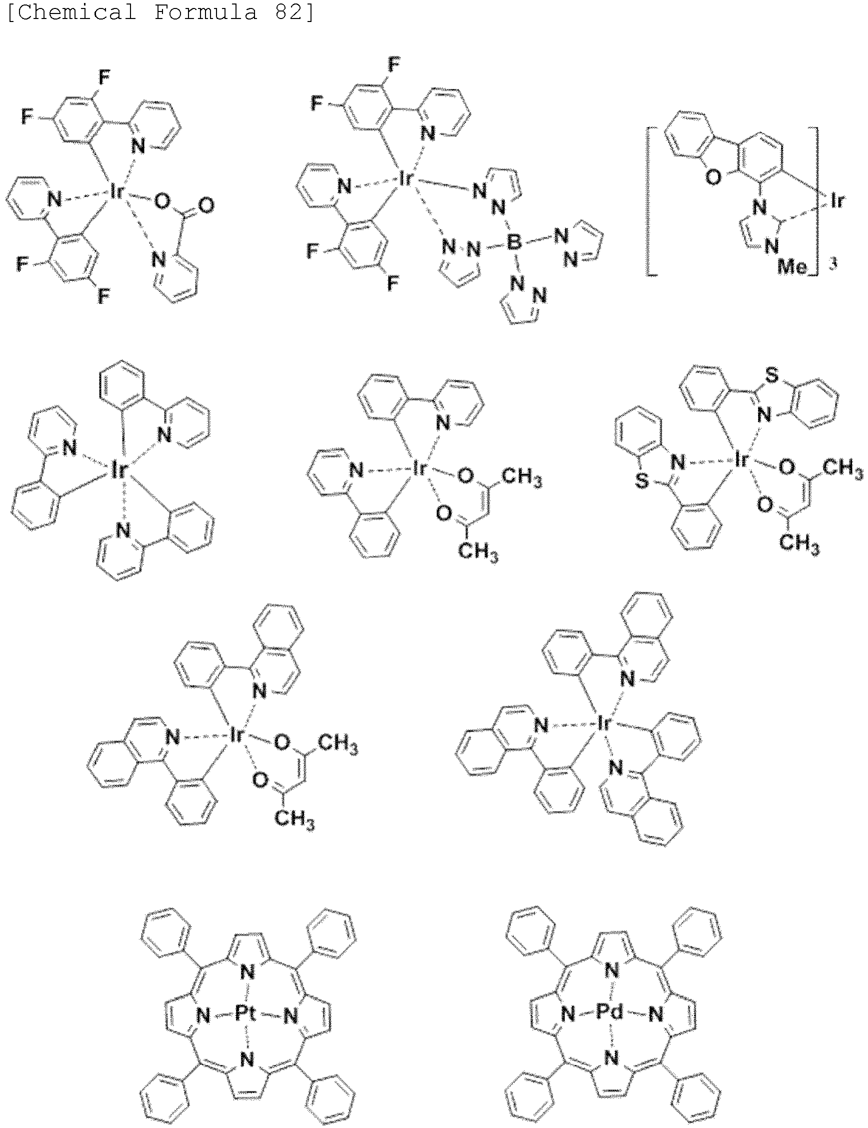

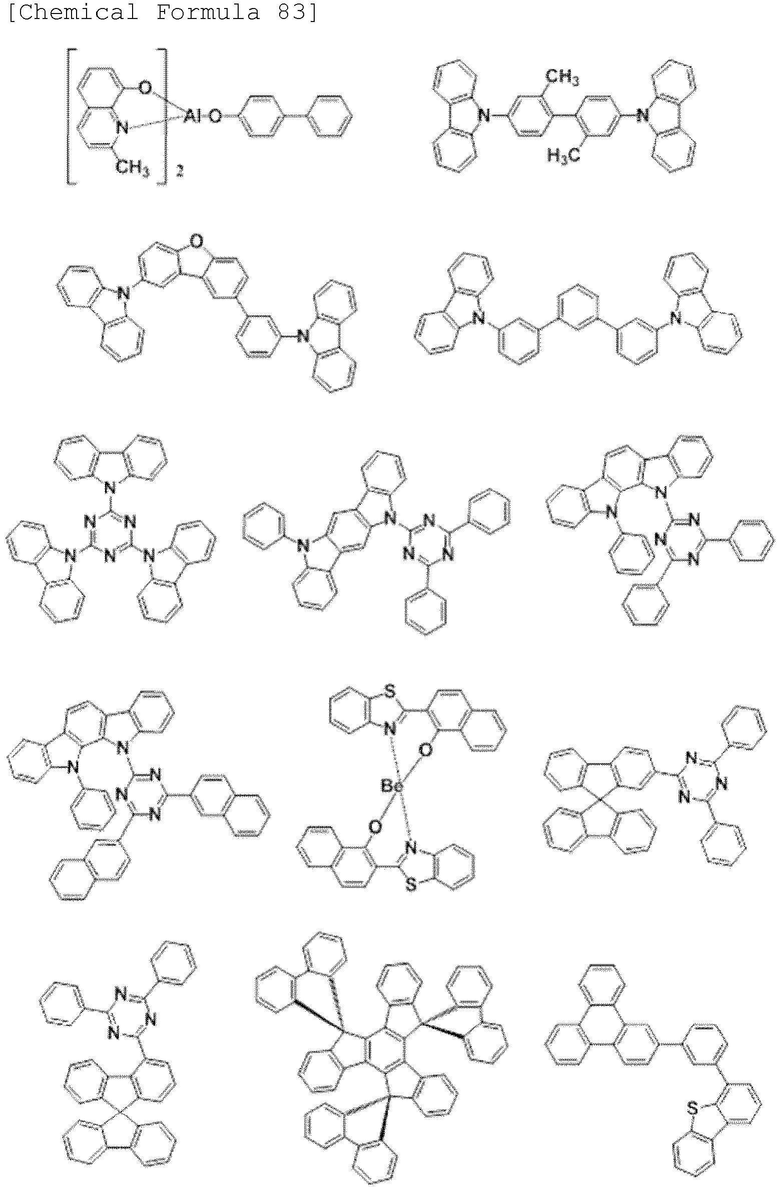

The emissive layer may contain a thermally activated delayed fluorescence material. The thermally activated delayed fluorescence material, which is also called a TADF material in general, is a material in which an energy gap between an energy level in a singlet excited state and an energy level in a triplet excited state is reduced to promote inverse intersystem crossing from the triplet excited state to the singlet excited state, so that the singlet exciton generation probability is increased. The thermally activated delayed fluorescence material may be a material that shows a thermally activated delayed fluorescence with a single material, or a material that shows a thermally activated delayed fluorescence with a plurality of materials. The thermally activated delayed fluorescence material to be used may include a single material or a plurality of materials, and for the thermally activated delayed fluorescence, a known material can be used. Specific examples of thereof include benzonitrile derivatives, triazine derivatives, disulfoxide derivatives, carbazole derivatives, indolocarbazole derivatives, dihydrophenazine derivatives, thiazole derivatives, and oxadiazole derivatives.

The phenanthroline derivative of the present invention can also be used as an emissive material, and is suitably used particularly as a phosphorescence host material.

### (Electron Transporting Layer)

In the present invention, the electron transporting layer is a layer existing between a cathode and an emissive layer. The electron transporting layer may include a single layer, or a plurality of layers, and may or may not be in contact with a cathode or an emissive layer.

The electron transporting layer is desired to have high electron injection efficiency from a cathode, efficiently transport injected electrons, and have high electron injection efficiency to an emissive layer. For this reason, it is preferred that the electron transporting layer is formed of a substance having great electron affinity and, moreover, great electron mobility and, further, excellent stability, and generating impurities that become a trap with difficulty at the time of production and at the time of use. However, when transportation balance between holes and electrons is considered, if the electron transporting layer mainly plays a role of being able to efficiently inhibiting holes from the anode from flowing to the cathode side without recombination, even when the layer is constituted by a material having not so high electron transporting ability, the effect of improving luminous efficiency becomes equivalent to that when the layer is constituted by a material having a high electron transporting ability. Therefore, the electron transporting layer in the present invention also includes a hole inhibition layer which can efficiently inhibit the transfer of holes as the same meaning.

Examples of the electron transporting material to be used for the electron transporting layer include fused polycyclic aromatic derivatives, such as naphthalene and anthracene, styryl-based aromatic ring derivatives typified by 4,4'-bis(diphenylethenyl)biphenyl, quinone derivatives, such as anthraquinone and diphenoquinone, phosphorus oxide derivatives, and various types of metal complexes, such as quinolinol complexes, e.g., tris(8-quinolinolato)aluminum(III), benzoquinolinol complexes, hydroxyazole complexes, azomethine complexes, tropolone metal complexes, and flavonol metal complexes. It is preferred to use a compound that includes an element selected from carbon, hydrogen, nitrogen, oxygen, silicon and phosphorus, and has an aromatic heterocyclic structure containing electron-accepting nitrogen because it can reduce a driving voltage and a highly efficient light emission can be obtained.

Examples of the compound having an aromatic heterocyclic structure including electron-accepting nitrogen include benzimidazole derivatives, benzoxazole derivatives, benzothiazole derivatives, oxadiazole derivatives, thiadiazole derivatives, triazole derivatives, pyrazine derivatives, phenanthroline derivatives, quinoline derivatives, benzoquinoline derivatives, oligopyridine derivatives such as bipyridine and terpyridine, quinoxaline derivatives and naphthyridine derivatives. Among them, imidazole derivatives such as
tris(N-phenylbenzimidazol-2-yl)benzene; oxadiazole derivatives such as
1,3-bis[(4-tert-butylphenyl)1,3,4-oxadiazolyl]phenylene; triazole derivatives such as
N-naphthyl-2,5-diphenyl-1,3,4-triazole; phenanthroline derivatives such as bathocuproine and
1,3-bis(1,10-phenanthrolin-9-yl)benzene; benzoquinoline derivatives such as
2,2'-bis(benzo[h]quinolin-2-yl)-9,9'-spirobifluorene; bipyridine derivatives such as
2,5-bis(6'-(2',2"-bipyridyl))-1,1-dimethyl-3,4-diphenylsilo le; terpyridine derivatives such as
1,3-bis(4'-(2,2':6'2"-terpyridinyl))benzene; and naphthyridine derivatives such as
bis(1-naphthyl)-4-(1,8-naphthyridin-2-yl)phenylphosphine oxide are suitably used in view of an electron transporting ability. It is more preferable that such a derivative has a fused polycyclic aromatic skeleton because if so, then the glass transition temperature will increase and an effect of reducing the voltage of a light emitting element is great due to increased electron mobility. Moreover, considering the improvement in durable life of an element, the easiness of synthesis, and easy availability of raw materials, it is particularly preferable that the fused polycyclic aromatic skeleton is an anthracene skeleton, a pyrene skeleton, or a phenanthroline skeleton.
While the electron transporting material may be used alone, two or more kinds of the electron transporting materials may be used in combination, or one or more kinds of other electron transporting materials may be used in a combination with the electron transporting material.

Preferable electron transporting materials are not particularly limited, and specific examples thereof include the following.

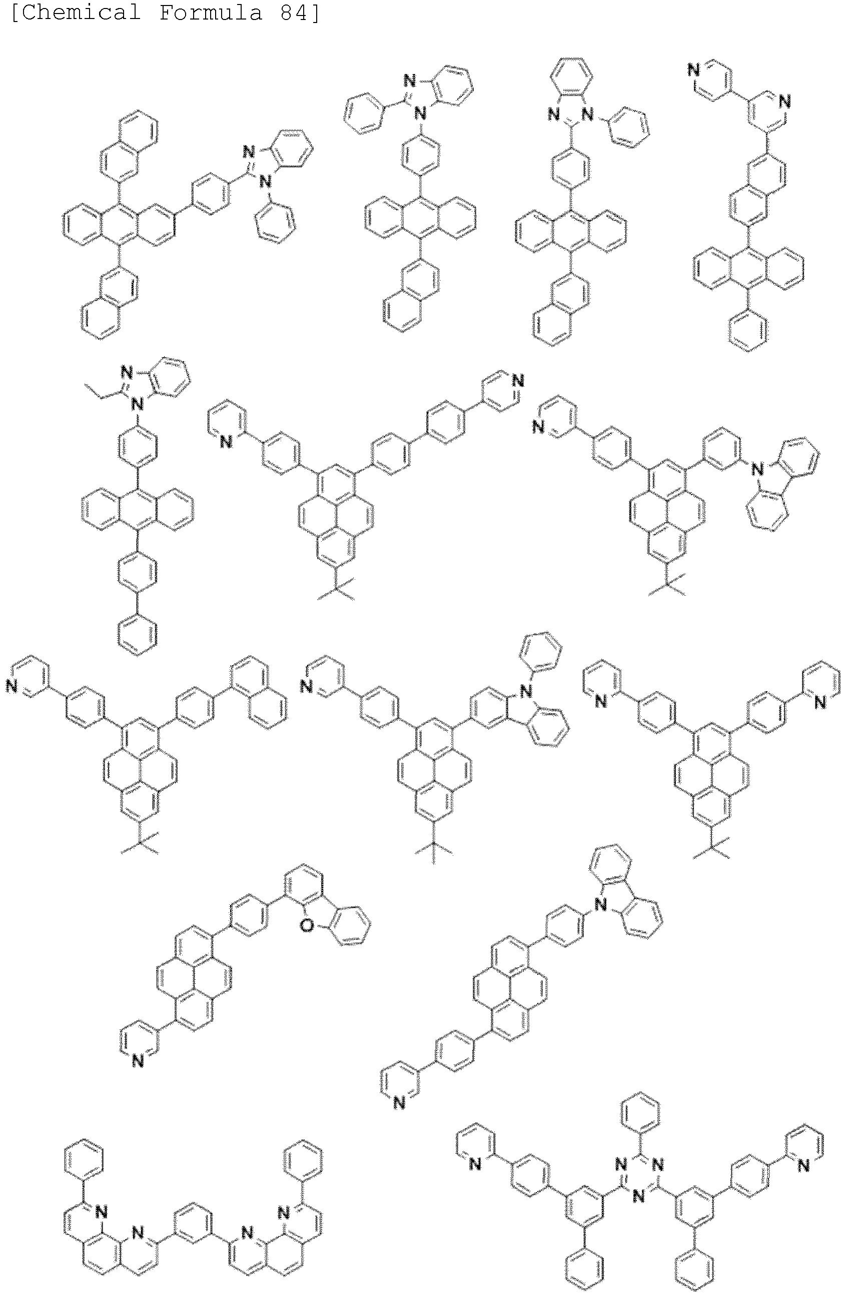

Besides these electron transporting materials, those disclosed in WO 2004/63159, WO 2003/60956, Appl. Phys. Lett. 74, 865 (1999), Org. Electron. 4, 113 (2003), WO 2010/113743 and WO 2010/1817 can be used.

The phenanthroline derivative of the present invention can also be suitably used as an electron transporting material because it has high electron injection/transporting abilities.

When the phenanthroline derivative of the present invention is used as an electron transporting material, it does not need to be restricted to each one type, and a plurality of phenanthroline derivatives according to the present invention may be used in admixture, or one or more of other electron transporting materials may be used in admixture with the phenanthroline derivative of the present invention as long as the effects of the present invention are not impaired. The electron transporting material that can be mixed is not particularly limited, and examples thereof include compounds having a fused aryl ring, such as naphthalene, anthracene and pyrene, and derivatives thereof, styryl-based aromatic ring derivatives typified by 4,4'-bis(diphenylethenyl)biphenyl, perylene derivatives, perinone derivatives, coumarin derivatives, naphthalimide derivatives, quinone derivatives such as anthraquinone and diphenoquinone, phosphorus oxide derivatives, carbazole derivatives and indole derivatives, quinolinol complexes such as lithium quinolinol, tris(8-quinolinolato)aluminum(III), hydroxyazole complexes such as hydroxyphenyloxazole complexes, azomethine complexes, tropolone metal complexes, and flavonol metal complexes.

While the electron transporting material may be used alone, two or more kinds of the electron transporting materials may be used in combination, or one or more kinds of other electron transporting materials may be used in a combination with the electron transporting material. Moreover, a donor material may be contained. The donor material denotes a compound which makes easy electron injection into the electron transporting layer from the cathode or the electron injection layer and, moreover, improves the electric conductivity of the electron transporting layer, by improving an electron injection barrier.

Preferable examples of the donor material in the present invention include an alkali metal, an inorganic salt containing an alkali metal, a complex of an alkali metal and an organic substance, an alkaline earth metal, an inorganic salt containing an alkaline earth metal, or a complex of an alkaline earth metal and an organic substance. Examples of the preferable kind of the alkali metal and the alkaline earth metal include alkali metals such as lithium, sodium and cesium, and alkaline earth metals such as magnesium and calcium which have a low work function and have a great effect of improving electron transporting ability.

In addition, since deposition in vacuum is easy and handling is excellent, the donor material is preferably in the state of an inorganic salt or a complex with an organic substance rather than a metal simple substance. Moreover, from the viewpoints of improvement in easiness in handling in the atmospheric air and easiness in control of the concentration to be added, the donor material is more preferably in the state of a complex with an organic substance. Examples of the inorganic salt include oxides such as LiO and Li₂O, nitrides, fluorides such as LiF, NaF and KF, and carbonates such as Li₂CO₃, Na₂CO₃, K₂CO₃, Rb₂CO₃ and Cs₂CO₃. Preferable examples of the alkali metal or alkaline earth metal include lithium from the viewpoints of an inexpensive raw material and ease of synthesis. In addition, preferable examples of the organic substance in complexes with an organic substance include quinolinol, benzoquinolinol, flavonol, hydroxyimidazopyridine, hydroxybenzazole, and hydroxytriazole. Particularly, a complex of an alkali metal and an organic substance is preferred, a complex of lithium and an organic substance is more preferred, and lithium quinolinol is especially preferred. Two or more of these donor materials may be used in admixture.

The preferred doping concentration varies depending on a material and a film thickness of the doping region, but for example when the donor material is an inorganic material such as an alkali metal or an alkaline earth metal, it is preferred that an electron transporting layer is formed by performing co-deposition so that the deposition rate ratio of an electron transporting material and a donor material is within the range of 10000 : 1 to 2 : 1. The deposition rate ratio is more preferably 100 : 1 to 5 : 1, further preferably 100 : 1 to 10 : 1. When the donor material is a complex of a metal and an organic substance, it is preferred that an electron transporting layer is formed by performing co-deposition so that the deposition rate ratio of an electron transporting material and the donor material is within the range of 100 : 1 to 1 : 100. The deposition rate ratio is more preferably 10 : 1 to 1 : 10, further preferably 7 : 3 to 3 : 7.

The method in which an electron transporting layer is doped with a donor material to improve an electron transporting ability exhibits an effect particularly when the film thickness of a thin-film layer is large. The method is particularly preferably used when the total film thickness of the electron transporting layer and the emissive layer is 50 nm or more. For example, there is a method in which an interference effect is used for improving luminous efficiency, and the method is intended to improve light extraction efficiency by matching the phases of light emitted directly from an emissive layer and light reflected at a cathode. The optimum conditions thereof vary depending on a light emitting wavelength, and the total film thickness of the electron transporting layer and the emissive layer becomes 50 nm or more, and may become a large film thickness close to 100 nm in the case of emission of light having a long wavelength, such as red light.

The film thickness of the electron transporting layer, which is doped, may be a part or the whole of the electron transporting layer. When a part of the electron transporting layer is doped, it is desirable to provide a doped-region at least at an electron transporting layer/cathode interface, and the effect of reducing a voltage is obtained by merely doping the vicinity of the cathode interface. On the other hand, when the donor material is in direct contact with the emissive layer, an adverse effect of reducing luminous efficiency may be caused, and in this case, it is preferred to provide a non-doped-region at an emissive layer/electron transporting layer interface.

### (Electron Injection Layer)

In the present invention, an electron injection layer may be provided between a cathode and an electron transporting layer . Generally, the electron injection layer is inserted for the purpose of helping injection of electrons from the cathode into the electron transporting layer, and when the electron injection layer is inserted, a compound having a heteroaryl ring structure containing electron-accepting nitrogen may be used, or a layer containing the above-mentioned donor material may be used. The phenanthroline derivative of the present invention may be contained in the electron injection layer.

An inorganic substance such as an insulator or a semiconductor can also be used for the electron injection layer. Use of such a material is preferred because a short-circuit of the light emitting element can be effectively prevented, and electron injection property can be improved.

It is preferred that at least one metal compound selected from the group consisting of an alkali metal chalcogenide, an alkaline earth metal chalcogenide, a halide of an alkali metal and a halide of an alkaline earth metal is used as the insulator. It is preferred that the electron injection layer is formed of the above-mentioned alkali metal chalcogenide and the like because electron injection property can be further improved.

Specifically, examples of the preferable alkali metal chalcogenide include Li₂O, Na₂S and Na₂Se, and examples of the preferable alkaline earth metal chalcogenide include CaO, BaO, SrO, BeO, BaS and CaSe. Examples of the preferable halide of an alkali metal include LiF, NaF, KF, LiCl, KCl and NaCl. Examples of the preferable halide of an alkaline earth metal include fluorides such as CaF₂, BaF₂, SrF₂, MgF₂ and BeF₂, and halides other than fluorides.

Further, a complex of an organic substance and a metal is suitably used. Use of a complex of an organic substance and a metal for the electron injection layer is preferred because the film thickness is easily adjusted. As examples of the above-mentioned organic metal complex, preferable examples of the organic substance in complexes with an organic substance include quinolinol, benzoquinolinol, pyridylphenol, flavonol, hydroxyimidazopyridine, hydroxybenzazole, and hydroxytriazole. Particularly, a complex of an alkali metal and an organic substance is preferred, a complex of lithium and an organic substance is more preferred, and lithium quinolinol is especially preferred.

### (Charge Generation Layer)

In the present invention, the charge generation layer is an intermediate layer existing between an anode and a cathode, and in the charge generation layer, holes and electrons are generated by charge separation. Generally, the charge generation layer is formed from a P-type layer on the cathode side and an N-type layer on the anode side. These layers are desired to perform efficient charge separation, and efficiently transport generated carriers.

For the P-type charge generation layer, materials to be used for the above-mentioned hole injection layer and hole transporting layer can be used. For example, benzidine derivatives such as HAT-CN6, NPD and TBDB; materials called starburst arylamine, such as m-MTDATA and 1-TNATA; and materials having skeletons represented by the general formulae (3) and (4) can be suitably used.

For the N-type charge generation layer, materials to be used for the above-mentioned electron injection layer and electron transporting layer can be used, and a compound having a heteroaryl ring structure containing electron-accepting nitrogen may be used, or a layer containing the above-mentioned donor material may be used. A layer in which the phenanthroline derivative of the present invention is doped with the donor material can also be suitably used.

Examples of a method of forming each of the aforementioned layers constituting the light emitting element include, but are not particularly limited to, resistance heating deposition, electron beam deposition, sputtering, a molecular lamination method, and a coating method, but usually, resistance heating deposition or electron beam deposition is preferable from the viewpoint of element property.

The thickness of the organic layer depends on the resistance value of an emissive substance and, therefore, it cannot be limited, but it is preferably 1 to 1000 nm. The film thickness of each of the emissive layer, the electron transporting layer and the hole transporting layer is preferably 1 nm or more and 200 nm or less, more preferably 5 nm or more and 100 nm or less.

The light emitting element of the present invention has a function of being able to convert electric energy into light. Herein, a direct current is mainly used as the electric energy, but a pulse current or an alternate current can also be used. A current value and a voltage value are not particularly limited, but when the power consumed and life of the element are considered, they should be selected so that the maximum luminance is obtained by energy as low as possible.

The light emitting element of the present invention is used suitably as a display that displays in a matrix and/or segment system.

In the matrix system, pixels for display are arranged two-dimensionally such as lattice-like arrangement or mosaic-like arrangement, and the collection of pixels displays letters and images. The shape and size of the pixel are determined depending on utility. For example, for displaying images and letters on personal computers, monitors and televisions, a square pixel being 300 µm or less at each side is usually used and, in the case of a large display such as a display panel, a pixel being millimeter order at each side is used. In the case of a monochromatic display, pixels having the same color may be arranged, and in the case of a color display, pixels having red, green and blue colors are arranged to perform display. In this case, typically, there are a delta type and a stripe type. A method of driving this matrix may be either a passive matrix driving method or an active matrix. The passive matrix driving has a simple structure, but when operation property is considered, the active matrix is more excellent in some cases, and it is necessary to use them properly depending on utility.

The segment system in the present invention is a system by which a pattern is formed so as to display predetermined information, and a region determined by arrangement of this pattern is made to emit light. Examples thereof include time and temperature displays in digital watches and thermometers, operating-state displays in audio equipment, IH cookers and so on, and panel displays of automobiles. The above-mentioned matrix display and segment display may exist together in the same panel.

The light emitting element of the present invention can also be preferably used as backlight of various instruments. Backlight is used mainly for the purpose of improving the visibility of display apparatuses which do not emit light by themselves, and is used in liquid crystal display equipment, clocks, audio equipment, automobile panels, display panels, signs, and the like. In particular, the light emitting element of the present invention is preferably used in backlight for liquid crystal display equipment, inter alia, for personal computers which are studied to be thinned, and can provide backlight thinner and lighter than conventional products. Examples

The present invention will be described by way of Examples, but the present invention is not limited thereto.

### Synthesis Example 1

### Synthesis of Compound [A-1]

12.1 g of 2-acetylpyridine, 17.2 g of 8-aminoquinoline-7-carboaldehyde, 14.0 g of potassium hydroxide and 1000 mL of ethanol were mixed, and the mixture was purged with nitrogen, and then heated and refluxed. After 4.5 hours, the mixture was cooled to room temperature, 500 mL of toluene and 1000 mL of water were then added, and the liquid was separated. The aqueous layer was extracted with 500 mL of toluene twice, and then combined with the foregoing organic layer, and ethanol was distilled off under reduced pressure. The solution was dried with magnesium sulfate, and the solvent was distilled off under reduced pressure, followed by performing vacuum-drying to obtain 23.9 g of an intermediate [a].

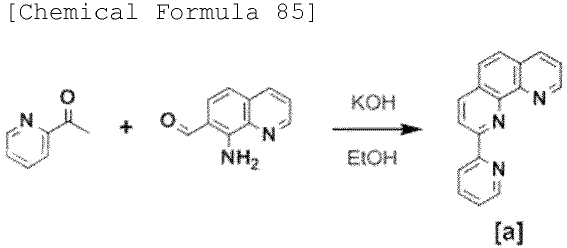

Next, 6.13 g of 1-bromo-4-chlorobenzene and 20.0 mL of dibutyl ether were mixed, and the mixture was cooled to -10°C. To this was added 20.0 mL of n-butyllithium (1.6 M, hexane solution), and the mixture was heated to 0°C, and then stirred for 30 minutes. This adjustment liquid was slowly added to a liquid obtained by mixing 8.23 g of the intermediate [a] and 165 mL of toluene, and cooling the mixture to -10°C. In addition of the liquid, adjustment was performed so that the reaction liquid temperature was -5°C or lower. After addition of the liquid, the mixture was stirred at -5°C or lower for 1 hour, and then quenched by adding 200 mL of water. The aqueous layer was removed, the organic layer was then washed with 100 mL of water three times, and dried with anhydrous sodium sulfate, and the solvent was distilled off under reduced pressure. To the resultant solid was added 300 mL of tetrahydrofuran to dissolve the solid, 16.7 g of manganese dioxide was then added, and the mixture was stirred at room temperature. After 10 hours, 200 mL of tetrahydrofuran was added, and insolubles were then removed by filtration with celite. The solvent of the filtrate was distilled off under reduced pressure, the resultant solid was then re-crystallized with toluene/cyclohexane, and the resultant solid was filtered, and vacuum-dried to obtain 9.39 g of an intermediate [b].

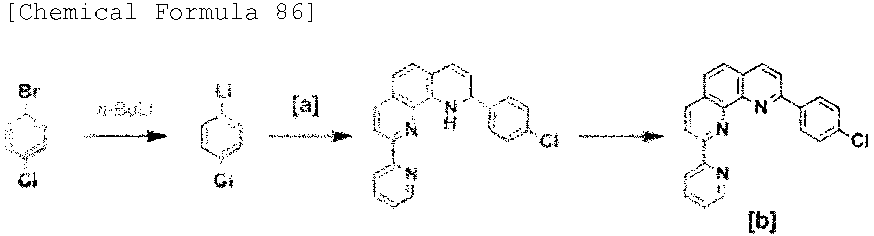

Next, 2.15 g of the intermediate [b], 1.59 g of 1-pyreneboronic acid, 60.0 mL of 1,4-dioxane, and 10.2 mL of a 1.27 M potassium phosphate aqueous solution were mixed, and the mixture was purged with nitrogen. To this mixed solution were added 135 mg of bis (dibenzylideneacetone) palladium (0) and 129 mg of tricyclohexyl phosphine tetrafluoroborane, and the mixture was heated and refluxed for 3.5 hours. The mixture was cooled to room temperature, 100 mL of water was then added, and the precipitate was filtered. After vacuum-drying, 400 mL of tetrahydrofuran was added, the mixture was heated and dissolved, the solution was cooled to a temperature near 50°C, 313 mg of activated carbon was added, and the mixture was heated and refluxed for 1 hour. The mixture was cooled, and then filtered with a silica pad. The solvent of the filtrate was distilled off, the resultant solid was then re-crystallized with o-xylene, and the resultant solid was filtered. Further, the solid was re-crystallized with o-xylene again, vacuum-dried, and then re-crystallized with pyridine/methanol. The resultant solid was filtered, and vacuum-dried to obtain 1.97 g of a compound [A-1] as a yellow solid. The compound was identified by mass spectrometry.

The compound [A-1] was used as a light emitting element material after sublimation purification was performed at about 300°C under a pressure of 1 × 10⁻³ Pa using an oil diffusion pump.

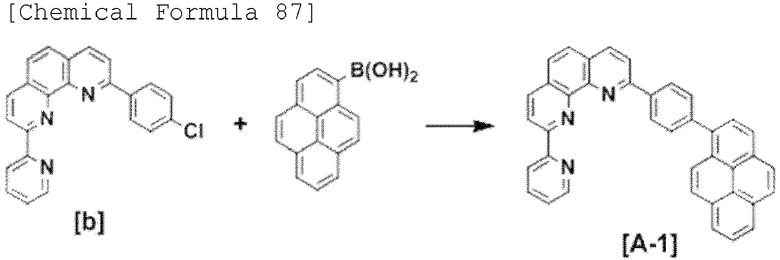

### Synthesis Example 2

### Synthesis of Compound [A-2]

12.1 g of 3-acetylpyridine, 17.2 g of 8-aminoquinoline-7-carboaldehyde, 14.0 g of potassium hydroxide and 1000 mL of ethanol were mixed, and the mixture was purged with nitrogen, and then heated and refluxed. After 4.5 hours, the mixture was cooled to room temperature, 500 mL of toluene and 1000 mL of water were then added, and the liquid was separated. The aqueous layer was extracted with 500 mL of toluene twice, and then combined with the foregoing organic layer, and ethanol was distilled off under reduced pressure. The solution was dried with anhydrous sodium sulfate, and the solvent was distilled off under reduced pressure, followed by performing vacuum-drying to obtain 14.4 g of an intermediate [c].

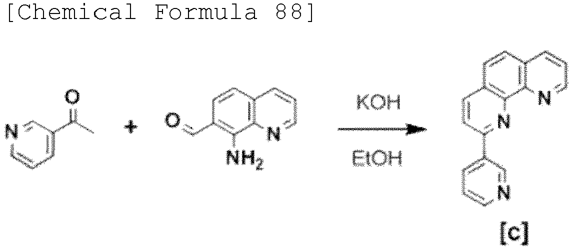

Next, 6.13 g of 1-bromo-3-chlorobenzene and 20.0 mL of dibutyl ether were mixed, and the mixture was cooled to -10°C. To this was added 20.0 mL of n-butyllithium (1.6 M, hexane solution), and the mixture was heated to 0°C, and then stirred for 30 minutes. This adjustment liquid was slowly added to a liquid obtained by mixing 8.23 g of the intermediate [c] and 165 mL of toluene, and cooling the mixture to -10°C. In addition of the liquid, adjustment was performed so that the reaction liquid temperature was -5°C or lower. After addition of the liquid, the mixture was stirred at -5°C or lower for 1 hour, and then quenched by adding 200 mL of water. The aqueous layer was removed, the organic layer was then washed with 100 mL of water three times, and dried with anhydrous sodium sulfate, and the solvent was distilled off under reduced pressure. To the resultant solid was added 300 mL of tetrahydrofuran to dissolve the solid, 16.7 g of manganese dioxide was then added, and the mixture was stirred at room temperature. After 8 hours, 200 mL of tetrahydrofuran was added, and insolubles were then removed by filtration with celite. The solvent of the filtrate was distilled off under reduced pressure, the resultant solid was then re-crystallized with toluene/cyclohexane, and the resultant solid was filtered, and vacuum-dried to obtain 9.34 g of an intermediate [d].

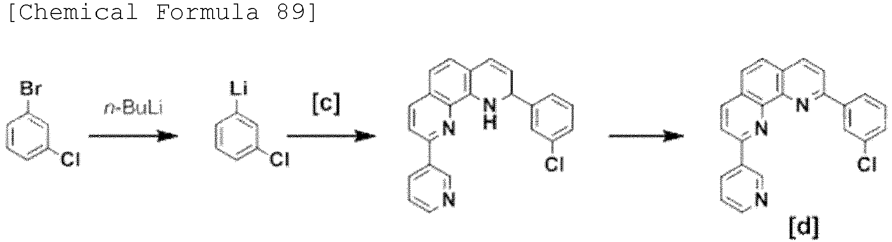

Next, 2.21 g of the intermediate [b], 1.62 g of 3-fluorantheneboronic acid, 60.0 mL of 1,4-dioxane, and 10.4 mL of a 1.27 M potassium phosphate aqueous solution were mixed, and the mixture was purged with nitrogen. To this mixed solution were added 138 mg of bis(dibenzylideneacetone)palladium (0) and 133 mg of tricyclohexyl phosphine tetrafluoroborane, and the mixture was heated and refluxed for 3 hours. The mixture was cooled to room temperature, 100 mL of water was then added, and the precipitate was filtered. After vacuum-drying, 300 mL of tetrahydrofuran was added, the mixture was dissolved, the solution was cooled, 313 mg of activated carbon was added, and the mixture was heated and refluxed for 1 hour. The mixture was cooled, and then filtered with a silica pad. The solvent of the filtrate was distilled off, and the resultant solid was then re-crystallized with pyridine/methanol. The resultant solid was filtered, and vacuum-dried to obtain 2.55 g of a compound [A-2] as a yellow solid. The compound was identified by mass spectrometry.

The compound [A-1] was used as a light emitting element material after sublimation purification was performed at about 300°C under a pressure of 1 × 10⁻³ Pa using an oil diffusion pump.

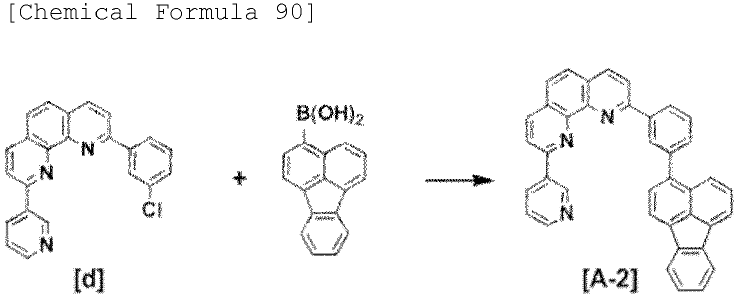

### Synthesis Example 3

### Synthesis of Compound [A-3]

15.5 g of 4'-chloroacetophenone, 17.2 g of 8-aminoquinoline-7-carboaldehyde, 14.0 g of potassium hydroxide and 1000 mL of ethanol were mixed, and the mixture was purged with nitrogen, and then heated and refluxed. After 4.5 hours, the mixture was cooled to room temperature, 500 mL of toluene and 1000 mL of water were then added, and the liquid was separated. The aqueous layer was extracted with 500 mL of toluene twice, and then combined with the foregoing organic layer, and ethanol was distilled off under reduced pressure. The solution was dried with anhydrous sodium sulfate, and the solvent was distilled off under reduced pressure, followed by performing vacuum-drying to obtain 25.9 g of an intermediate [e].

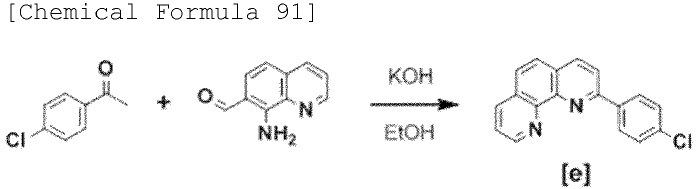

Next, 13.6 g of the intermediate [e], 17.8 g of bis (pinacolato) diboron, 13.8 g of potassium acetate and 468 mL of 1, 4-dioxane were mixed, the mixture was purged with nitrogen, 538 mg of bis (dibenzylideneacetone) palladium (0) and 892 mg of 2-dicyclohexylphosphino-2',4',6'-triisopropylbiphenyl were then added, and the mixture was heated and refluxed for 3 hours . The mixture was cooled to room temperature, the precipitate was then filtered, and the solvent of the filtrate was distilled off. The resultant solid was washed with 100 mL of water twice, and 50 mL of heptane twice, and then vacuum-dried to obtain 15.2 g of an intermediate [f].

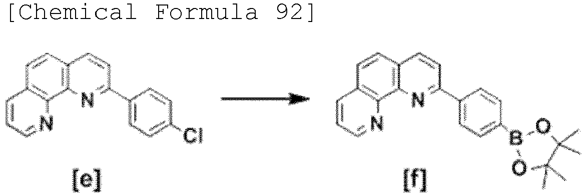

Next, 12.5 g of the intermediate [f], 4.11 g of 8-chloroquinoline, 7.47 g of potassium phosphate and 251 mL of toluene were mixed, the mixture was purged with nitrogen, 289 mg of bis(dibenzylideneacetone)palladium (0) and 292 mg of tri-tert-butylphosphine tetrafluoroborane were then added, and the mixture was heated and refluxed for 2 hours . The mixture was cooled to room temperature, 250 mL of water was then added, and the precipitated solid was filtered with celite. The filtrate was separated, the separated organic layer was washed with 200 mL of water twice, and the solvent of the organic layer was then distilled off under reduced pressure. The resultant solid was heated and dissolved in 100 mL of pyridine, 963 mg of activated carbon and 1.50 g of "QuadraSil" (registered trademark) were then added, and the mixture was stirred at 100°C for 1 hour, and then filtered with celite at room temperature. The solvent of the filtrate was distilled off under reduced pressure, the resultant solid was then re-crystallized with o-xylene, and the resultant solid was filtered, and vacuum-dried to obtain 8.96 g of an intermediate [g].

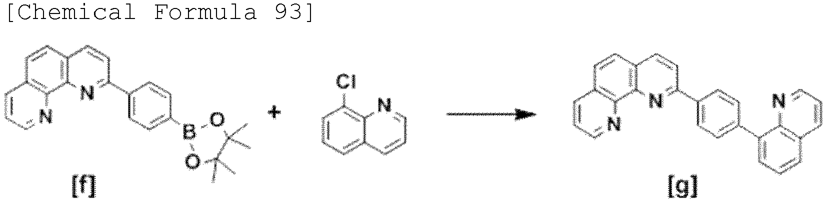

Next, 4.53 g of 1-bromopyrene and 40.0 mL of dibutyl ether were mixed, and the mixture was cooled to -10°C. To this was added 10.0 mL of n-butyllithium (1.6 M, hexane solution), and the mixture was heated to 0°C, and then stirred for 30 minutes. This adjustment liquid was slowly added to a liquid obtained by mixing 6.13 g of the intermediate [g] and 82.0 mL of toluene, and cooling the mixture to -10°C. In addition of the liquid, adjustment was performed so that the reaction liquid temperature was -5°C or lower. After addition of the liquid, the mixture was stirred at -5°C or lower for 2 hours, and then quenched by adding 100 mL of water. 100 mL of toluene was added, the liquid was separated, and the aqueous layer was then removed. The organic layer was washed with 100 mL of water twice, and dried with anhydrous sodium sulfate, and the solvent was distilled off under reduced pressure. To the resultant solid was added 400 mL of tetrahydrofuran to dissolve the solid, 6.96 g of manganese dioxide was then added, and the mixture was stirred at room temperature. After 7 hours, insolubles were removed by filtration with celite, and the solvent of the filtrate was distilled off under reduced pressure. The resultant solid was then re-crystallized with toluene, and the resultant solid was further re-crystallized with pyridine. Re-crystallization was repeated once again, and the resultant solid was filtered, and vacuum-dried to obtain 5.22 g of a compound [A-3] as a yellow solid. The compound was identified by mass spectrometry.

The compound [A-3] was used as a light emitting element material after sublimation purification was performed at about 290°C under a pressure of 1 × 10⁻³ Pa using an oil diffusion pump.

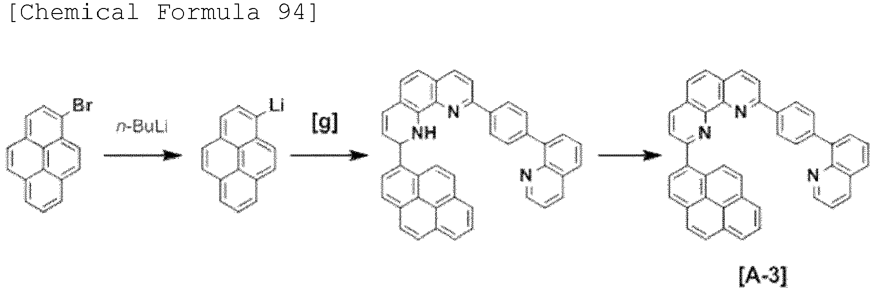

### Synthesis Example 4

### Synthesis of Compound [A-4]

8.20 g of the intermediate [e], 6.80 g of 3-fluorantheneboronic acid, 282.0 mL of 1,4-dioxane, and 48.9 mL of a 1.27 M potassium phosphate aqueous solution were mixed, and the mixture was purged with nitrogen. To this mixed solution were added 578 mg of bis(dibenzylideneacetone)palladium (0) and 555 mg of tricyclohexyl phosphine tetrafluoroborane, and the mixture was heated and refluxed for 2 hours. The mixture was cooled to room temperature, 300 mL of water was then added, and the precipitate was filtered. After vacuum-drying, 800 mL of tetrahydrofuran was added, the mixture was dissolved, the solution was cooled, 1.29 mg of activated carbon was added, and the mixture was heated and refluxed for 1 hour. The mixture was cooled, and then filtered with a silica pad. The solvent of the filtrate was distilled off, and the resultant solid was then re-crystallized with pyridine/methanol. The resultant solid was filtered, and vacuum-dried to obtain 10.9 g of an intermediate [h].

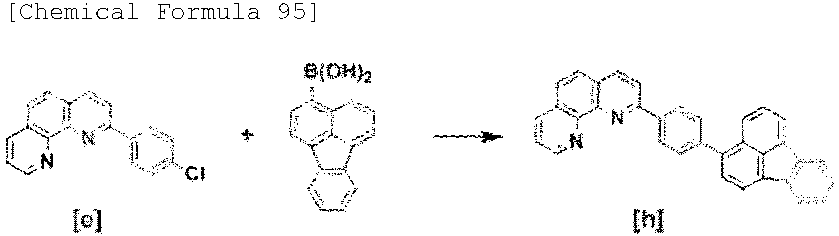

Next, 3.74 g of 2-(4-bromophenyl) pyridine and 40.0 mL of dibutyl ether were mixed, and the mixture was cooled to -10°C. To this was added 10.0 mL of n-butyllithium (1.6 M, hexane solution), and the mixture was heated to 0°C, and then stirred for 30 minutes. This adjustment liquid was slowly added to a liquid obtained by mixing 7.30 g of the intermediate [h] and 82.0 mL of toluene, and cooling the mixture to -10°C. In addition of the liquid, adjustment was performed so that the reaction liquid temperature was -5°C or lower. After addition of the liquid, the mixture was stirred at -5°C or lower for 2 hours, and then quenched by adding 100 mL of water. 100 mL of toluene was added, the liquid was separated, and the aqueous layer was then removed. The organic layer was washed with 100 mL of water twice, and dried with anhydrous sodium sulfate, and the solvent was distilled off under reduced pressure. To the resultant solid was added 500 mL of tetrahydrofuran to dissolve the solid, 6.96 g of manganese dioxide was then added, and the mixture was stirred at room temperature. After 8 hours, insolubles were removed by filtration with celite, and the solvent of the filtrate was distilled off under reduced pressure. The resultant solid was then re-crystallized with o-xylene, and further re-crystallized with pyridine. The resultant solid was filtered, and vacuum-dried to obtain 5.41 g of a compound [A-4] as a yellow solid. The compound was identified by mass spectrometry.

The compound [A-4] was used as a light emitting element material after sublimation purification was performed at about 310°C under a pressure of 1 × 10⁻³ Pa using an oil diffusion pump.

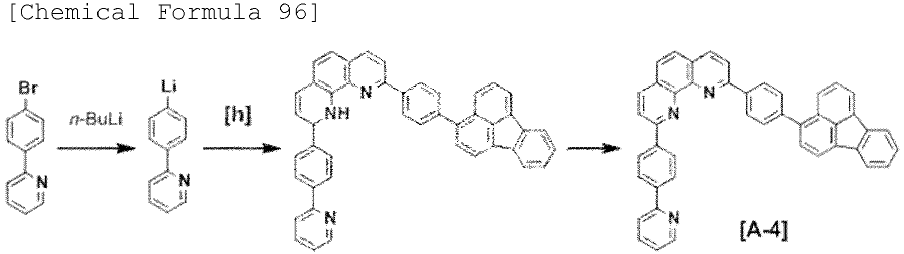

In examples below, compounds B-1 to B-12 are the compounds shown below, wherein compounds B-10 and B-12 do not fall under the scope of the present invention.

Formulas marked with "☆" do not fall under the scope of the present invention.

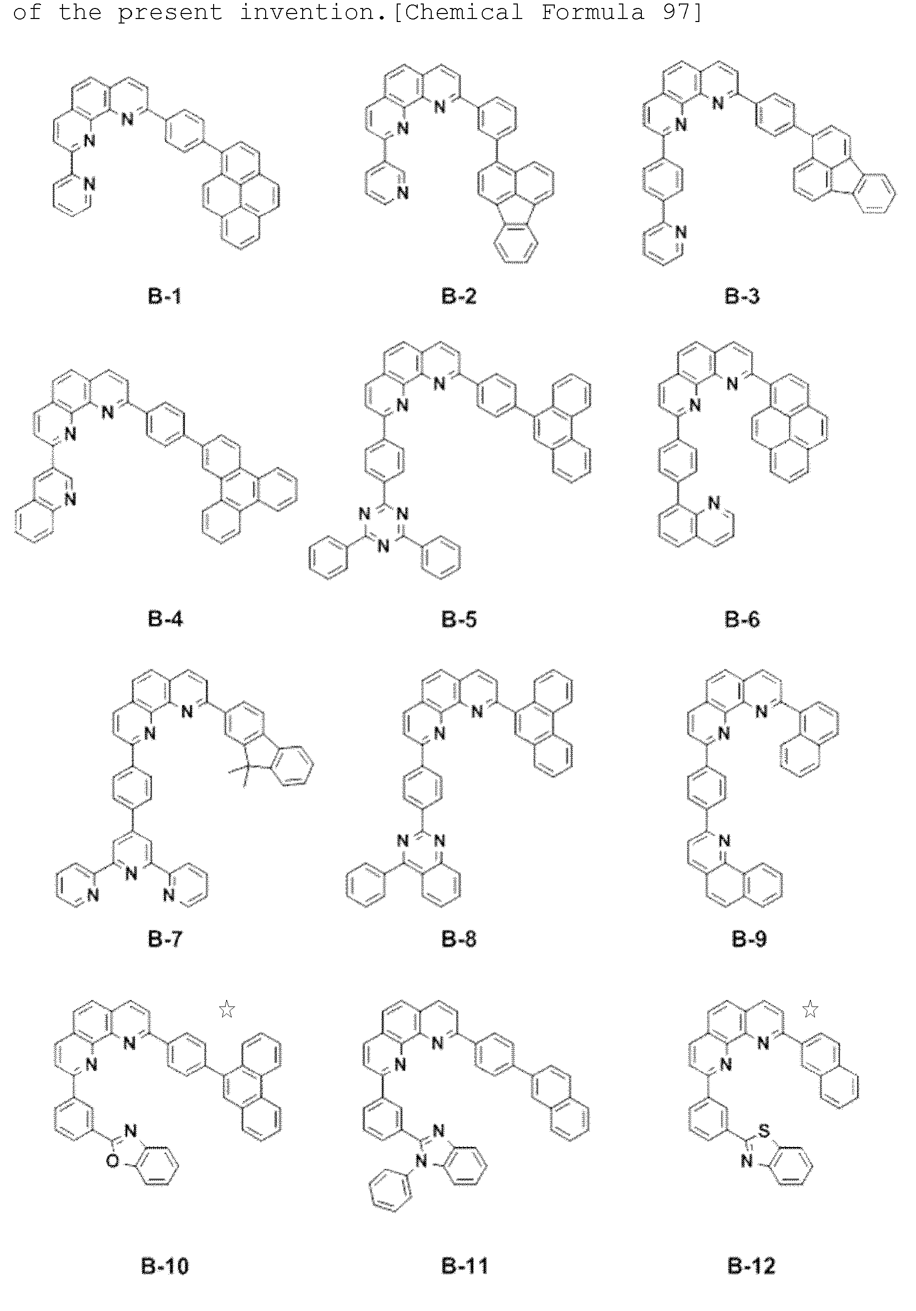

### Example 1

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of an element, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 50 nm. Next, as an emissive layer, a host material H-1 and a dopant material D-1 were deposited in a thickness of 20 nm so that the doping concentration was 5% by weight. Next, as an electron transporting layer, the compound B-1 was deposited and laminated in a thickness of 35 nm. Next, lithium fluoride was deposited in a thickness of 0.5 nm, and aluminum was then deposited in a thickness of 1000 nm to form a cathode, so that a 5 × 5 mm square element was prepared. The film thickness referred to herein is an indicated value on a crystal oscillation film thickness monitor. The properties of the light emitting element at 1000 cd/m² included a driving voltage of 4.3 V and an external quantum efficiency of 4.8%. When the light emitting element was driven at a constant current with the initial luminance set to 1000 cd/m², the time at which the luminance decreased by 20% was 1500 hours. Compounds HAT-CN6, HT-1, H-1 and D-1 are the compounds shown below.

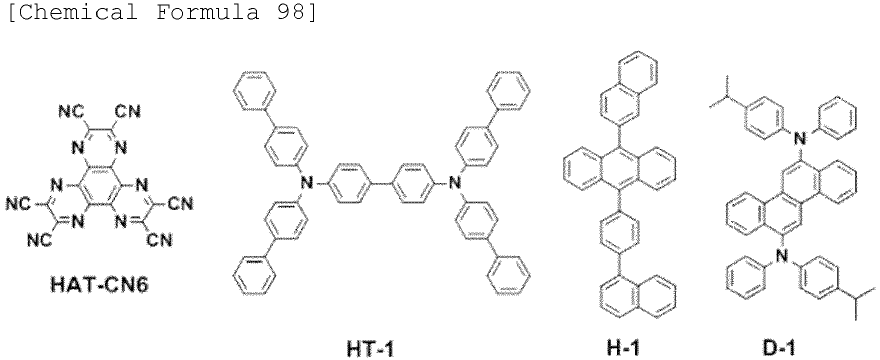

Examples 2 to 9 and 11 and Reference Examples 10 and 12 In the same manner as in Example 1 except that compounds described in Table 1 were used for the electron transporting layer, light emitting elements were prepared and evaluated. The results are shown in Table 1.

### Comparative Examples 1 to 5

In the same manner as in Example 1 except that compounds described in Table 1 were used for the electron transporting layer, light emitting elements were prepared and evaluated. The results are shown in Table 1. E-1 to E-5 are the compounds shown below.

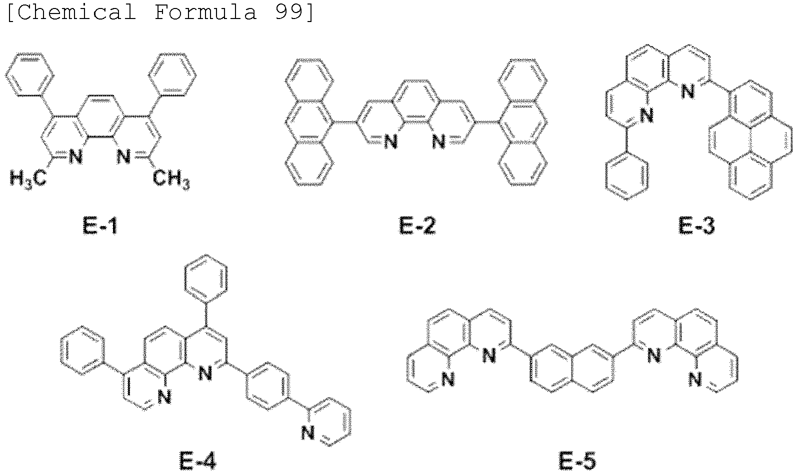

### Example 13

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of an element, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 50 nm. Next, as an emissive layer, a host material H-1 and a dopant material D-1 were deposited in a thickness of 20 nm so that the doping concentration was 5% by weight. Next, as a first electron transporting layer, the compound B-1 was deposited and laminated in a thickness of 25 nm. Further as a second electron transporting layer, the compound B-1 used as an electron transporting material and lithium used as a donor material were laminated in a thickness of 10 nm so that the deposition rate ratio of the compound B-1 and lithium was 20 : 1. Next, lithium fluoride was deposited in a thickness of 0.5 nm, and aluminum was then deposited in a thickness of 1000 nm to form a cathode, so that a 5 × 5 mm square element was prepared. The properties of the light emitting element at 1000 cd/m² included a driving voltage of 3.9 V and an external quantum efficiency of 5.8%. When the light emitting element was driven at a constant current with the initial luminance set to 1000 cd/m², the time at which the luminance decreased by 20% was 1650 hours.

Examples 13 to 21 and 23 and Reference Examples 22 and 24

In the same manner as in Example 13 except that compounds described in Table 2 were used for the electron transporting layer, light emitting elements were prepared and evaluated. The results are shown in Table 2.

### Comparative Examples 6 to 10

In the same manner as in Example 13 except that compounds described in Table 2 were used for the electron transporting layer, light emitting elements were prepared and evaluated. The results are shown in Table 2.

### Example 25

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of an element, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 50 nm. Next, as an emissive layer, a host material H-1 and a dopant material D-1 were deposited in a thickness of 20 nm so that the doping concentration was 5% by weight. Further as an electron transporting layer, the compound B-1 used as an electron transporting material and 2E-1 used as a donor material were laminated in a thickness of 35 nm so that the deposition rate ratio of the compound B-1 and 2E-1 was 1 : 1. This electron transporting layer is shown as a second electron transporting layer in Table 4. Next, lithium fluoride was deposited in a thickness of 0.5 nm, and magnesium and silver were then co-deposited in a thickness of 1000 nm to form a cathode, so that a 5 × 5 mm square element was prepared. The properties of the light emitting element at 1000 cd/m² included a driving voltage of 3.9 V and an external quantum efficiency of 6.0%. When the light emitting element was driven at a constant current with the initial luminance set to 1000 cd/m², the time at which the luminance decreased by 20% was 1800 hours. 2E-1 is the compound shown below.

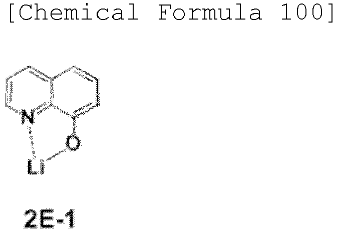

Examples 26 to 33 and 35 and Reference Examples 34 and 36

In the same manner as in Example 25 except that compounds described in Table 3 were used for the electron transporting layer and the donor material, light emitting elements were prepared and evaluated. The results are shown in Table 3.

### Comparative Examples 11 to 15

In the same manner as in Example 25 except that compounds described in Table 3 were used for the electron transporting layer and the donor material, light emitting elements were prepared and evaluated. The results are shown in Table 3.

### Example 37

As a first electron transporting layer, the compound B-1 was deposited in a thickness of 25 nm, and laminated, and further, as a second electron transporting layer, the compound B-1 used as an electron transporting material and 2E-1 used as a donor material were laminated in a thickness of 10 nm so that the deposition rate ratio of the compound B-1 and 2E-1 was 1 : 1. Otherwise in the same manner as in Example 25, a light emitting element was prepared. The properties of the light emitting element at 1000 cd/m² included a driving voltage of 4.0 V and an external quantum efficiency of 5.9%. When the light emitting element was driven at a constant current with the initial luminance set to 1000 cd/m², the time at which the luminance decreased by 20% was 1950 hours.

### Examples 38 to 45 and 47 and Reference Examples 46 and 48

In the same manner as in Example 37 except that compounds described in Table 4 were used for the electron transporting layer and the donor material, light emitting elements were prepared and evaluated. The results are shown in Table 4.

### Comparative Examples 15 to 20

In the same manner as in Example 37 except that compounds described in Table 4 were used for the electron transporting layer and the donor material, light emitting elements were prepared and evaluated. The results are shown in Table 4.

### Example 49

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of an element, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 50 nm. This hole transporting layer is shown as a first hole transporting layer in Table 6. Next, as an emissive layer, a host material H-2 and a dopant material D-2 were deposited in a thickness of 20 nm so that the doping concentration was 10% by weight. Next, as an electron transporting layer, the compound B-2 was deposited and laminated in a thickness of 35 nm. Next, lithium fluoride was deposited in a thickness of 0.5 nm, and aluminum was then deposited in a thickness of 1000 nm to form a cathode, so that a 5 × 5 mm square element was prepared. The film thickness referred to herein is an indicated value on a crystal oscillation film thickness monitor. The properties of the light emitting element at 4000 cd/m² included a driving voltage of 3.9 V and an external quantum efficiency of 10.4%. When the light emitting element was driven at a constant current with the initial luminance set to 4000 cd/m², the time at which the luminance decreased by 20% was 1400 hours. H-2 and D-2 are the compounds shown below.

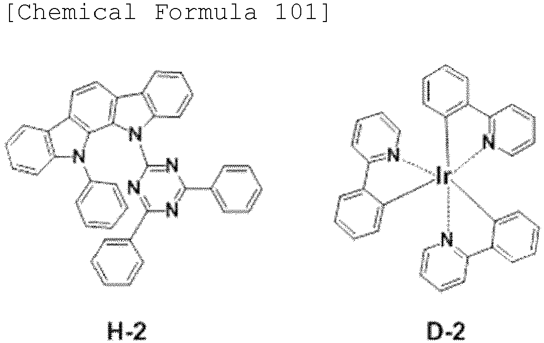

### Examples 50 to 52 and 54 and Reference Example 53

In the same manner as in Example 49 except that compounds described in Table 5 were used for the electron transporting layer, light emitting elements were prepared and evaluated. The results are shown in Table 5.

### Comparative Examples 21 to 23

In the same manner as in Example 49 except that compounds described in Table 5 were used for the electron transporting layer, light emitting elements were prepared and evaluated. The results are shown in Table 5.

### Example 55

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of an element, and placed in a vacuum deposition apparatus, and the air was evacuated until the degree of vacuum in the apparatus was 5 × 10⁻⁴ Pa or lower. By a resistance heating method, first, HAT-CN₆ was deposited as a hole injection layer in a thickness of 5 nm, and HT-1 was deposited as a first hole transporting layer in a thickness of 40 nm. Further, HT-2 was deposited as a second hole transporting layer in a thickness of 10 nm. Next, as an emissive layer, a host material H-2 and a dopant material D-2 were deposited in a thickness of 20 nm so that the doping concentration was 10% by weight. Next, as an electron transporting layer, the compound B-2 was deposited and laminated in a thickness of 35 nm. Next, lithium fluoride was deposited in a thickness of 0.5 nm, and aluminum was then deposited in a thickness of 1000 nm to form a cathode, so that a 5 × 5 mm square element was prepared. The film thickness referred to herein is an indicated value on a crystal oscillation film thickness monitor. The properties of the light emitting element at 4000 cd/m² included a driving voltage of 3.9 V and an external quantum efficiency of 13.3%. When the light emitting element was driven at a constant current with the initial luminance set to 4000 cd/m², the time at which the luminance decreased by 20% was 1600 hours. HT-2 is the compound shown below.

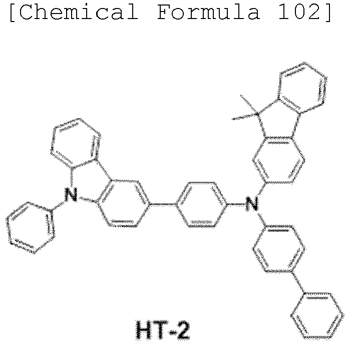

### Examples 56 to 65, 68 and 69 and Reference Examples 66 and 67

In the same manner as in Example 55 except that compounds described in Table 5 were used for the second hole transporting layer and the electron transporting layer, elements were prepared and evaluated. The results are shown in Table 5. HT-3, HT-4 and HT-5 are the compounds shown below.

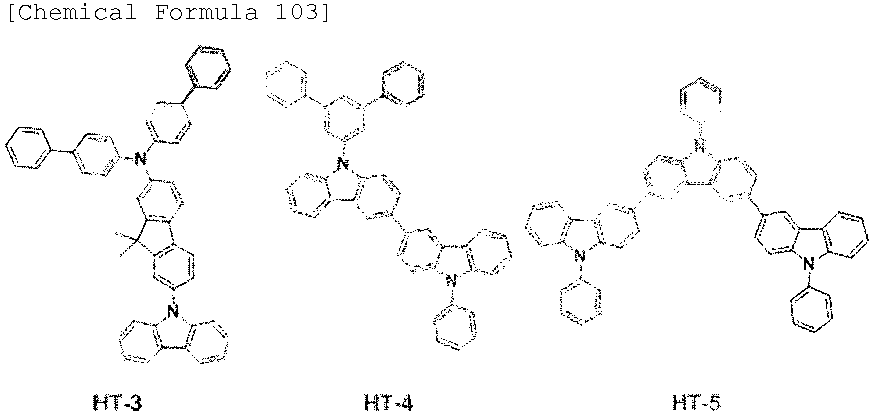

### Comparative Examples 24 to 31

In the same manner as in Example 55 except that compounds described in Table 5 were used for the second hole transporting layer and the electron transporting layer, elements were prepared and evaluated. The results are shown in Table 5.

### Example 70

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of an element, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 10 nm, and HT-6 was deposited as a hole transporting layer in a thickness of 90 nm. Next, as an emissive layer, a host material H-1 and a dopant material D-3 were deposited in a thickness of 30 nm so that the doping concentration was 5% by weight, and as an electron transporting layer, a compound ET-1 was deposited in a thickness of 30 nm, and laminated on the emissive layer. Further as an N-type charge generation layer, the compound B-1 used as an electron transporting material and lithium used as a donor material were laminated in a thickness of 10 nm so that the deposition rate ratio of the compound B-1 and lithium was 20 : 1, and as a P-type charge generation layer, HT-6 was deposited in a thickness of 10 nm on the N-type charge generation layer. Further, a hole injection layer, a hole transporting layer, an emissive layer and an electron transporting layer were laminated under the same conditions as described above, lithium fluoride was then deposited in a thickness of 0.5 nm, and aluminum was then deposited in a thickness of 1000 nm to form a cathode, so that a 5 × 5 mm square element was prepared. The properties of the light emitting element at 1000 cd/m² included a driving voltage of 8.7 V and an external quantum efficiency of 6.9%. When the light emitting element was driven at a constant current with the initial luminance set to 1000 cd/m², the time at which the luminance decreased by 20% was 1000 hours . HT-6, ET-1 and D-3 are the compounds shown below.

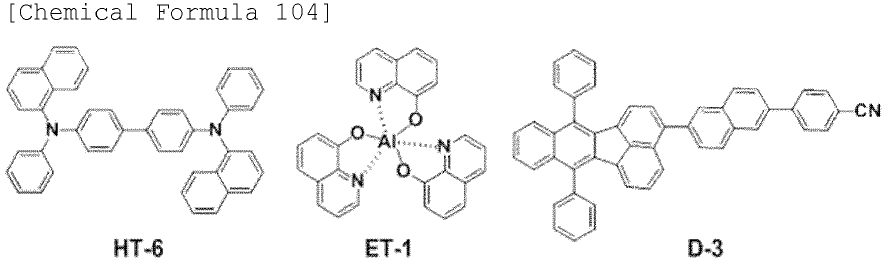

### Examples 71 to 74 and 76 and Reference Example 75

In the same manner as in Example 70 except that compounds described in Table 6 were used as an electron transporting material for the N-type charge generation layer, elements were prepared and evaluated. The results are shown in Table 6.

### Comparative Examples 32 to 36

In the same manner as in Example 70 except that compounds described in Table 6 were used as an electron transporting material for the N-type charge generation layer, elements were prepared and evaluated. The results are shown in Table 6.

**[Table 1]**

| | Emissive material | | | Electron transporting layer | Cathode | External quantum efficiency (%) | Driving voltage (V) | Durability (h) |
|---|---|---|---|---|---|---|---|---|
| | Host material | Dopant material | Emitted color | Compound | Metal | | | |
| Example 1 | H-1 | D-1 | Blue | B-1 | Al | 4.8 | 4.3 | 1500 |
| Example 2 | | | Blue | B-2 | Al | 4.7 | 4.3 | 1550 |
| Example 3 | | | Blue | B-3 | Al | 4.6 | 4.4 | 1500 |
| Example 4 | | | Blue | B-4 | Al | 4.5 | 4.4 | 1400 |
| Example 5 | | | Blue | B-5 | Al | 4.4 | 4.5 | 1300 |
| Example 6 | | | Blue | B-6 | Al | 4.2 | 4.7 | 1100 |
| Example 7 | | | Blue | B-7 | Al | 4.1 | 4.7 | 1000 |
| Example 8 | | | Blue | B-8 | Al | 4.0 | 4.8 | 1000 |
| Example 9 | | | Blue | B-9 | Al | 4.0 | 4.9 | 900 |
| Reference Example 10 | | | Blue | B-10 | Al | 4.1 | 4.7 | 1000 |
| Example 11 | | | Blue | B-11 | Al | 3.6 | 5.1 | 750 |
| Reference Example 12 | | | Blue | B-12 | Al | 3.6 | 5.1 | 700 |
| Comparative Example 1 | H-1 | D-1 | Blue | E-1 | Al | 2.3 | 5.9 | 400 |
| Comparative Example 2 | | | Blue | E-2 | Al | 2.5 | 5.7 | 400 |
| Comparative Example 3 | | | Blue | E-3 | Al | 2.9 | 5.5 | 400 |
| Comparative Example 4 | | | Blue | E-4 | Al | 3.2 | 5.4 | 500 |
| Comparative Example 5 | | | Blue | E-5 | Al | 3.3 | 5.3 | 500 |

**[Table 2]**

| | Emissive material | | | First electron transporting layer | Second electron transporting layer | | Cathode | External quantum efficiency (%) | Driving voltage (V) | Durability (h) |
|---|---|---|---|---|---|---|---|---|---|---|
| | Host material | Dopant material | Emitted color | Compound | Compound | Donor compound | Metal | | | |
| Example 13 | H-1 | D-1 | Blue | B-1 | B-1 | Li | Al | 5.8 | 3.9 | 1650 |
| Example 14 | | | Blue | B-2 | B-2 | Li | Al | 5.7 | 3.9 | 1750 |
| Example 15 | | | Blue | B-3 | B-3 | Li | Al | 5.6 | 4.0 | 1700 |
| Example 16 | | | Blue | B-4 | B-4 | Li | Al | 5.5 | 4.1 | 1600 |
| Example 17 | | | Blue | B-5 | B-5 | Li | Al | 5.5 | 4.2 | 1500 |
| Example 18 | | | Blue | B-6 | B-6 | Li | Al | 5.2 | 4.3 | 1250 |
| Example 19 | | | Blue | B-7 | B-7 | Li | Al | 5.1 | 4.4 | 1200 |
| Example 20 | | | Blue | B-8 | B-8 | Li | Al | 5.0 | 4.5 | 1150 |
| Example 21 | | | Blue | B-9 | B-9 | Li | Al | 4.9 | 4.5 | 1050 |
| Reference Example 22 | | | Blue | B-10 | B-10 | Li | Al | 5.0 | 4.4 | 1150 |
| Example 23 | | | Blue | B-11 | B-11 | Li | Al | 4.2 | 4.7 | 900 |
| Reference Example 24 | | | Blue | B-12 | B-12 | Li | Al | 4.2 | 4.8 | 800 |
| Comparative Example 6 | H-1 | D-1 | Blue | E-1 | E-1 | Li | Al | 2.7 | 5.7 | 400 |
| Comparative Example 7 | | | Blue | E-2 | E-2 | Li | Al | 3.0 | 5.5 | 400 |
| Comparative Example 8 | | | Blue | E-3 | E-3 | Li | Al | 3.4 | 5.3 | 500 |
| Comparative Example 9 | | | Blue | E-4 | E-4 | Li | Al | 3.8 | 5.2 | 600 |
| Comparative Example 10 | | | Blue | E-5 | E-5 | Li | Al | 3.9 | 5.0 | 600 |

**[Table 3]**

| | Emissive material | | | First electron transporting layer | Second electron transporting layer | | Cathode | External quantum efficiency (%) | Driving voltage (V) | Durability (h) |
|---|---|---|---|---|---|---|---|---|---|---|
| | Host material | Dopant material | Emitted color | Compound | Compound | Donor compound | Metal | | | |
| Example 25 | H-1 | D-1 | Blue | None | B-1 | 2E-1 | Mg/Ag | 6.0 | 3.9 | 1800 |
| Example 26 | | | Blue | None | B-2 | 2E-1 | Mg/Ag | 5.9 | 4.0 | 1850 |
| Example 27 | | | Blue | None | B-3 | 2E-1 | Mg/Ag | 5.9 | 4.0 | 1850 |
| Example 28 | | | Blue | None | B-4 | 2E-1 | Mg/Ag | 5.8 | 4.1 | 1700 |
| Example 29 | | | Blue | None | B-5 | 2E-1 | Mg/Ag | 5.7 | 4.1 | 1600 |
| Example 30 | | | Blue | None | B-6 | 2E-1 | Mg/Ag | 5.4 | 4.3 | 1350 |
| Example 31 | | | Blue | None | B-7 | 2E-1 | Mg/Ag | 5.3 | 4.4 | 1200 |
| Example 32 | | | Blue | None | B-8 | 2E-1 | Mg/Ag | 5.2 | 4.4 | 1200 |
| Example 33 | | | Blue | None | B-9 | 2E-1 | Mg/Ag | 5.1 | 4.5 | 1100 |
| Reference Example 34 | | | Blue | None | B-10 | 2E-1 | Mg/Ag | 5.2 | 4.4 | 1200 |
| Example 35 | | | Blue | None | B-11 | 2E-1 | Mg/Ag | 4.4 | 4.8 | 900 |
| Reference Example 36 | | | Blue | None | B-12 | 2E-1 | Mg/Ag | 4.3 | 4.8 | 800 |
| Comparative Example 11 | H-1 | D-1 | Blue | None | E-1 | 2E-1 | Mg/Ag | 2.8 | 5.5 | 400 |
| Comparative Example 12 | | | Blue | None | E-2 | 2E-1 | Mg/Ag | 3.1 | 5.3 | 450 |
| Comparative Example 13 | | | Blue | None | E-3 | 2E-1 | Mg/Ag | 3.5 | 5.1 | 600 |
| Comparative Example 14 | | | Blue | None | E-4 | 2E-1 | Mg/Ag | 3.9 | 5.1 | 650 |
| Comparative Example 15 | | | Blue | None | E-5 | 2E-1 | Mg/Ag | 4.0 | 5.0 | 650 |

**[Table 4]**

| | Emissive material | | | First electron transporting layer | Second electron transporting layer | | Cathode | External quantum efficiency (%) | Driving voltage (V) | Durability (h) |
|---|---|---|---|---|---|---|---|---|---|---|
| | Host material | Dopant material | Emitted color | Compound | Compound | Donor compound | Metal | | | |
| Example 37 | H-1 | D-1 | Blue | B-1 | B-1 | 2E-1 | Mg/Ag | 5.9 | 4.0 | 1950 |
| Example 38 | | | Blue | B-2 | B-2 | 2E-1 | Mg/Ag | 5.8 | 4.1 | 2000 |
| Example 39 | | | Blue | B-3 | B-3 | 2E-1 | Mg/Ag | 5.7 | 4.1 | 1950 |
| Example 40 | | | Blue | B-4 | B-4 | 2E-1 | Mg/Ag | 5.6 | 4.2 | 1800 |
| Example 41 | | | Blue | B-5 | B-5 | 2E-1 | Mg/Ag | 5.5 | 4.2 | 1700 |
| Example 42 | | | Blue | B-6 | B-6 | 2E-1 | Mg/Ag | 5.3 | 4.4 | 1400 |
| Example 43 | | | Blue | B-7 | B-7 | 2E-1 | Mg/Ag | 5.2 | 4.5 | 1300 |
| Example 44 | | | Blue | B-8 | B-8 | 2E-1 | Mg/Ag | 5.1 | 4.5 | 1300 |
| Example 45 | | | Blue | B-9 | B-9 | 2E-1 | Mg/Ag | 5.0 | 4.6 | 1150 |
| Reference Example 46 | | | Blue | B-10 | B-10 | 2E-1 | Mg/Ag | 5.1 | 4.5 | 1300 |
| Example 47 | | | Blue | B-11 | B-11 | 2E-1 | Mg/Ag | 4.2 | 4.8 | 1000 |
| Reference Example 48 | | | Blue | B-12 | B-12 | 2E-1 | Mg/Ag | 4.2 | 4.9 | 900 |
| Comparative Example 16 | H-1 | D-1 | Blue | E-1 | E-1 | 2E-1 | Mg/Ag | 2.7 | 5.6 | 450 |
| Comparative Example 17 | | | Blue | E-2 | E-2 | 2E-1 | Mg/Ag | 3.0 | 5.5 | 500 |
| Comparative Example 18 | | | Blue | E-3 | E-3 | 2E-1 | Mg/Ag | 3.4 | 5.3 | 600 |
| Comparative Example 19 | | | Blue | E-4 | E-4 | 2E-1 | Mg/Ag | 3.8 | 5.2 | 700 |
| Comparative Example 20 | | | Blue | E-5 | E-5 | 2E-1 | Mg/Ag | 3.9 | 5.1 | 700 |

**[Table 5]**

| | Hole injection layer | First hole transporting layer | Second hole transporting layer | Emissive layer | | | Electron transportin g layer | External quantum efficiency (%) | Driving voltage (V) | Durability (h) |
|---|---|---|---|---|---|---|---|---|---|---|
| | | | | Host material | Dopant material | Emitted color | | | | |
| Example 49 | | | None | | | Green | B-2 | 10.4 | 3.9 | 1400 |
| Example 50 | | | None | | | Green | B-1 | 10.7 | 3.8 | 1300 |
| Example 51 | | | None | | | Green | B-4 | 9.9 | 3.9 | 1150 |
| Example 52 | | | None | | | Green | B-9 | 9.2 | 4.1 | 900 |
| Reference Example 53 | | | None | | | Green | B-10 | 9.3 | 4.1 | 1000 |
| Example 54 | | | None | | | Green | B-11 | 8.5 | 4.3 | 800 |
| Example 55 | | | HT-2 | | | Green | B-2 | 13.3 | 3.9 | 1600 |
| Example 56 | | | HT-3 | | | Green | | 15.8 | 4.1 | 2400 |
| Example 57 | | | HT-4 | | | Green | | 18.0 | 4.1 | 2600 |
| Example 58 | HAT-CN6 | HT-1 | HT-5 | H-2 | D-2 | Green | | 17.6 | 4.0 | 2600 |
| Example 59 | | | HT-2 | | | Green | B-1 | 13.5 | 3.8 | 1450 |
| Example 60 | | | HT-3 | | | Green | | 15.9 | 4.1 | 2200 |
| Example 61 | | | HT-4 | | | Green | | 18.1 | 4.0 | 2400 |
| Example 62 | | | HT-2 | | | Green | B-4 | 12.5 | 3.9 | 1250 |
| Example 63 | | | HT-4 | | | Green | | 16.7 | 4.1 | 2100 |
| Example 64 | | | HT-2 | | | Green | B-9 | 11.1 | 4.1 | 1000 |
| Example 65 | | | HT-4 | | | Green | | 15.2 | 4.3 | 1600 |
| Reference Example 66 | | | HT-2 | | | Green | B-10 | 11.6 | 4.1 | 1100 |
| Reference Example 67 | | | HT-4 | | | Green | | 15.7 | 4.3 | 1800 |
| Example 68 | | | HT-2 | | | Green | B-11 | 10.1 | 4.3 | 900 |
| Example 69 | | | HT-4 | | | Green | | 13.5 | 4.5 | 1400 |
| Comparative Example 21 | HAT-CN6 | HT-1 | None | H-2 | D-2 | Green | E-2 | 6.9 | 5.0 | 600 |
| Comparative Example 22 | | | None | | | Green | E-3 | 7.4 | 4.9 | 650 |
| Comparative Example 23 | | | None | | | Green | E-5 | 8.0 | 4.6 | 700 |
| Comparative Example 24 | | | HT-2 | | | Green | E-2 | 8.4 | 5.0 | 700 |
| Comparative Example 25 | | | HT-3 | | | Green | | 9.1 | 5.3 | 1000 |
| Comparative Example 26 | | | HT-4 | | | Green | | 10.5 | 5.4 | 1000 |
| Comparative Example 27 | | | HT-5 | | | Green | | 9.9 | 5.2 | 1100 |
| Comparative Example 28 | | | HT-2 | | | Green | E-3 | 8.9 | 4.9 | 750 |
| Comparative Example 29 | | | HT-4 | | | Green | | 11.4 | 5.2 | 1100 |
| Comparative Example 30 | | | HT-2 | | | Green | E-5 | 9.6 | 4.6 | 800 |
| Comparative Example 31 | | | HT-4 | | | Green | | 12.6 | 4.9 | 1150 |

**[Table 6]**

| | Hole injection layer | Hole transporting layer | Emissive layer | | Charge generation layer | | Electron transporting layer | External quantum efficiency (%) | Driving voltage (V) | Durability (h) |
|---|---|---|---|---|---|---|---|---|---|---|
| | | | Host material | Dopant material | N-type | P-type | | | | |
| Example 70 | HAT-CN6 | HT-6 | H-1 | D-3 | B-1 | HT-6 | ET-1 | 6.9 | 8.7 | 1000 |
| Example 71 | | | | | B-2 | | | 7.0 | 8.6 | 1000 |
| Example 72 | | | | | B-3 | | | 6.9 | 8.7 | 1000 |
| Example 73 | | | | | B-4 | | | 6.7 | 8.8 | 950 |
| Example 74 | | | | | B-9 | | | 6.5 | 9.0 | 850 |
| Reference Example 75 | | | | | B-10 | | | 6.5 | 8.9 | 900 |
| Example 76 | | | | | B-11 | | | 6.3 | 9.1 | 800 |
| Comparative Example 32 | HAT-CN6 | HT-6 | H-1 | D-3 | E-1 | HT-6 | ET-1 | 5.4 | 10.2 | 500 |
| Comparative Example 33 | | | | | E-2 | | | 5.4 | 10.0 | 500 |
| Comparative Example 34 | | | | | E-3 | | | 5.7 | 9.8 | 600 |
| Comparative Example 35 | | | | | E-4 | | | 6.0 | 9.7 | 600 |
| Comparative Example 36 | | | | | E-5 | | | 6.1 | 9.4 | 600 |

## Claims

1. A phenanthroline derivative represented by the following general formula (1): 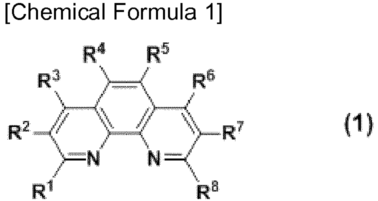 wherein R¹ to R⁸ may be the same or different, and are each selected from the group consisting of hydrogen, heavy hydrogen, an alkyl group, a cycloalkyl group, a heterocyclic group, an alkenyl group, a cycloalkenyl group, an alkynyl group, a halogen, a cyano group, a carbonyl group, a carboxyl group, an oxycarbonyl group, a carbamoyl group, an amino group, a boryl group, a silyl group and -P(=O)R⁹R¹⁰_{;} R⁹ and R¹⁰ each represent an aryl group or a heteroaryl group, with the proviso that any one of R¹ and R² is a group represented by L¹-B, and any one of R⁷ and R⁸ is a group represented by L²-C; R¹ to R¹⁰ may be each substituted or unsubstituted; R¹ to R⁸ has no phenanthroline skeleton;
L¹ and L² may be the same or different, and are each selected from a single bond and a phenylene group;
wherein B is a pyridyl group, a quinolinyl group, an isoquinolinyl group, a pyrazinyl group, a pyrimidinyl group, a pyridazinyl group, a triazinyl group, a quinoxalinyl group, a quinazolinyl group, a benzoquinolinyl group, an acridinyl group, a benzoimidazolyl group or an imidazopyridyl group,
wherein C is a naphthyl group, a fluorenyl group, phenanthryl group, a pyrenyl group, a triphenylenyl group or a fluoranthenyl group,
substituents that B and C optionally have are each selected from the group consisting of heavy hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, a halogen, a cyano group, a carbonyl group, a carboxyl group, an oxycarbonyl group, a carbamoyl group, an amino group, a boryl group, a silyl group, a phenyl group, a naphthyl group, a pyridyl group, a quinolinyl group, and -P(=O)R⁹R¹⁰; and these groups may be each substituted with heavy hydrogen, an alkyl group, a halogen, a phenyl group, a naphthyl group, a pyridyl group or a quinolinyl group.

2. The phenanthroline derivative according to claim 1, wherein R¹ is a group represented by L¹-B, and R⁸ is a group represented by L²-C.

3. The phenanthroline derivative according to claim 1 or 2, wherein L² is a phenylene group.

4. The phenanthroline derivative according to any one of claims 1 to 3, wherein C is a fluorenyl group, phenanthryl group, a pyrenyl group, a triphenylenyl group or a fluoranthenyl group.

5. The phenanthroline derivative according to any one of claims 1 to 4, wherein C is a pyrenyl group or a fluoranthenyl group.

6. The phenanthroline derivative according to any one of claims 1 to 5, wherein B is a pyridyl group, a quinolinyl group or an isoquinolinyl group.

7. A light emitting element which has a plurality of organic layers each including an emissive layer between an anode and a cathode, and emits light by electric energy, wherein at least one of the organic layers contains the phenanthroline derivative according to any one of claims 1 to 6.

8. The light emitting element according to claim 7, wherein the organic layers include at least an electron transporting layer, and the electron transporting layer contains the phenanthroline derivative according to any one of claims 1 to 6.

9. The light emitting element according to claim 7 or 8, wherein the organic layers include at least an electron injection layer, and the electron injection layer contains the phenanthroline derivative according to any one of claims 1 to 6.

10. The light emitting element according to any one of claims 7 to 9, wherein the organic layers include at least a charge generation layer, and the charge generation layer contains the phenanthroline derivative according to any one of claims 1 to 6.

11. A photoelectric conversion element which has at least one organic layer between a first electrode and a second electrode, and converts light energy into electric energy, wherein the organic layer contains the phenanthroline derivative according to any one of claims 1 to 6.

12. The photoelectric conversion element according to claim 11, wherein the organic layer includes a photoelectric conversion layer, and the photoelectric conversion layer contains the phenanthroline derivative according to any one of claims 1 to 6.

13. The photoelectric conversion element according to claim 11 or 12, wherein the organic layer includes at least one photoelectric conversion layer and one electron extraction layer between the first electrode and the second electrode, and the electron extraction layer contains the phenanthroline derivative according to any one of claims 1 to 6.

14. An image sensor comprising the photoelectric conversion element according to any one of claims 11 to 13.

## Patentansprüche

1. Phenanthrolinderivat, dargestellt durch die folgende allgemeine Formel (1): 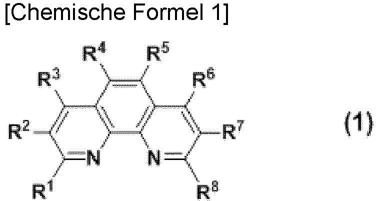 wobei R¹ bis R⁸ gleich oder verschieden sein können, und jeweils ausgewählt sind aus der Gruppe bestehend aus Wasserstoff, schwerem Wasserstoff, einer Alkylgruppe, einer Cycloalkylgruppe, einer heterocyclischen Gruppe, einer Alkenylgruppe, einer Cycloalkenylgruppe, einer Alkinylgruppe, einem Halogen, einer Cyanogruppe, einer Carbonylgruppe, einer Carboxylgruppe, einer Oxycarbonylgruppe, einer Carbamoylgruppe, einer Aminogruppe, einer Borylgruppe, einer Silylgruppe und -P(=O)R⁹R¹⁰; R⁹ und R¹⁰ jeweils eine Arylgruppe oder eine Heteroarylgruppe darstellen, vorausgesetzt, dass ein beliebiges R¹ und R² eine durch L¹-B dargestellte Gruppe darstellt, und ein beliebiges R⁷ und R⁸ eine durch L²-C dargestellte Gruppe darstellt; R¹ bis R¹⁰ jeweils substituiert oder unsubstituiert sein können; R¹ bis R⁸ kein Phenanthrolingerüst haben;
L¹ und L² gleich oder verschieden sein können, und jeweils ausgewählt sind aus einer Einfachbindung und einer Phenylengruppe;
wobei B eine Pyridylgruppe, eine Chinolinylgruppe, eine Isochinolinylgruppe, eine Pyrazinylgruppe, eine Pyrimidinylgruppe, eine Pyridazinylgruppe, eine Triazinylgruppe, eine Chinoxalinylgruppe, eine Chinazolinylgruppe, eine Benzochinolinylgruppe, eine Acridinylgruppe, eine Benzoimidolgruppe oder eine Imidazopyridylgruppe ist,
wobei C eine Naphthylgruppe, eine Fluorenylgruppe, eine Phenanthrylgruppe, eine Pyrenylgruppe, eine Triphenylenylgruppe oder eine Fluoranthenylgruppe ist,
Substituenten, die B und C gegebenenfalls haben, jeweils ausgewählt sind aus der Gruppe bestehend aus schwerem Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einem Halogen, einer Cyanogruppe, einer Carbonylgruppe, einer Carboxylgruppe, einer Oxycarbonylgruppe, einer Carbamoylgruppe, einer Aminogruppe, einer Borylgruppe, einer Silylgruppe, einer Phenylgruppe, einer Naphthylgruppe, einer Pyridylgruppe, einer Chinolinylgruppe, und -P(=O)R⁹R¹⁰; und diese Gruppen jeweils mit schwerem Wasserstoff, einer Alkylgruppe, einem Halogen, einer Phenylgruppe, einer Naphthylgruppe, einer Pyridylgruppe oder einer Chinolinylgruppe substituiert sein können.

2. Phenanthrolinderivat nach Anspruch 1, wobei R¹ eine durch L¹-B dargestellte Gruppe ist und R⁸ eine durch L²-C dargestellte Gruppe ist.

3. Phenanthrolinderivat nach Anspruch 1 oder 2, wobei L² eine Phenylengruppe ist.

4. Phenanthrolinderivat nach einem der Ansprüche 1 bis 3, wobei C eine Fluorenylgruppe, Phenanthrylgruppe, eine Pyrenylgruppe, eine Triphenylenylgruppe oder eine Fluoranthenylgruppe ist.

5. Phenanthrolinderivat nach einem der Ansprüche 1 bis 4, wobei C eine Pyrenylgruppe oder eine Fluoranthenylgruppe ist.

6. Phenanthrolinderivat nach einem der Ansprüche 1 bis 5, wobei B eine Pyridylgruppe, eine Chinolinylgruppe oder eine Isochinolinylgruppe ist.

7. Licht emittierendes Element, das eine Vielzahl von organischen Schichten hat, die jeweils eine Emissionsschicht zwischen einer Anode und einer Kathode enthalten, und Licht durch elektrische Energie emittiert, wobei mindestens eine der organischen Schichten das Phenanthrolinderivat nach einem der Ansprüche 1 bis 6 enthält.

8. Licht emittierendes Element nach Anspruch 7, wobei die organischen Schichten mindestens eine Elektronentransportschicht enthalten, und die Elektronentransportschicht das Phenanthrolinderivat nach einem der Ansprüche 1 bis 6 enthält.

9. Licht emittierendes Element nach Anspruch 7 oder 8, wobei die organischen Schichten mindestens eine Elektroneninjektionsschicht enthalten, und die Elektroneninjektionsschicht das Phenanthrolinderivat nach einem der Ansprüche 1 bis 6 enthält.

10. Licht emittierendes Element nach einem der Ansprüche 7 bis 9, wobei die organischen Schichten mindestens eine Ladungserzeugungsschicht enthalten, und die Ladungserzeugungsschicht das Phenanthrolinderivat nach einem der Ansprüche 1 bis 6 enthält.

11. Photoelektrisches Umwandlungselement, das mindestens eine organische Schicht zwischen einer ersten Elektrode und einer zweiten Elektrode hat, und Lichtenergie in elektrische Energie umwandelt, wobei die organische Schicht das Phenanthrolinderivat nach einem der Ansprüche 1 bis 6 enthält.

12. Photoelektrisches Umwandlungselement nach Anspruch 11, wobei die organische Schicht eine photoelektrische Umwandlungsschicht enthält, und die photoelektrische Umwandlungsschicht das Phenanthrolinderivat nach einem der Ansprüche 1 bis 6 enthält.

13. Photoelektrisches Umwandlungselement nach Anspruch 11 oder 12, wobei die organische Schicht mindestens eine photoelektrische Umwandlungsschicht und eine Elektronenextraktionsschicht zwischen der ersten Elektrode und der zweiten Elektrode umfasst, und die Elektronenextraktionsschicht das Phenanthrolinderivat nach einem der Ansprüche 1 bis 6 enthält.

14. Bildsensor, der das photoelektrische Umwandlungselement nach einem der Ansprüche 11 bis 13 aufweist.

## Revendications

1. Dérivé de phénanthroline représenté par la formule générale (1) suivante : 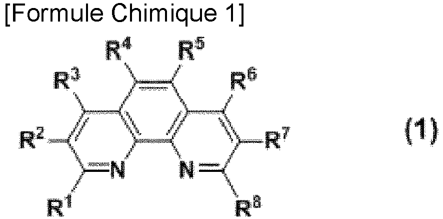 où R¹ à R⁸ peuvent être identiques ou différents, et sont chacun choisis dans le groupe constitué par un hydrogène, un hydrogène lourd, un groupe alkyle, un groupe cycloalkyle, un groupe hétérocyclique, un groupe alcényle, un groupe cycloalcényle, un groupe alcynyle, un halogène, un groupe cyano, un groupe carbonyle, un groupe carboxyle, un groupe oxycarbonyle, un groupe carbamoyle, un groupe amino, un groupe boryle, un groupe silyle et - P(=O)R⁹R¹⁰; R⁹ et R¹⁰ représentent chacun un groupe aryle ou un groupe hétéroaryle, à condition que l'un quelconque de R¹ et R² soit un groupe représenté par L¹-B, et l'un quelconque de R⁷ et R⁸ soit un groupe représenté par L²-C; R¹ à R¹⁰ peuvent être chacun substitués ou non substitués ; R¹ à R⁸ n'a pas de squelette phénanthroline ;
L¹ et L² peuvent être identiques ou différents, et sont chacun choisis parmi une liaison simple et un groupe phénylène ;
où B est un groupe pyridyle, un groupe quinolinyle, un groupe isoquinolinyle, un groupe pyrazinyle, un groupe pyrimidinyle, un groupe pyridazinyle, un groupe triazinyle, un groupe quinoxalinyle, un groupe quinazolinyle, un groupe benzoquinolinyle, un groupe acridinyle, un groupe benzoimidazolyle ou un groupe imidazopyridyle,
où C est un groupe naphtyle, un groupe fluorényle, un groupe phénanthryle, un groupe pyrényle, un groupe triphénylényle ou un groupe fluoranthényle,
les substituants que B et C ont éventuellement sont chacun choisis dans le groupe constitué par un hydrogène lourd, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un halogène, un groupe cyano, un groupe carbonyle, un groupe carboxyle, un groupe oxycarbonyle, un groupe carbamoyle, un groupe amino, un groupe boryle, un groupe silyle, un groupe phényle, un groupe naphtyle, un groupe pyridyle, un groupe quinolinyle et - P(=O)R⁹R¹⁰ ; et ces groupes peuvent être chacun substitués par un hydrogène lourd, un groupe alkyle, un halogène, un groupe phényle, un groupe naphtyle, un groupe pyridyle ou un groupe quinolinyle.

2. Dérivé de phénanthroline selon la revendication 1, dans lequel R¹ est un groupe représenté par L¹-B, et R⁸ est un groupe représenté par L²-C.

3. Dérivé de phénanthroline selon la revendication 1 ou 2, dans lequel L² est un groupe phénylène.

4. Dérivé de phénanthroline selon l'une quelconque des revendications 1 à 3, dans lequel C est un groupe fluorényle, un groupe phénanthryle, un groupe pyrényle, un groupe triphénylényle ou un groupe fluoranthényle.

5. Dérivé de phénanthroline selon l'une quelconque des revendications 1 à 4, dans lequel C est un groupe pyrényle ou un groupe fluoranthényle.

6. Dérivé de phénanthroline selon l'une quelconque des revendications 1 à 5, dans lequel B est un groupe pyridyle, un groupe quinolinyle ou un groupe isoquinolinyle.

7. Élément électroluminescent qui a une pluralité de couches organiques comportant chacune une couche émissive entre une anode et une cathode, et émet de la lumière par de l'énergie électrique, dans lequel au moins une des couches organiques contient le dérivé de phénanthroline selon l'une quelconque des revendications 1 à 6.

8. Élément électroluminescent selon la revendication 7, dans lequel les couches organiques comportent au moins une couche de transport d'électrons, et la couche de transport d'électrons contient le dérivé de phénanthroline selon l'une quelconque des revendications 1 à 6.

9. Élément électroluminescent selon la revendication 7 ou 8, dans lequel les couches organiques comportent au moins une couche d'injection d'électrons, et la couche d'injection d'électrons contient le dérivé de phénanthroline selon l'une quelconque des revendications 1 à 6.

10. Élément électroluminescent selon l'une quelconque des revendications 7 à 9, dans lequel les couches organiques comportent au moins une couche de génération de charge, et la couche de génération de charge contient le dérivé de phénanthroline selon l'une quelconque des revendications 1 à 6.

11. Élément de conversion photoélectrique qui a au moins une couche organique entre une première électrode et une deuxième électrode, et convertit l'énergie lumineuse en énergie électrique, dans lequel
la couche organique contient le dérivé de phénanthroline selon l'une quelconque des revendications 1 à 6.

12. Élément de conversion photoélectrique selon la revendication 11, dans lequel la couche organique comporte une couche de conversion photoélectrique, et la couche de conversion photoélectrique contient le dérivé de phénanthroline selon l'une quelconque des revendications 1 à 6.

13. Élément de conversion photoélectrique selon la revendication 11 ou 12, dans lequel la couche organique comporte au moins une couche de conversion photoélectrique et une couche d'extraction d'électrons entre la première électrode et la deuxième électrode, et la couche d'extraction d'électrons contient le dérivé de phénanthroline selon l'une quelconque l'une des revendications 1 à 6.

14. Capteur d'image comprenant l'élément de conversion photoélectrique selon l'une quelconque des revendications 11 à 13.
